(19) Europäisches Patentamt
European Patent Office
Office européen des brevets

(11) EP 4 350 330 A1

# (12) EUROPEAN PATENT APPLICATION
published in accordance with Art. 153(4) EPC

(43) Date of publication:
**10.04.2024 Bulletin 2024/15**

(21) Application number: **22811325.4**

(22) Date of filing: **24.05.2022**

(51) International Patent Classification (IPC):
***G01N 21/64** (2006.01)* ***B82Y 15/00** (2011.01)*
***B82Y 30/00** (2011.01)* ***C09K 11/06** (2006.01)*
***C09K 11/08** (2006.01)* ***C09K 11/58** (2006.01)*
***G01N 21/78** (2006.01)* ***G01N 33/543** (2006.01)*
***G01N 33/553** (2006.01)*

(52) Cooperative Patent Classification (CPC):
**B82Y 15/00; B82Y 30/00; C09K 11/06; C09K 11/08; C09K 11/58; G01N 21/64; G01N 21/78; G01N 33/543; G01N 33/553**

(86) International application number:
**PCT/JP2022/021270**

(87) International publication number:
**WO 2022/250055 (01.12.2022 Gazette 2022/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**27.05.2021 JP 2021089468**
**27.05.2021 JP 2021089524**
**10.09.2021 JP 2021147949**
**01.02.2022 JP 2022014357**
**01.02.2022 JP 2022014361**
**01.02.2022 JP 2022014218**

(71) Applicant: **PHC Holdings Corporation**
**Tokyo 105-8433 (JP)**

(72) Inventors:
- **KOMATSU, Yoshie**
**Ehime 791-0395 (JP)**
- **KITAWAKI, Fumihisa**
**Ehime 791-0395 (JP)**

(74) Representative: **Eisenführ Speiser**
**Patentanwälte Rechtsanwälte PartGmbB**
**Postfach 10 60 78**
**28060 Bremen (DE)**

## (54) NANOPARTICLE BODY, METHOD FOR PRODUCING SAME, AND NANOPARTICLE BODY COMPOSITION CONTAINING NANOPARTICLE BODY

(57) A nanoparticle comprising a metallic nanoparticle, a polymer layer covering a surface of the metallic nanoparticle, and a nanosized specifically bondable substance that is bonded to a surface of the polymer layer and is to be specifically bonded to a test substance in a specimen.

FIG.1

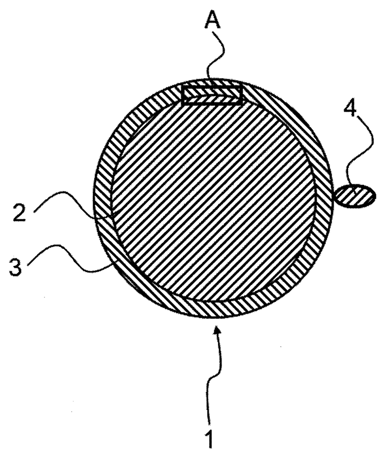

## Description

### TECHNICAL FIELD

The present invention relates to a nanoparticle (particularly, a nanoparticle to be used for plasmon-excited fluorescence analysis), a method for producing the same, and a nanoparticle composition comprising a nanoparticle (particularly, a nanoparticle composition to be used for plasmon-excited fluorescence analysis).

### BACKGROUND ART

A biosensor makes a specific test substance to be detected specifically react with a specific specifically bondable substance to form a composite, and detects the test substance by a signal derived from a specific bond in the composite.
In plasmon-excited fluorescence analysis, the composite further comprises, for example, a fluorescent substance and a metallic particle in addition to a test substance and a specifically bondable substance (the composite comprises, for example, a metallic particle, a specifically bondable substance, a fluorescent substance, and a test substance). When the composite is irradiated with excitation light, surface plasmon resonance is generated in the metallic particles in the composite, and a near field is formed in the vicinity of the surface of the metallic particle. The near field increases the fluorescence intensity of the fluorescent substance.
The composite particle for immunochromatogram described in Patent Document 1 is composed of a fine particle that has a structure in which the exterior of a fine particle made of metal is covered with at least one layer of silica containing at least one fluorescent substance, and has a surface modified with a labeling substance that specifically recognizes a target substance. In the composite particle of Patent Document 1, the surface of the metallic particle is covered with the silica layer, and the fluorescent substance is immobilized to the silica layer, whereby the fluorescent substance excited by a near field formed by surface plasmon resonance is prevented from coming into contact with the metal particle. Accordingly, deactivation (quenching) of the excited fluorescent substance is inhibited.

### PRIOR ART DOCUMENT

### PATENT DOCUMENT

Patent Document 1: JP-A-2011-220705

### SUMMARY OF THE INVENTION

### PROBLEMS TO BE SOLVED BY THE INVENTION

Incidentally, the present inventors have found through intensive studies that the sensor as described above has room for further improvement in detection stability. Specifically, since it is usually difficult for a specifically bondable substance to be directly bonded to a silica layer, it is necessary to perform surface modification or the like on the silica layer in order to improve the bonding property to the silica layer. Owing to this, the thickness of the silica layer is increased, and the distance between metallic particles in a composite could not be sufficiently reduced. As a result, detection sensitivity is lowered.
On the other hand, in the case of preparing a silica layer, tetraethoxysilane is hydrolyzed under a basic condition to form a silica layer in a polymerizing manner, but the rate of the formation thereof is greatly affected by the reaction concentration, the reaction temperature, the reaction time, and so on, and therefore it is difficult to stably prepare the silica layer.
The present invention has been devised in light of the above problems. That is, a main object of the present invention is to provide a nanoparticle having further superior detection stability by forming a thin layer stably fixed on the surface of a metallic particle. More specifically, a main object of the present invention is to provide a nanoparticle further superior in detection sensitivity by surely captures a test substance, sufficiently inhibits quenching of a fluorescent substance, and decreases the distance between metallic particles in a composite.

### MEANS FOR SOLVING THE PROBLEMS

A nanoparticle (or nanoparticle structure or nanoparticle construction) according to one embodiment of the present invention comprises a metallic nanoparticle, a polymer layer covering a surface of the metallic nanoparticle, and a nanosized specifically bondable substance that is bonded to a surface of the polymer layer and is to be specifically bonded to a test substance in a specimen,

wherein the polymer layer contains at least one selected from the group consisting of a binding site with a sulfur atom interposing therein, a positively charged group, and a hydrophobic group between the polymer layer and a surface of the metallic nanoparticle.

BRIEF DESCRIPTION OF THE DRAWINGS

Fig. 1 is a cross-sectional view schematically illustrating a nanoparticle according to the first embodiment.
Fig. 2 is an enlarged cross-sectional view of the portion A in Fig. 1.
Fig. 3 is a cross-sectional view schematically illustrating a nanoparticle composition according to the second embodiment.
Fig. 4 is a cross-sectional view schematically illustrating a composite according to the third embodiment.
Fig. 5 is a cross-sectional view schematically illustrating a composite according to a modification example of the third embodiment.
Fig. 6 is a diagram schematically illustrating the measuring device according to the fourth embodiment.
Fig. 7 is a schematic diagram for explaining a method for producing a polymer layer.
Fig. 8 is a schematic diagram for explaining a method for producing a polymer layer.
Fig. 9 is a diagram showing a scanning electron microscope (SEM) image of a metallic nanoparticle coated with a polymer layer. The metallic nanoparticle is a silver nanoparticle (particle diameter: 80 nm) in Fig. 9(a) and a gold nanoparticle (particle diameter: 20 nm) in Fig. 9(b).
Fig. 10 is a diagram showing a graph of the zeta potential of a metallic nanoparticle coated with a polymer layer. The metallic nanoparticle is a silver nanoparticle (particle diameter: 80 nm) in Fig. 10(a) and a gold nanoparticle (particle diameter: 20 nm) in Fig. 10(b).
Fig. 11 is a diagram showing a graph of the zeta potential of a metallic nanoparticle not coated with a polymer layer. The metallic nanoparticle is a silver nanoparticle (particle diameter: 80 nm) in Fig. 11(a) and a gold nanoparticle (particle diameter: 20 nm) in Fig. 11(b).
Fig. 12 is a diagram showing a fluorescence spectrum of a fluorescent substance-gold nanoparticle mixed system. The gold nanoparticle is coated with a polymer layer in Fig. 12(a), and is not coated with a polymer layer in Fig. 12(b).
Fig. 13 is a cross-sectional view schematically illustrating a nanoparticle of Example 1.
Fig. 14 is a diagram showing a fluorescence spectrum of a test substance-nanoparticle system. The solid line indicates the fluorescence spectrum in the case where a CRP antigen is reacted, and the broken line indicates the fluorescence spectrum of a blank sample.
Fig. 15 is a view showing an SEM image of the composite prepared using the nanoparticle of Example 2.
Fig. 16 is a schematic cross-sectional view illustrating a nanoparticle in the nanoparticle composition of Example 3.
Fig. 17 is a graph showing the absorption spectrum of the nanoparticle composition of Example 3.
Fig. 18 is a graph showing the fluorescence spectrum of the nanoparticle composition of Example 3.
Fig. 19 is a schematic diagram for explaining the method for producing the nanoparticle of Example 4.
Fig. 20 includes graphs showing an absorption spectrum and a fluorescence spectrum of the nanoparticle composition of Example 4.
Fig. 21 includes diagrams showing the frequency distribution of size of Example 5.
Fig. 22 includes diagrams showing the frequency distribution of the zeta potential of Example 5.
Fig. 23 includes schematic views showing the relationship between the number of times of washing treatment and the form of an associated body of Example 5.
Fig. 24 includes cross-sectional views schematically illustrating a nanoparticle according to the fourth embodiment.
Fig. 25 is an enlarged schematic view of the portion A in Fig. 24.
Fig. 26 is a reaction scheme showing one example of the method for producing the nanoparticle according to the fifth embodiment.
Fig. 27 is a cross-sectional view schematically illustrating the composite according to the sixth embodiment.
Fig. 28 is a cross-sectional view schematically illustrating the measuring device according to the seventh embodiment.
Fig. 29 is a schematic diagram for explaining the method for producing the nanoparticle of Example 6.
Fig. 30 is a diagram showing the fluorescence spectrum of the nanoparticle of Example 6.
Fig. 31 is a view showing an SEM image of the composite prepared using the nanoparticle of Example 7.

MODES FOR CARRYING OUT THE INVENTION

Hereinafter, a nanoparticle, a composite, and a measuring device which are embodiments of the present invention will be described in detail with reference to the embodiments shown in the drawings. Note that the drawings

include some schematic ones and may not reflect actual dimensions or proportions.

The numerical ranges referred to herein are intended to include the lower and upper limits themselves. That is, taking a numerical range of 1 nm to 10 nm as an example, the numerical range is interpreted as including the lower limit value "1 nm" and the upper limit value "1 nm".

<First Embodiment: Nanoparticle>

The nanoparticle according to the first embodiment

comprises a metallic nanoparticle, a polymer layer covering a surface of the metallic nanoparticle, and a nanosized specifically bondable substance that is bonded to a surface of the polymer layer and is to be specifically bonded to a test substance in a specimen,
wherein the polymer layer contains at least one selected from the group consisting of a binding site with a sulfur atom interposing therein, a positively charged group, and a hydrophobic group between the polymer layer and a surface of the metallic nanoparticle.

[Method for Detecting Test Substance]

First, for convenience of description of the nanoparticle according to the present embodiment, for the purpose of aiding understanding thereof, a method for detecting a test substance using the nanoparticle according to the present embodiment will be described.

The nanoparticle according to the present embodiment comprises a metallic nanoparticle, a polymer layer covering the surface of the metallic nanoparticle, and a specifically bondable substance bonded to the polymer layer. The nanoparticle according to the present embodiment is dissolved or dispersed in a specimen, and a test substance contained in the specimen is captured to form a composite (second embodiment). More specifically, the composite is formed by specifically bonding of the specifically bondable substance of the nanoparticle with the test substance. The composite has a structure in which two nanoparticles are bonded with the test substance interposing therebetween. As described above, in the composite, two metallic nanoparticles are disposed apart from each other at a certain distance as a result of bonding to the same test substance with their respective specifically bondable substances interposing. Further, the composite comprises a fluorescent substance.

In surface plasmon fluorescence spectroscopy (SPFS), irradiation of the composite with excitation light causes localized surface plasmon resonance (LSPR), so that a near field is efficiently formed near the surfaces of the metallic nanoparticles (in particular, near the surfaces located between two metallic nanoparticles). The near field efficiently excites the fluorescent substance of the composite and increases the fluorescence intensity. By measuring the fluorescence intensity, the test substance in the specimen can be detected.

[Mechanism of Action]

The nanoparticle according to the present embodiment is further superior in detection stability. Without being bound by a particular theory, the reason for this is presumed as follows. The nanoparticle according to the present embodiment comprises a metallic nanoparticle and a polymer layer covering the surface of the metallic nanoparticle, and the polymer layer comprises at least one selected from the group consisting of a binding site with a sulfur atom interposing therein, a positively charged group, and a hydrophobic group between the surface of the metallic nanoparticle and the polymer layer. Therefore, the polymer layer forms a relatively strong bond with the surface of the metallic nanoparticle, so that the polymer layer is stably fixed to the surface of the metallic nanoparticle. As a result, for example, peeling of the polymer layer from the surface of the metallic nanoparticle is prevented, and eventually, "exposure of the surface of the metallic nanoparticle" and "detachment of the specifically bondable substance" associated with peeling of the polymer layer or the like are inhibited, and a decrease in detection sensitivity is inhibited. Furthermore, since the polymer layer contains a polymer, the polymer layer is more easily chemically modified than a silica layer. As a result, the thickness of the polymer layer can be made smaller than that of a silica layer, and the distance between metallic particles in the composite can be reduced. Therefore, the detection sensitivity can be improved. In view of the above, it is considered that the nanoparticle according to the present embodiment is further superior in detection stability.

[Configuration of Nanoparticle]

Hereinafter, the configuration of a nanoparticle will be described. With reference to Fig. 1, the nanoparticle will be described. Fig. 1 is a cross-sectional view schematically illustrating a nanoparticle. The nanoparticle 1 according to the present embodiment comprises a metallic nanoparticle 2, a polymer layer 3 covering the surface of the metallic

nanoparticle 2, and a specifically bondable substance 4 bonded to the surface of the polymer layer 3.

The nanoparticle 1 can be used for plasmon-excited fluorescence analysis. In other words, the nanoparticle 1 can be used for surface plasmon-field enhanced fluorescence spectroscopic immunoassay. The nanoparticle 1 can capture a test substance in a specimen and form a composite comprising two nanoparticles 1 and the test substance. When the composite is irradiated with excitation light, localized surface plasmon resonance occurs to form a near field. The near field increases the fluorescence intensity.

The nanoparticle 1 may be blocked with a blocking agent at a nonspecific binding site. The blocked nanoparticle 1 is inhibited from forming a nonspecific bond to a substance other than the detection target of the specifically bondable substance 4 (that is, a substance other than the test substance) to reduce the background and the false positive signal, and can improve the signal-noise ratio (SN ratio). Examples of the blocking agent include proteins such as bovine serum albumin (BSA), skim milk, and casein, and chemically synthesized polymers.

When the nanoparticle 1 is present in a solvent, the dispersion of the nanoparticle 1 may further contain a dispersant for the purpose of improving the dispersibility of the nanoparticle 1. Examples of such a dispersant include sodium heparin. This point will be described in detail in the description on the nanoparticle composition according to the second embodiment.

Hereinafter, the metallic nanoparticle 2, the polymer layer 3, the specifically bondable substance 4, and the fluorescent substance constituting the nanoparticle 1 will be described.

(Metallic Nanoparticle)

The metallic nanoparticle 2 is coated on the surface thereof with a polymer layer 3. The metallic nanoparticle 2 interacts with light having a specific wavelength, which varies depending on the type of metal, and cause localized surface plasmon resonance. There is a plasmon resonance peak in a range of from 400 nm to 530 nm for silver nanoparticles, and from 510 nm to 580 nm for gold nanoparticles. This depends on the particle diameter. For example, nanoparticles made of silver and having a particle diameter of 20 nm resonate with light having a wavelength of 405 nm, and nanoparticles made of gold and having a particle diameter of 20 nm resonate with light having a wavelength of 524 nm. The particle diameter (average primary particle diameter) of the metallic nanoparticles 2 is, for example, 5 nm to 100 nm. The particle diameter of the metallic nanoparticles 2 can be determined by capturing an image of the metallic nanoparticles 2 using a scanning electron microscope (SEM) or a transmission electron microscope (TEM), measuring the particle diameter of the metallic nanoparticles 2 in the image, and calculating the average value (the number of measurements: for example, at least 10) of a plurality of particle diameters.

The metallic nanoparticle 2 preferably comprises gold or silver, and more preferably comprises silver.

(Polymer layer)

The polymer layer 3 covers the surface of the metallic nanoparticle 2. The polymer layer 3 functions as a metallic quenching molecular layer. In the composite, the polymer layer 3 can make a fluorescent substance place apart from the surface of the metallic nanoparticle 2 by at least the thickness of the polymer layer 3. Therefore, it is possible to inhibit the excited fluorescent substance from coming into contact with the surface of the metallic nanoparticle 2 and quenching, and to inhibit a decrease in detection sensitivity. The presence of the polymer layer 3 can be confirmed by capturing an image of the nanoparticle 1 using SEM or TEM, and observing the nanoparticle 1 in the image.

The polymer layer 3 will be described with reference to Fig. 2. Fig. 2 is an enlarged view of the portion A in Fig. 1, and is an enlarged cross-sectional view of the vicinity of the interface between the polymer layer 3 and the surface of the metallic nanoparticle 2 in the nanoparticle 1. The polymer layer 3 contains at least one selected from the group consisting of a binding site 3a with a sulfur atom interposing therein between the polymer layer 3 and the surface of the metallic nanoparticle 2, a positively charged group 3b, and a hydrophobic group 3c. More specifically, the polymer layer 3 contains a binding site 3a with a sulfur atom interposing therein the polymer layer 3 and the surface of the metallic nanoparticle 2, a primary ammonium group ($-NH^{3+}$) as the positively charged group 3b, and a hydrophobic group 3c. The binding site 3a binds between the surface of the metallic nanoparticle 2 and the polymer layer 3 with a sulfur atom interposing therebetween. The positively charged group 3b forms an electrostatic bond (ionic bond) b with the surface of the negatively charged metallic nanoparticle 2. The hydrophobic group 3c forms a hydrophobic bond c with the surface of the metallic nanoparticle 2.

The polymer layer 3 is stably fixed to the surface of the metallic nanoparticle 2 by at least one of the three bonds described above because all of the three bonds are relatively strong bonds with the surface of the metallic nanoparticle 2. As a result, for example, peeling of the polymer layer 3 from the surface of the metallic nanoparticle 2 is prevented. As a result, detachment of the specifically bondable substance 4 associated with the peeling or the like of the polymer layer 3 is inhibited, and a decrease in detection sensitivity is inhibited. In addition, exposure of the surface of the metallic nanoparticle 2 due to peeling or the like of the polymer layer 3 is inhibited, so that quenching due to

contact with an excited fluorescent substance is inhibited and a decrease in detection sensitivity is inhibited. Furthermore, since the polymer layer 3 comprises the polymer 3A, the polymer layer 3 is easily chemically modified as compared with a silica layer, and the necessity for surface modification or the like is low. As a result, the thickness of the polymer layer can be made smaller than that of a silica layer, and the distance between metallic nanoparticles 2 in the composite can be reduced. Therefore, a near field is formed more efficiently, and the detection sensitivity can be improved. For these reasons, the nanoparticle according to the present embodiment is further superior in detection stability.

As illustrated in Fig. 2, the polymer 3A that constitutes the polymer layer 3 can contain at least one selected from the group consisting of a binding site 3a with a sulfur atom interposing therein, a positively charged group 3b, and a hydrophobic group 3c between the polymer 3A and the surface of the metallic nanoparticles 2. The presence of the binding site 3a with a sulfur atom interposing therein, the positively charged group 3b, and the hydrophobic group 3c can be confirmed by measuring signals derived therefrom using infrared spectroscopy and nuclear magnetic resonance spectroscopy. The polymer 3A constituting the polymer layer 3 can have a moiety containing a disulfide linkage (-S-S-) as a side chain. The moiety containing a disulfide linkage can have a positively charged group 3b. The moiety containing a disulfide linkage can have a hydrophobic group 3c.

Hereinafter, the binding site 3a with a sulfur atom interposing therein, the positively charged group 3b, and the hydrophobic group 3c will be described.

- Binding Site with Sulfur Atom Interposing Therein-

The binding site 3a with a sulfur atom interposing therein is formed, for example, by mixing a polymer having a moiety containing a disulfide linkage as a side chain with a metallic nanoparticle 2. When the polymer as a raw material has, for example, a hydrophobic group 3c in a side chain with a disulfide linkage interposing therein as shown in Fig. 8 described later, a binding site with a sulfur atom interposing therein is formed between the surface of the metallic nanoparticle 2 and the polymer (see Fig. 2 and the right side in Fig. 8).

- Positively Charged Group -

The positively charged group forms a strong electrostatic bond with the surface of the metallic nanoparticle 2. In the present description, the positively charged group is a group having a valence of one or more and completely positively ionized. In consideration of a plurality of positively charged groups 3b contained in the polymer constituting the polymer layer 3, the positively charged groups 3b refer to groups having a pKa of 7 or more, the pKa being represented by the following expression (1):
[Expression 1]

$$pKa = pH - log\frac{[B]}{[BH^+]} \cdots (1)$$

in the expression (1), pKa represents a pKa of a group that is an electrically neutral group contained in the polymer 3A constituting the polymer layer 3 and can become a positively charged group (specifically, a primary ammonium group ($-NH^{3+}$) or the like) 3b when positively charged (this type of group is also referred to as an electrically neutral group) (more specifically, a primary amino group ($-NH_2$) or the like); pH represents a pH of an environment where a test substance is detected (more specifically, a specimen or the like); B represents an electrically neutral group contained in the polymer 3A; and BH+ represents a positively charged group 3b contained in the polymer 3A. The positively charged group 3b refers to a group having a concentration ($[BH^+]$) of the positively charged group that is 10 times or more larger than the concentration ([B)) of the electrically neutral group in the case where the electrically neutral group of the polymer 3A constituting the polymer layer 3 and the positively charged group 3b are forming an equilibrium state represented by the following chemical equilibrium formula (2)

[Chemical Formula 1]

$$B + H^+ \rightleftarrows BH^+ \cdots (2)$$

in an environment where the electrically neutral group and the positively charged group detect a test substance (for example, a specimen having a pH of about 6 to 8).

The positively charged group 3b is preferably at least one selected from the group consisting of a primary

ammonium group, a secondary ammonium group, a tertiary ammonium group, a quaternary ammonium group, and a guanidyl group ($-NHC(=NH_2^+)NH_2$).

- Hydrophobic Group -

The hydrophobic group 3c is at least one selected from the group consisting of an aromatic cyclic group, an aliphatic cyclic group, and an aliphatic chain group.

Examples of the aromatic cyclic group include an aromatic carbocyclic group and an aromatic heterocyclic group. The aromatic carbocyclic group is a group which does not contain any aromatic heterocyclic ring and contains an aromatic ring in which all ring atoms are carbon atoms. Examples of the aromatic carbocyclic group include aryl groups (more specifically, a phenyl group and the like) and arylalkyl groups (more specifically, a benzyl group and the like). The aromatic heterocyclic group is a group containing an aromatic ring in which at least one of the ring atoms is a hetero atom (more specifically, an oxygen atom, a sulfur atom, a nitrogen atom, or the like). Examples of the aromatic heterocyclic group include nitrogen-containing aromatic heterocyclic groups (more specifically, an imidazoyl group, a pyridyl group (pyridinyl group), and the like.), sulfur-containing aromatic heterocyclic groups, and oxygen-containing aromatic heterocyclic groups.

The aliphatic cyclic group is a group which does not contain any aromatic ring and contains a cyclic group composed of a non-aromatic ring. Examples of the aliphatic cyclic group include an aliphatic carbocyclic group and an aliphatic heterocyclic group. The aliphatic carbocyclic group is a group containing a non-aromatic ring in which all ring atoms are carbon atoms, and examples thereof include a cycloalkyl group. An aliphatic heterocyclic group is a group containing a non-aromatic ring in which at least one of the ring atoms is a heteroatom.

The aliphatic chain group is a chain (more specifically, linear or branched) group containing no aromatic ring and no non-aromatic ring. Examples of the aliphatic chain group include aliphatic carbon chain groups (more specifically, an alkyl group, an alkylene group, and the like) and aliphatic heterochain groups.

The polymer 3A constituting the polymer layer 3 can form a hydrophobic bond between the hydrophobic group 3c that the polymer 3A can have and the surface of the metallic nanoparticle 2. The polymer 3A constituting the polymer layer 3 can also form other hydrophobic bonds. For example, a hydrophobic bond can be formed between a hydrophobic group bonded to the surface of the metallic nanoparticle 2 with a sulfur atom interposing (more specifically, for example, a pyridyl group (pyridinyl group) bonded to the surface of the metallic nanoparticle 2 with a sulfur atom interposing shown in Fig. 8) and a hydrophobic group 3c that can be possessed by the polymer 3A constituting the polymer layer 3 (more specifically, an alkylene group possessed by the polymer 3A shown in Fig. 8). When such a hydrophobic bond is formed, the polymer layer 3 is more stably fixed to the surface of the metallic nanoparticle 2. The hydrophobic group bonded to the surface of the metallic nanoparticle 2 with a sulfur atom interposing is formed as follows. As described above, the binding site with a sulfur atom interposing therein can be formed, for example, by mixing a polymer having a hydrophobic group 3c with a disulfide linkage interposing in a side chain with the metallic nanoparticles 2. Here, the hydrophobic group 3c bonded to a sulfur atom is also bonded to the surface of the metallic nanoparticle 2. In this way, a hydrophobic group bonded to the surface of the metallic nanoparticle 2 with a sulfur atom interposing is formed.

The polymer 3A constituting the polymer layer 3 may form, with a moiety derived from a crosslinker (a linker moiety) interposing, a linkage with a sulfur atom interposing therein. Examples of such a crosslinker include an amino group-sulfhydryl group crosslinker (more specifically, an NHS-maleimide group crosslinker or the like).

The thickness of the polymer layer 3 is preferably 1 nm to 50 nm, and more preferably 1 nm to 10 nm. When the thickness of the polymer layer 3 is 50 nm or less, a separative distance (separation distance) with which a near field is efficiently formed in a space between two metallic nanoparticles is obtained, and thus detection sensitivity is further improved. When the thickness of the polymer layer 3 is 1 nm or more, the metallic nanoparticle 2 and a fluorescent substance are disposed at a prescribed distance, so that quenching of a fluorescent substance excited in measurement is inhibited, and detection sensitivity is further improved.

In the present description, the separative distance (separation distance) refers to the minimum value (shortest distance) of the distance between the surfaces of the metallic nanoparticles contained in two nanoparticles bound with a test substance interposing therebetween in a composite.

(Specifically Bondable Substance)

The specifically bondable substance 4 is a nanosized (having a size of 3 to 15 nm at the longest) substance that is to be specifically bonded to a test substance (described in the second embodiment) in a specimen. Examples of the specifically bondable substance 4 include an antibody (hereinafter, referred to as a nanoantibody), a ligand, an enzyme, and a nucleic acid strand (more specifically, a DNA strand and an RNA strand). For example, the nanoantibody as the specifically bondable substance 4 is specifically bonded to an antigen as a test substance at the tip portion (antigen binding site) of the nanoantibody through an antigen-antibody reaction to form a composite. The ligand as the specifically

bondable substance 4 forms a composite by specifically protein-ligand bonding to a protein as a test substance through a ligand receptor reaction. A nucleic acid strand as the specifically bondable substance 4 forms a pair (double strand) of a nucleic acid strand and another nucleic acid strand in a complementary relationship on the basis of the complementarity of a base pair. An enzyme as the specifically bondable substance 4 forms an enzyme-substrate composite with a substrate as a test substance on the basis of the substrate specificity (stereospecificity) at the active moiety (active center) of the enzyme. These specific bonds are non-covalent bonds, for example, hydrogen bonds and bonds caused by intermolecular force, hydrophobic interaction, and charge interaction.

Examples of the nanoantibodies include variable domain of heavy chain antibody (VHH) antibodies, fragment antigen binding (Fab) antibodies, and variants thereof. A VHH antibody is a single domain antibody. The variant is an antibody in which a part of an amino acid sequence is recombined or an antibody in which a substituent is introduced, as long as the variant has specific binding to an antigen. The nanoantibody is preferably a VHH antibody. When the nanoantibody is a VHH antibody, because of a relatively small volume of the VHH antibody, it is possible to reduce the distance (separation distance) between two metallic nanoparticles 2 in a composite, more efficiently form a near field, and further increase the fluorescence intensity.

The molecular mass of the nanoantibody is preferably 60,000 Da or less, more preferably 30,000 Da or less, and still more preferably 20,000 Da or less. When the molecular mass is 60,000 Da or less (in particular, 30,000 Da or less, or 20,000 Da or less), since the volume of the nanoantibody is relatively small, it is possible to reduce the separation distance in a composite to efficiently form a near field and further increase the fluorescence intensity. Examples of the method for measuring the molecular mass include electrophoresis (SDS-PAGE), gel filtration chromatography, and static light scattering.

The specifically bondable substance 4 may be directly bonded to the polymer layer 3, or may be indirectly bonded to the polymer layer 3 with interposition of a linker portion (more specifically, SM(PEG)6 or the like) derived from a crosslinker (more specifically, an NHS-maleimide group crosslinker or the like).

(Fluorescent Substance)

The nanoparticle 1 may further comprise a fluorescent substance. In this case, the fluorescent substance is labeled on at least one of the surface of the polymer layer 3 and the specifically bondable substance. The fluorescent substance is excited by a near field formed by localized surface plasmon resonance and emits fluorescence. Examples of the fluorescent substance include complexes of metals such as europium and ruthenium (metal complexes), and dyes of the Alexsa Fluor series (registered trademark) (Molecular Probes (registered trademark)).

The fluorescent substance preferably has a large Stokes shift. Here, the Stokes shift is a difference between an absorption peak wavelength (maximum excitation wavelength) in an absorption spectrum of the fluorescent substance and a fluorescence peak wavelength (maximum fluorescence wavelength) in a fluorescence spectrum. When the Stokes shift of the fluorescent substance is large, the absorption spectrum and the fluorescence spectrum hardly overlap each other and (scattered light of) excitation light hardly enters the fluorescence to be detected, so that more accurate fluorescence intensity can be measured.

Preferably, the fluorescence spectrum of the fluorescent substance is sharp. When the fluorescence spectrum is sharp, the fluorescence spectrum hardly overlaps the absorption spectrum, and thus (scattered light of) excitation light hardly enters the fluorescence to be detected, so that fluorescence intensity can be measured more accurately.

<Second Embodiment: Nanoparticle Composition>

The nanoparticle composition according to a second embodiment comprises a nanoparticle and a solvent containing the nanoparticle,

wherein the nanoparticle comprises a metallic nanoparticle, a polymer layer covering the surface of the metallic nanoparticle, and a specifically bondable substance that is bonded to the surface of the polymer layer or the surface of the metallic nanoparticle and is to be specifically bonded to a test substance in a specimen, and
the solvent contains a polyanion-based polymer in addition to the nanoparticle.

[Background to Nanoparticle Composition according to Second Embodiment]

The composite particle (nanoparticle) for immunochromatogram described in Patent Document 1 is composed of a fine particle that has a structure in which the exterior of a fine particle made of metal (metallic nanoparticle) is covered with at least one layer of silica containing at least one fluorescent substance, and has a surface modified with a labeling substance (specifically bondable substance) that specifically recognizes a target substance (test substance). As described above, in the nanoparticle described in Patent Document 1, the coating layer covering the exterior of the metallic

nanoparticle is surface-modified with the specifically bondable substance.

Incidentally, as a result of intensive studies by the present inventors, it has been found that in the sensor as described above there is room for further improving dispersibility while maintaining the degree of freedom in designing the nanoparticle. Specifically, the design of the nanoparticle may be changed as necessary. For example, in order to sufficiently capture a desired test substance, the number of specifically bondable substances which are bonded to the coating layer is increased. In addition, in order to increase the fluorescence intensity to be detected, the number of fluorescent substances bonded to the coating layer is increased. In these cases, as the number of the specifically bondable substance or the fluorescent substance increases, the dispersibility of nanoparticle decreases, and in the worst case, the nanoparticle may agglomerate.

On the other hand, when the dispersant is bonded to the coating layer to improve the dispersibility of the nanoparticle, there arises a problem that the number of bonding sites on the coating layer decreases and the degree of freedom in designing the nanoparticle is impaired. As described above, it was not possible to sufficiently achieve both a high degree of freedom in design and superior dispersibility.

It is a main object of the present embodiment to provide a nanoparticle composition comprising a nanoparticle having further superior dispersibility while maintaining a degree of freedom in design in addition to the superior detection sensitivity described above.

[Method for Detecting Test Substance]

First, for convenience of description of the nanoparticle composition according to the present embodiment, for the purpose of aiding understanding thereof, one example of a method for detecting a test substance using the nanoparticle composition according to the present embodiment will be described.

The nanoparticle composition according to the present embodiment comprises a nanoparticle and a solvent containing the nanoparticle. The nanoparticle comprises a metallic nanoparticle, a polymer layer covering the surface of the metallic nanoparticle, and a specifically bondable substance that is bonded to the surface of the polymer layer or the surface of the metallic nanoparticle. The nanoparticle composition according to the present embodiment is added to a specimen, and captures a test substance contained in the specimen to form a composite. More specifically, the composite is formed by specifically bonding of the specifically bondable substance of the nanoparticle with the test substance. The composite has a structure in which two nanoparticles are bonded with the test substance interposing therebetween. As described above, in the composite, two metallic nanoparticles are disposed apart from each other at a certain distance as a result of bonding to the same test substance with their respective specifically bondable substances interposing. Further, the composite comprises a fluorescent substance.

In surface plasmon fluorescence spectroscopy (SPFS), irradiation of the composite with excitation light causes localized surface plasmon resonance (LSPR), so that a near field is efficiently formed near the surfaces of the metallic nanoparticles (in particular, near the surfaces located between two metallic nanoparticles). The near field efficiently excites the fluorescent substance of the composite and increases the fluorescence intensity. By measuring the fluorescence intensity, the test substance in the specimen can be detected.

[Mechanism of Action]

The nanoparticle composition according to the present embodiment comprises a nanoparticle having further superior dispersibility while maintaining the degree of freedom in design. Without being bound by a particular theory, the reason for this is presumed as follows. The nanoparticle composition according to the present embodiment comprises a nanoparticle and a solvent containing the nanoparticle. The nanoparticle comprises a metallic nanoparticle, a polymer layer covering the surface of the metallic nanoparticle, and a specifically bondable substance that is bonded to the surface of the polymer layer or the surface of the metallic nanoparticle and is to be specifically bonded to a test substance in a specimen. The solvent contains a polyanion-based polymer in addition to the nanoparticle. Therefore, in the nanoparticle composition according to the present embodiment, the nanoparticle can form an associated body (or associated product or association or associate) (assembly) in which individual particles are surrounded by the polyanion-based polymer. The polyanion-based polymer acts as a spacer among a plurality of nanoparticles, and prevents aggregation due to contact of the nanoparticles with each other. Furthermore, since a plurality of anionic groups of the polyanion-based polymer are present on the exterior surface of the associated body, electrostatic repulsive force acts between associated bodies and brings nanoparticles away from each other. Individual nanoparticles can thereby be dispersed independently in the nanoparticle composition according to the present embodiment. That is, the nanoparticle is superior in dispersibility in the nanoparticle composition according to the present embodiment.

As described above, the nanoparticle has not been subjected to surface modification for improving dispersibility. Therefore, there is room for modifying the surface of the nanoparticle, and the degree of freedom in design can be maintained. In view of the above, it is considered that the nanoparticle composition according to the present embodiment

comprises a nanoparticle having both a high degree of freedom in design and superior dispersibility.

[Configuration of Nanoparticle Composition]

Hereinafter, the configuration of the nanoparticle composition will be described. The nanoparticle composition comprises a nanoparticle and a solvent containing the nanoparticle.

(Solvent)

The solvent contains a polyanion-based polymer in addition to the nanoparticle. The solvent comprises, for example, an aqueous solvent. The aqueous solvent comprises at least water (more specifically, pure water). Examples of the aqueous solvent include water (pure water), a mixed solvent comprising water and an organic solvent, and a solvent in which a salt component is dissolved (more specifically, a buffer solution or the like). The organic solvent is an organic solvent miscible with water, and examples thereof include alcohols (more specifically, methanol, ethanol, propanol, and so on), and at least one selected from the group consisting of tetrahydrofuran, dimethylformamide, dimethyl sulfoxide, and the like.

[Polyanion-Based Polymer]

The polyanion-based polymer is a polymer having a plurality of anionic groups. The anionic group may be, for example, at least one anionic group selected from the group consisting of a carboxylate salt group, a sulfate salt group, a sulfonate salt group, a nitrate salt group, a phosphate salt group, and a borate salt group. That is, the polyanion-based polymer may have at least one anionic group selected from the group consisting of a carboxylate salt group, a sulfate salt group, a sulfonate salt group, a nitrate salt group, a phosphate salt group, and a borate salt group.

The polyanion-based polymer may be, for example, at least one selected from polyglutamic acid, heparin:

[Chemical Formula 2]

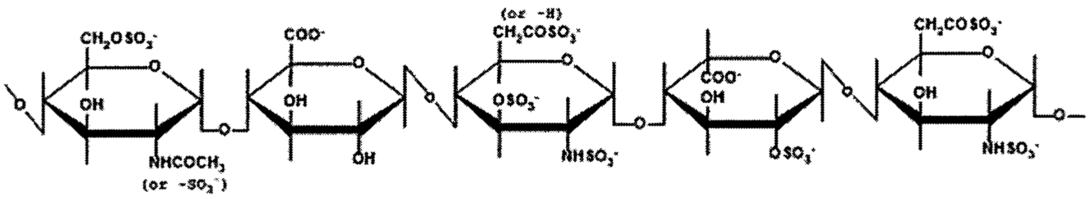

,

polyaspartic acid, polyacrylic acid, salts thereof, and DNA. Examples of the counter ion (counter cation) of the salts recited above include cations of alkali metals (more specifically, $Li^+$, $Na^+$, $K^+$, and the like) and cations of alkaline earth metals (more specifically, $Mg^{2+}$, $Ca^{2+}$, and the like). Heparin has a sulfate salt group ($-OSO_3^-X^+$), a carboxylate salt group ($-COO^-X^+$), and a sulfonate salt group ($-SO_3^-X^+$), wherein $X^+$ is a monovalent counter cation.

Examples of the polyglutamic acid salt include a sodium salt:

[Chemical Formula 3]

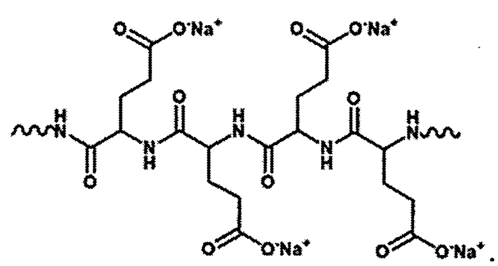

The sodium salt of polyglutamic acid has a carboxylate salt group ($COO^-Na^+$).

The polyanion-based polymer acts as a dispersant for the nanoparticle in the nanoparticle composition according to the present embodiment. The function of the polyanion-based polymer as a dispersant will be described with reference to Fig. 3. Fig. 3 is a cross-sectional view schematically illustrating a nanoparticle in the nanoparticle composition according

to the present embodiment. In the nanoparticle composition according to the present embodiment, polyanion-based polymers 7 exist to surround individual particles of the nanoparticle 1. That is, the nanoparticle composition comprises an associated body (assembly) 9 of the nanoparticle 1 and the polyanion-based polymers 7. The polyanion-based polymers 7 are bonded to the polymer layer 3 of the nanoparticle 1 by electrostatic interaction to form the associated body 9. The associated body 9 behaves like one assembly in the nanoparticle composition according to the present embodiment.

As described above, since the polyanion-based polymers 7 are present to surround the nanoparticle 1, the polyanion-based polymer 7 acts like a spacer between the nanoparticles 1 to prevent aggregation due to contact of nanoparticles 1. Furthermore, since a plurality of anionic groups of the polyanion-based polymer 7 are present on the external surface of the associated body 9, it is considered that an electric double layer is formed near the interface between the associated body and the solvent. As a result, an electrostatic repulsive force (more specifically, electric double layer repulsive force) acts between associated bodies 9 and keeps the nanoparticles 1 away from each other. As a result, the nanoparticles 1 can be dispersed independently in the nanoparticle composition according to the present embodiment. That is, it is considered that the nanoparticle 1 is further superior in dispersibility in the nanoparticle composition according to the present embodiment.

The presence of the associated body 9 can be confirmed by measuring zeta potential. Specifically, the treatment of washing the associated body 9 in the nanoparticle composition with a solvent is performed a plurality of times, and the zeta potential is measured for each washing treatment. When the obtained frequency distribution of a plurality of zeta potentials exhibits a behavior of increasing from a negative value with an increase in the number of times of washing, the presence of the associated body 9 is strongly suggested. Such behavior of the zeta potential is not bound by a specific theory, but is presumed to be due to the following reason. Before the washing treatment, the zeta potential indicates a negative value due to the polyanion-based polymers 7 that form the associated body 9. As the washing treatment is performed, the plurality of polyanion-based polymers 7 forming the associated body 9 are gradually dissociated from the associated body 9 and dissolved in the washing solvent. Therefore, the zeta potential increases and approaches the zeta potential of the nanoparticle 1 as the number of the polyanion-based polymers 7 of the associated body 9 decreases as the washing treatment is performed repeatedly.

The presence of the associated body 9 can also be confirmed by measuring the size. Specifically, the treatment of washing the associated body 9 in the nanoparticle composition with a solvent is performed a plurality of times, and the size is measured for each washing treatment. When a plurality of frequency distributions of size obtained exhibits a behavior of increasing with an increase in the number of times of washing, the presence of the associated body 9 is strongly suggested. Such behavior of the size is not bound by a specific theory, but is presumed to be due to the following reason. As the washing treatment is performed, the plurality of polyanion-based polymers 7 forming the associated body 9 are gradually dissociated from the associated body 9 and dissolved in the washing solvent. Specifically, in the frequency distribution of size in the case of performing many times of washing treatment, the peak shifts toward a larger size. Such behavior of the size is not bound by a specific theory, but is presumed to be due to the following reason. When a large number of times of washing treatment is performed, the dispersibility of the nanoparticle 1 is deteriorated. As a result, the nanoparticles 1 agglomerate to form an agglomerate, and the size increases.

Preferably, the content of the polyanion-based polymer 7 is much larger (that is, excessive) as compared with the content of the nanoparticle 1 in the nanoparticle composition according to the present embodiment. This is because, in such a case, the nanoparticle 1 is easily surrounded by the polyanion-based polymer 7 in the nanoparticle composition according to the present embodiment. As a result, the dispersibility of the nanoparticle 1 is further improved.

(Nanoparticle)

- Linker -

The nanoparticle 1 can have a linker that is bonded to the polymer layer 3 as long as the degree of freedom in designing the nanoparticle 1 is not significantly reduced. The specifically bondable substance 4 is not bonded to a terminal of the linker. When the nanoparticle 1 has a linker bonded to the polymer layer 3, the linker acts like a spacer between nanoparticles 1 (acts as a steric hindrance) to prevent aggregation due to contact of nanoparticles 1. In such a case, the dispersibility of the nanoparticle 1 in the nanoparticle composition according to the present embodiment is further improved.

The linker contains, for example, at least one selected from the group consisting of a polyalkylene ether chain and an alkyl chain. The polyalkylene ether chain and the alkyl chain may be a part of or the entire of the linker moiety. The polyalkylene ether chain is, for example, a polyalkyleneoxy group (more specifically, a polyethyleneoxy group or the like). The alkyl chain is, for example, an alkylene group (more specifically, a -propylene group, a n-butylene group, or the like).

<Third Embodiment: Composite>

The composite will be described with reference to Fig. 4. Fig. 4 is a cross-sectional view schematically illustrating the composite. The composite 40 comprises a test substance 30 to be detected and two nanoparticles 10 and 20. In the composite 40, the two nanoparticles 10 and 20 are bound with the test substance 30 interposing therebetween. That is, the nanoparticles 10 and 20 according to the first embodiment form the composite according to the second embodiment bound with the test substance 30 interposing. Of the two nanoparticles 10 and 20, one is referred to as a first nanoparticle 10, and the other is referred to as a second nanoparticle 20. As described above, the composite 40 includes the first nanoparticle 10 and the second nanoparticle 20 as the nanoparticle 1.

In the composite 40, the first nanoparticle 10 includes the first metallic nanoparticle 12, the first polymer layer 13 covering the surface of the first metallic nanoparticle 12, the first specifically bondable substance 14 bonded to the surface of the first polymer layer 13, and the first fluorescent substance 16 labeled on the first polymer layer 13. That is, the first nanoparticle 10 includes the first metallic nanoparticles 12 as a metallic nanoparticle, the first polymer layer 13 as a polymer layer, and the first specifically bondable substance 14 as a specifically bondable substance, and the first fluorescent substance 16 is labeled on the first polymer layer 13. The second nanoparticle 20 includes the second metallic nanoparticle 22, the second polymer layer 23 covering the surface of the second metallic nanoparticle 22, the second specifically bondable substance 24 bonded to the second polymer layer 23, and the second fluorescent substance 26 labeled on the second polymer layer 23. That is, the second nanoparticle 20 includes the second metallic nanoparticle 22 as a metallic nanoparticle, the second polymer layer 23 as a polymer layer, and the second specifically bondable substance 24 as a specifically bondable substance, and the second fluorescent substance 26 is labeled on the second polymer layer 23.

From the viewpoint of further enhancing the fluorescence intensity, the separation distance L is preferable to be as small as possible as long as excited fluorescent substances 16 and 26 are hardly quenched. More specifically, in a preferred embodiment, the two nanoparticles 10 and 20 in the composite 40 are close to each other. In a more preferred embodiment, the two nanoparticles 10, 20 are close to each other such that the first polymer layer 13 of the first nanoparticle 10 and the second polymer layer 23 of the second nanoparticle 20 in the composite 40 are in contact with each other. In a still more preferred embodiment, the two nanoparticles 10 and 20 are close to each other in such a manner that the first polymer layer 13 of the first nanoparticle 10 and the second polymer layer 23 of the second nanoparticle 20 in the composite 40 come in contact with each other with at least one of the polymer layers shrinking.

In a further preferred embodiment, in the case where the polymer layers 13 and 23 come into contact with each other with at least one of the polymer layers shrinking, for example, in the composite 40 illustrated in Figs. 4 and 5, it is considered to be possible to make at least one of the test substance 30, the specifically bondable substances 14 and 24 bonded to the test substance 30, and the fluorescent substances 16 and 26 to be embedded in the polymer layers 13 and 23. In a still further preferred embodiment, in the case where the polymer layers 13 and 23 are in contact with each other, for example, in the composite 40 illustrated in Figs. 4 and 5, it is considered to be possible, similarly to the further preferred embodiment described above, to make at least one of the test substance 30, the specifically bondable substances 14 and 24 that are bonded to the test substance 30, and the fluorescent substances 16 and 26 to be embedded in the polymer layers 13 and 23.

In the present embodiment, since the layers covering the surfaces of the metallic nanoparticles 12 and 22 are the polymer layers 13 and 23, the fluorescence intensity can be enhanced. The reason for this is presumed as follows. The layers covering the surfaces of the metallic nanoparticles 12 and 22 are the polymer layers 13 and 23, and the polymer layers 13 and 23 have relatively high flexibility as compared with an inorganic film comprising an inorganic oxide. For this reason, in the composite 40, the polymer layers 13 and 23 can shrink, so that the two metallic nanoparticles 12 and 22 can be closer to each other than a distance equivalent to a double of the thickness of each polymer layer (the thickness of the polymer layer 13 + the thickness of the polymer layer 23). That is, since the layers covering the surfaces of the metallic nanoparticles 12 and 22 are the polymer layers 13 and 23, the separation distance L can be less than a double of the thickness of each polymer layer. As a result, the plasmonic enhancement effect is easily obtained, and the fluorescence intensity is further enhanced. In the present description, the thickness of the polymer layer in "a double of the thickness of each polymer layer" is not the thickness of the polymer layer 13 or 23 at the shrinking portion, which serves as the target of the separation distance, but is the thickness of the polymer layer 13 or 23 at a non-shrinking portion, which does not serve as the target of the separation (for example, T1 in Fig. 15 to be described later).

In the present embodiment, since the polymer layers 13 and 23 each comprise at least one selected from the group consisting of a binding site 3a with a sulfur atom interposing therein, a positively charged group 3b, and a hydrophobic group 3c (for example, the polymer 3A constituting the polymer layers 13 and 23 comprises at least one selected from the group consisting of a binding site 3a with a sulfur atom interposing therein, a positively charged group 3b, and a hydrophobic group 3c), the fluorescence intensity can be further enhanced. The reason for this is presumed as follows. In such a case, at least one of the binding site 3a, the positively charged group 3b, and the hydrophobic group 3c forms a bond with the surfaces of the metallic nanoparticles 12 and 22. For this reason, it is considered that the polymer 3A

has a network structure and covers the surfaces of the metallic nanoparticles 12 and 22 in a network manner. As described above, since the polymer 3A has a network structure, the polymer 3A further has relatively high flexibility. For this reason, in the composite 40, the polymer layers 13 and 23 can further shrink, so that the two metallic nanoparticles 12 and 22 can be closer to each other than a distance equivalent to a double of the thickness of each polymer layer. Therefore, in the present embodiment, the separation distance L can be less than a double of the thickness of each polymer layer, the plasmonic enhancement effect is further obtained, and the fluorescence intensity is further enhanced.

In a preferred embodiment, the polymer 3A constituting the polymer layers 13 and 23 contains at least one selected from the group consisting of a binding site 3a with a sulfur atom interposing therein, a positively charged group 3b, and a hydrophobic group 3c on a side chain (more specifically, a side chain terminal) thereof. In a preferred embodiment, the fluorescence intensity can be further enhanced. The reason for this is presumed as follows. In such a case, at least one of the binding site 3a, the positively charged group 3b, and the hydrophobic group 3c forms a bond with the surfaces of the metallic nanoparticles 12 and 22. For this reason, it is considered that the polymer 3A has a network structure and covers the surfaces of the metallic nanoparticles 12 and 22 in a network manner with side chains thereof used as binding sites. As described above, since the polymer 3A has a network structure, the polymer 3A further has relatively high flexibility. For this reason, in the composite 40, the polymer layers 13 and 23 can further shrink, so that the two metallic nanoparticles 12 and 22 can be closer to each other than a distance equivalent to a double of the thickness of each polymer layer. Therefore, in the present embodiment, the separation distance L can be less than a double of the thickness of each polymer layer, the plasmonic enhancement effect is further obtained, and the fluorescence intensity is further enhanced.

The separation distance L between the first nanoparticle 10 and the second nanoparticle 20 is, for example, 12 nm to 52 nm, and preferably 12 nm to 27 nm. The separation distance L is a distance between the first metallic nanoparticle 12 and the second metallic nanoparticle 22, and is a distance with which a line segment connecting between a first point P1 on the surface of the first nanoparticle 10 and a second point P2 on the surface of the second nanoparticle 20 is minimized. In a case where the separation distance L is 52 nm or less, when the composite 40 is irradiated with excitation light, a near field occurs more efficiently in a space near the surfaces between the first and second metallic nanoparticles 12 and 22, so that the fluorescence intensity can be further enhanced.

In one preferred embodiment, the polymer 3A constituting the polymer layers 13 and 23 has at least one selected from the group consisting of a binding site 3a with a sulfur atom interposing therein, a positively charged group 3b, and a hydrophobic group 3c (for example, on a side chain (more specifically, on a side chain terminal)). Therefore, as described above, the separation distance L can be smaller than the distance equivalent to a double of the thickness of each polymer layer covering the surfaces of the two metallic nanoparticles 12 and 22 in the composite 40. For example, when the thicknesses of the polymer layers 13 and 23 are 5 nm, the separation distance L can be less than 10 nm (more specifically, 2 to 9 nm, 3 to 8 nm, 4 to 7 nm, or the like).

(Fluorescent Substance)

As shown in Fig. 4, the fluorescent substances 16 and 26 are preferably positioned between the first metallic nanoparticle 12 and the second metallic nanoparticle 22. This is because, since the near field is efficiently generated a space between the metallic nanoparticles 12 and 22, the fluorescence intensity is easily enhanced by positioning the fluorescent substances 16 and 26 in the space between the metallic nanoparticles 12 and 22.

(Test Substance)

The test substance 30 is a substance to be detected contained in a specimen. Examples of the test substance 30 include an antigen, a protein, a substrate, and a nucleic acid strand. The test substance 30 is specifically bonded to the specifically bondable substances 14 and 24. For example, an antigen has at least two antigenic determinants (epitopes) and forms specific bonding with the first and second specifically bondable substances 14 and 24 at the antigenic determinants. Examples of the antigen include proteins such as c-reactive protein, myoglobin, troponin T, troponin I, and BNP, and antigen proteins of viruses such as influenza virus and RS virus. The test substance 30 is a test substance derived from a specimen such as blood, plasma, urine, or saliva. That is, examples of the specimen containing the test substance 30 include blood, plasma, serum, urine, and saliva. The specimen may further comprise a solvent and a buffer (more specifically, phosphate-buffered saline (PBS), Tris buffer, HEPES buffer, MOPS buffer, MES buffer, or the like).

<Fourth Embodiment: Measuring Device>

The measuring device will be described with reference to Fig. 6. Fig. 6 is a diagram illustrating a measuring device. As illustrated in Fig. 6, the measuring device 100 includes an excitation light source 110, an excitation light

radiation optical system 120, a reagent container 130, a light receiving optical system 140, and a light receiving element 150.

The excitation light source 110 emits excitation light 112. The excitation light source 110 is, for example, a laser. The excitation light radiation optical system 120 performs adjustment of the cross-sectional diameter like condensing of the excitation light 112, and outputs the incident excitation light 122. The excitation light radiation optical system 120 includes a lens 124 and a polarizing element ($\lambda$/2 plate) 126. The incident excitation light 122 output from the excitation light radiation optical system 120 is incident on the reagent container 130, and the measurement sample in the reagent container 130 is irradiated with the incident excitation light. The reagent container 130 is, for example, a detachable container (more specifically, a cell, a preparation, or the like) and a microchannel chip. The microchannel chip is a chip having a minute channel. When the reagent container 130 is a microchannel chip, for example, a nanoparticle (reagent) according to the first embodiment and a specimen can be mixed and continuously supplied. Therefore, it is not necessary to prepare a measurement sample by mixing in advance, and it is possible to continuously perform measurement.

The measurement sample irradiated with the incident excitation light 122 emits fluorescence (detection light 132). The light receiving optical system 140 is arranged in a direction perpendicular to the traveling direction of the incident excitation light 122 toward the reagent container 130. The light receiving optical system 140 adjusts the cross-sectional diameter or the like of the detection light 132 emitted from the measurement sample, and can remove scattered light of the incident excitation light 122 or adjust the amount of light. The light receiving optical system 140 includes a lens 144 and an optical filter 146. The optical filter 146 includes, for example, a band pass filter and a dichroic mirror.

Fluorescence 142 having passed through light receiving optical system 140 is detected by the light receiving element 150. The light receiving element 150 includes, for example, a PD, an APD, a PMT, a CCD camera, and a spectroscope. The light receiving element 150 can measure the amount of fluorescence of a single wavelength, measure a fluorescence spectrum, and create two-dimensional planar fluorescence imaging.

The present invention is not limited to the above-described embodiments, and can be modified in design without departing from the gist of the present invention.

In the third embodiment, the first and second fluorescent substances 16 and 26 are labeled on the first and second polymer layers 13 and 23, respectively, but the present invention is not limited this configuration. For example, Fig. 5 is a cross-sectional view schematically illustrating a composite according to a modification example of the second embodiment. As illustrated in Fig. 5, the first and second fluorescent substances 16 and 26 may be labeled on the first and second specifically bondable substances 14 and 24, respectively. This case is more preferable because the first and second fluorescent substances 16 and 26 are easily positioned between the first metallic nanoparticle 12 and the second metallic nanoparticle 22 and the detection intensity is improved. One of the first and second fluorescent substances 16 and 26 may be labeled on the polymer layers 13 and 23, and the other may be labeled on the specifically bondable substances 14 and 24.

In the third embodiment, two fluorescent substances 16 and 26 are labeled on the composite 40, but the present invention is not limited this configuration. For example, the number of the fluorescent substances labeled on the composite 40 may be 1 or 3 or more.

In the fourth embodiment, the light receiving optical system 140 in the measuring device 100 is disposed in a direction perpendicular to the traveling direction of the incident excitation light 122 toward the reagent container 130, but the present invention is not limited this configuration. For example, the light receiving optical system 140 may be arranged in a direction parallel to the traveling direction of the incident excitation light 122, or may be arranged in a direction having an acute angle or an obtuse angle with respect to the traveling direction of the incident excitation light 122.

## EXAMPLES

Hereinafter, the present invention will be described in more detail with reference to Examples. The present invention is not limited by the following Examples at all. In addition, unless otherwise specified, parts and % in the Examples are on mass basis.

In Examples and Comparative Examples, the concentration of a metallic nanoparticle in a dispersion may be expressed by absorbance. The absorbance was measured using an ultraviolet-visible spectrophotometer ("infinite M200 PRO" manufactured by TECAN Japan Co., Ltd.). Since the absorption wavelength varies depending on each sample, the absorption wavelength was described for each sample. Note that a subscript number attached to the notation OD of the absorbance indicates an absorption wavelength. For example, "$OD_{455}$ = 0.1" indicates that the absorbance at a wavelength of 455 nm is 0.1.

[Preparation of Polymer layer]

(Production Example 1)

The method for producing a polymer layer will be described with reference to Figs. 7 and 8. Figs. 7 and 8 are schematic diagrams for explaining a method for producing a polymer layer. As shown in Fig. 7, poly-L-lysine ("3075 " manufactured by Peptide Institute, Inc.) and 3-(2-pyridyldithio)propionamide-PEG4-NHS (manufactured by Thermo Fisher SCIENTIFIC Inc., serial number "26128", "NHS-PEG4-SPDP") were stirred and mixed at room temperature for 4 hours using a small rotary incubator ("RT-30 mini" manufactured by TAITEC Corporation). As a result, a polymer was obtained. This synthesis reaction is a nucleophilic substitution reaction in which a primary amino group of poly-L-lysine attacks the NHS ester group of 3-(2-pyridyldithio)propionamide-PEG4-NHS. The polymer synthesized had a hydrophobic group (pyridyl group (pyridinyl group)) 3c and a positively charged group (primary ammonium group) 3b. The polymer obtained was added to 1 mL of a dispersion of a silver nanoparticle ("AGCB80-1M" manufactured by nanoComposix, diameter: 80 nm, $OD_{455}$ = 0.1) as a metallic nanoparticle, and the mixture was stirred and mixed at room temperature overnight using a small rotary incubator ("RT-30 mini" manufactured by TAITEC Corporation). As a result, a dispersion of the silver nanoparticle coated with the polymer layer 3 was obtained.

As illustrated in Fig. 8, the polymer layer 3 contains a binding site 3a with a sulfur atom interposing therein on the surface of the silver nanoparticle 2, a hydrophobic group (pyridyl group (pyridinyl group)) 3c that forms a hydrophobic bond with the surface of the silver nanoparticle 2, and a positively charged group (primary ammonium group) 3b that forms an electrostatic bond with the surface of the silver nanoparticle 2. That is, the polymer 3A constituting the polymer layer 3 has a binding site 3a with a sulfur atom interposing therein on the surface of the silver nanoparticle 2, a hydrophobic group (pyridyl group (pyridinyl group)) 3c that forms a hydrophobic bond with the surface of the silver nanoparticle 2, and a positively charged group (primary ammonium group) 3b that forms an electrostatic bond with the surface of the silver nanoparticle 2. An SEM image (500,000 magnifications) of the silver nanoparticle 2 obtained was prepared, and it was confirmed that the surface of the silver nanoparticle 2 was continuously coated with the polymer layer 3. In addition, the thickness of the polymer layer 3 covering the silver nanoparticle 2 was measured from the SEM image (see Fig. 9(a)). The thickness of the polymer layer was 4.98 nm (expected value (average value) M) $\pm$ 2.7 nm (standard deviation $\sigma$, the number of measurements: n = 7), and the variation in thickness ($\sigma$/M) was 27.4%.

(Production Example 2)

A dispersion of a gold nanoparticle 2 coated with a polymer layer 3 (hereinafter, also referred to as a coated gold nanoparticle dispersion) was prepared in the same manner as in Production Example 1 except that 1 mL of the dispersion of the silver nanoparticle ("AGCB80-1M" manufactured by nanoComposix, diameter: 80 nm, $OD_{455}$ = 0.1) in Production Example 1 was changed to 1 mL of a dispersion of a gold nanoparticle ("753610-25 ml" manufactured by SigmaAldirich Co. LLC, diameter: 20 nm, $OD_{520}$ = 0.1). The polymer layer 3 has a binding site 3a with a sulfur atom interposing therein, a positively charged group (primary ammonium group) 3b, and a hydrophobic group (pyridyl group (pyridinyl group)) 3c on the surface of the gold nanoparticle 2. An SEM image (500,000 magnifications) of the gold nanoparticle 2 obtained was prepared, and it was confirmed that the surface of the gold nanoparticle 2 was continuously coated with the polymer layer 3. From the SEM image, the thickness of the polymer layer of the gold nanoparticle 2 was 8.64 nm (expected value M) $\pm$ 0.58 nm (standard deviation $\sigma$, the number of measurements: n = 6), and the variation in thickness ($\sigma$/M) was 4.36% (see Fig. 9(b)).

[Measurement Method and Measurement Results]

(Chargeability of Metallic Nanoparticle Surface and Polymer layer)

The zeta potential of the metallic nanoparticle 2 and the metallic nanoparticle 2 coated with the polymer layer 3 was measured using a zeta potential analyzer ("ZETA SIZER Nanoseries nano-ZS" manufactured by MALVERN). Fig. 10 includes graphs of the zeta potential of the metallic nanoparticle 2 coated with the polymer layer 3 (horizontal axis: zeta potential (unit: mV) and vertical axis: relative intensity (unit: arbitrary unit)). The metallic nanoparticle 2 is a silver nanoparticle (particle diameter: 80 nm) in Fig. 10(a) and a gold nanoparticle (particle diameter: 20 nm) in Fig. 10(b). Fig. 11 includes graphs of the zeta potential of the metallic nanoparticle 2 not coated with the polymer layer 3 (horizontal axis: zeta potential (unit: mV) and vertical axis: relative intensity (unit: arbitrary unit)). The metallic nanoparticle 2 is a silver nanoparticle (particle diameter: 80 nm) in Fig. 11(a) and a gold nanoparticle (particle diameter: 20 nm) in Fig. 11(b).

As shown in Figs. 11(a) and 11(b), each of the zeta potentials of the silver nanoparticle (particle diameter: 80 nm) and the gold nanoparticle (particle diameter: 20 nm) had a peak at a negative potential, and the frequency distribution

of the zeta potentials had a shape in which the entire frequency distribution was almost within a negative potential range. Therefore, it was confirmed that the surfaces of the metallic nanoparticles 2 not coated with the polymer layer 3 were negatively charged.

As shown in Figs. 10(a) and 10(b), each of the zeta potentials of the silver nanoparticle (particle diameter: 80 nm) and the gold nanoparticle (particle diameter: 20 nm) both coated on the surfaces thereof with the polymer layer 3 had a peak at a positive potential, and the frequency distribution of the zeta electron had a shape in which the entire frequency distribution was almost within a positive potential range. Therefore, it was confirmed that the surfaces of the metallic nanoparticles 2 coated with the polymer layer 3 (that is, the surface of polymer layer 3) were positively charged.

From the results of Figs. 10 and 11, it was confirmed that the surface of the metallic nanoparticle 2 and the polymer layer 3 had opposite charging properties. Therefore, it has been strongly suggested that in a metallic nanoparticle 2 coated with a polymer layer 3, the surface of the metallic nanoparticle 2 and the polymer layer 3 form an electrostatic bond.

Furthermore, dispersions of metallic nanoparticles (a silver nanoparticle and a gold nanoparticle) were separately prepared according to Production Examples 1 and 2, and were allowed to stand for 3 months from the preparation. Thereafter, the zeta potential of the metallic nanoparticles 2 coated with the polymer layer 3 was measured. It was confirmed that the metallic nanoparticles 2 coated with the polymer layer 3 left at rest for 3 months were positively charged similarly to the metallic nanoparticles 2 coated with the polymer layer 3 immediately after preparation. Therefore, the polymer layer 3 was shown to be stably held for a long period of time (for example, 3 months).

(Fluorescence Quenching Inhibitability)

(A dispersion of) a metallic nanoparticle 2 coated with a polymer layer 3 was added to a fluorescent substance solution, and the fluorescence intensity with respect to the addition amount was measured, and it was confirmed that the polymer layer 3 contributed to inhibition of fluorescence quenching.

(Fluorescent Substance-Coated Metallic Nanoparticle Mixed System)

The coated gold nanoparticle dispersion of Production Example 2 was added to 5 μL of a dimethyl sulfoxide solution (concentration 10 mg/ml) of tris(2,2'-bipyridyl)ruthenium(II) chloride hexsahydrate (T1655, available from Tokyo Chemical Industry Co., Ltd.) as the fluorescent substances 16 and 26, and mixed to prepare a mixed liquid. The mixed liquid was placed in a microplate. The measurement container was placed in a fluorometer ("infinite M200 PRO" manufactured by TECAN Japan Co., Ltd.), and a fluorescence spectrum was measured. The measurement conditions were a wavelength of excitation light of 430 nm and a detection wavelength of 470 to 700 nm. The addition amount of the coated gold nanoparticle dispersion was varied (60 μL, 120 μL, 180 μL, and 240 μL), and the fluorescence spectrum was measured. The results are shown in Fig. 12(a).

Fig. 12(a) shows a fluorescence spectrum (horizontal axis: fluorescence wavelength (unit: nm) and vertical axis: fluorescence intensity (unit: arbitrary unit)) of a fluorescent substance-gold nanoparticle coated with polymer layer mixed system (hereinafter, also referred to as a fluorescent substance-coated metallic nanoparticle mixed system). The fluorescence spectrum of a system in which the coated gold nanoparticle dispersion was not added (addition amount of coated gold nanoparticle dispersion: 0 μL) exhibited a spectrum profile having peaks at around 614 nm and around 530 nm. The peak at 614 nm is assigned to the ruthenium complex, and the peak around 530 nm is background fluorescence. Taking the fluorescence spectrum with the addition amount of 0 μL of the coated gold nanoparticle dispersion as a standard, it was confirmed that the intensity of the fluorescence spectrum (fluorescence intensity) decreased continuously as a whole as the addition amount of the coated gold nanoparticle dispersion was increased from 60 μL (60 μL → 240 μL).

(Fluorescent Substance-Non-Coated Metallic Nanoparticle Mixed System)

In the same manner as above except that the coated gold nanoparticle dispersion of Production Example 2 was changed to a dispersion of a gold nanoparticle ("753610-25 ml" manufactured by SigmaAldirich Co. LLC, diameter: 20 nm, $OD_{520}$ = 0.1) (hereinafter, this dispersion is also referred to as a non-coated gold nanoparticle dispersion), the fluorescence spectrum of a fluorescent substance-gold nanoparticle not coated with polymer layer mixed system (hereinafter, also referred to as a fluorescent substance-non-coated metallic nanoparticle mixed system) was measured. The measurement result is shown in Fig. 12(b).

Fig. 12(b) shows a fluorescence spectrum (horizontal axis: fluorescence wavelength (unit: nm) and vertical axis: fluorescence intensity (unit: arbitrary unit)) of a fluorescent substance-non-coated metallic nanoparticle mixed system. The fluorescence spectrum of a system in which the non-coated gold nanoparticle dispersion was not added (addition amount of non-coated gold nanoparticle dispersion: 0 μL) exhibited a spectrum profile having peaks at around

614 nm and around 530 nm. Taking the fluorescence spectrum with the addition amount of 0 μL of the non-coated gold nanoparticle dispersion as a standard, it was confirmed that the intensity of the entire fluorescence spectrum decreased continuously as the addition amount of the non-coated gold nanoparticle dispersion increased (60 μL → 240 μL).

(Comparison of Fluorescence Intensity)

As shown in Figs. 12(a) and 12(b), it was confirmed that the intensity of the fluorescence spectrum continuously decreased as the addition amount of the dispersion (a coated gold nanoparticle dispersion in Fig. 12(a) and a non-coated gold nanoparticle dispersion in Fig. 12(b)) increased. Comparing the intensities of these fluorescence spectra, as the addition amount of the dispersion increased, the intensity of the fluorescence spectrum shown in Fig. 12(a) was greater than the intensity of the fluorescence spectrum shown in Fig. 12(b), and it was confirmed that the decrease in fluorescence intensity was inhibited.

That is, as to Fig. 12(a), it is considered that since the gold nanoparticle was coated with the polymer layer, direct contact of the ruthenium complex as a fluorescent substance with the gold nanoparticle was avoided and a decrease in fluorescence intensity was inhibited. On the other hand, as to Fig. 12(b), it is considered that since the gold nanoparticle was not coated with the polymer layer, the ruthenium complex was in direct contact with the gold nanoparticle and the fluorescence intensity greatly decreased. As described above, it has been shown that a polymer layer disposed on the surfaces of a gold nanoparticle inhibits fluorescence quenching.

[Example 1]

[Production of Nanoparticle to which Fluorescently Labeled Antibody is Bonded (Preparation of Nanoparticle Composition)]

Fig. 13 is a schematic view showing the structure of a nanoparticle to which a fluorescently labeled antibody is bonded. The nanoparticle illustrated in Fig. 13 was prepared by first bonding a crosslinker to the surface of a polymer-coated metallic nanoparticle, separately bonding a fluorescent substance and a crosslinker to a nanoantibody, and then bonding the crosslinker bonded to the polymer-coated metallic nanoparticle and the crosslinker bonded to the nanoantibody. Hereinafter, details of the preparation of a nanoparticle to which a fluorescently labeled antibody is bonded will be described. In Fig. 13, n represents the number of ethylene oxide repeating unit, and represents 6.

(Polymer layer-Coated Silver Nanoparticle)

First, a dispersion of a silver nanoparticle 2 coated with the polymer layer 3 (hereinafter, also referred to as a polymer layer-coated silver nanoparticle) was obtained in the same manner as in Production Example 1 described above except that the metallic nanoparticle was changed from ""AGCB80-1M" manufactured by nanoComposix, diameter: 80 nm, $OD_{455}$ = 0.1" to ""AGCB50-1M" manufactured by nanoComposix, diameter: 50 nm, $OD_{455}$ = 0.1".

The resulting polymer layer 3 covered the entire surface of the silver nanoparticle. The polymer layer 3 comprises a binding site 3a with a sulfur atom interposing therein on the surface of the silver nanoparticle 2, a hydrophobic group (pyridyl group (pyridinyl group)) 3c that forms a hydrophobic bond with the surface of the silver nanoparticle 2, and a positively charged group (primary ammonium group) 3b that forms an electrostatic bond with the surface of the silver nanoparticle 2. That is, the polymer 3A constituting the polymer layer 3 has a binding site 3a with a sulfur atom interposing therein on the surface of the silver nanoparticle 2, a hydrophobic group (pyridyl group (pyridinyl group)) 3c that forms a hydrophobic bond with the surface of the silver nanoparticle 2, and a positively charged group (primary ammonium group) 3b that forms an electrostatic bond with the surface of the silver nanoparticle 2 (see Fig. 8).

(Bonding of Crosslinker to Polymer layer-Coated Silver Nanoparticle)

Next, to 1 mL of the prepared dispersion of a polymer layer-coated silver nanoparticle were further added a crosslinker SM(PEG)6 (PEGylated, long-chain SMCC crosslinker) ("22105" manufactured by Thermo Fisher SCIENTIFIC Inc.) and sodium heparin (" 081-00136 " manufactured by FUJIFILM Wako Pure Chemical Corporation), and the mixture was stirred and mixed at room temperature for 1 hour using a small rotary incubator ("RT-30 mini" manufactured by TAITEC Corporation). As a result, a dispersion of a silver nanoparticle coated with a polymer layer 3 to which a crosslinker SM(PEG)6 is bonded (hereinafter, also referred to as a polymer layer-coated silver nanoparticles to which an SM(PEG)6 linker is bonded) was obtained. The SM(PEG)6 linker bonded to the polymer layer-coated silver nanoparticle had a maleimide group.

(Fluorescently Labeling to VHH Antibody)

Alexa Fluor 430 carboxylic acid, succinimidyl ester ("A10169" manufactured by Invitrogen) was added to 100 μg of a VHH antibody (manufactured by RePHAGEN Co., Ltd., molecular mass: 18,000 Da), and the mixture was stirred and mixed at room temperature for 1 hour using a small rotary incubator ("RT-30 mini" manufactured by TAITEC Corporation). As a result, a VHH antibody with a fluorescent substance bonded thereto (hereinafter, also referred to as a fluorescently labeled VHH antibody) was obtained.

(Bonding of Crosslinker to Fluorescently Labeled VHH Antibody)

Subsequently, 3-(2-pyridyldithio)propionamide-PEG4-NHS ("NHS-PEG4-SPDP" manufactured by Tokyo Chemical Industry Co., Ltd.) as an NHS-bipyridyl disulfide crosslinker was added to the fluorescent substance-labeled VHH antibody in a molar ratio of 8 times equivalent, and the mixture was stirred and mixed at room temperature for 1 hour using a small rotary incubator ("RT-30 mini" manufactured by TAITEC Corporation). As a result, a VHH antibody to which a fluorescent substance and an SPDP linker were bonded (hereinafter, also referred to as a fluorescently labeled VHH antibody with an SPDP linker bonded thereto) was obtained.

(Thiolation of Fluorescently Labeled VHH Antibody with Crosslinker Bonded Thereto)

Next, to the fluorescently labeled VHH antibody with an SPDP linker bonded thereto, a reducing agent TCEP ("77720" manufactured by Thermo Fisher SCIENTIFIC Inc.) was added in a molar ratio of 2 times equivalent, and the mixture was stirred and mixed at 37°C for 1 hour using a stirrer ("TS-100" manufactured by BioSan). As a result, a VHH antibody to which a fluorescent substance and an SPDP linker having been reduced (hereinafter, also referred to as a reduced SPDP linker) were bonded (hereinafter, also referred to as a fluorescently labeled VHH antibody with a reduced SPDP linker bonded thereto) was obtained. The reduced SPDP linker had a thiol group (-SH group) generated through reduction of a disulfide linkage.

(Bonding of Fluorescently Labeled VHH Antibody to Silver Nanoparticle)

Subsequently, the fluorescently labeled VHH antibody with a reduced SPDP linker bonded thereto was added to a dispersion ($OD_{430}$ = 0.1) of a polymer layer-coated silver nanoparticle with a maleimide group-containing SM(PEG)6 linker bonded thereto, and the mixture was stirred and mixed at room temperature overnight using a small rotary incubator ("RT-30 mini" manufactured by TAITEC Corporation). As a result, the maleimide group of the SM(PEG)6 linker reacted with the thiol group of the reduced SPDP linker, affording a nanoparticle to which a fluorescently labeled VHH antibody was bond with a linker moiety interposing (see Fig. 13). The thickness of the polymer layer 3 was 8.31 nm (expected value M) $\pm$ 1.89 nm (standard deviation $\sigma$), and the variation in thickness ($\sigma$/M) was 13.7%. The resulting nanoparticle composition contained the nanoparticle illustrated in Fig. 13 and a solvent containing sodium heparin as a polyanion-based polymer.

[Detection of Test Substance by Surface Plasmon-Field Enhanced Fluorescence Spectroscopic Immunoassay]

C Reactive Protein ("00-AGN-AP-CRP-00" manufactured by ADVY CHEMICAL Sigma-Aldrich Co. LLC) (hereinafter, also referred to as a CRP antigen) as a test substance was added to a phosphate buffer solution of the resulting nanoparticle, and the mixture was stirred at room temperature for 5 minutes using a small rotary incubator ("RT-30 mini" manufactured by TAITEC Corporation) to prepare a measurement sample ($OD_{455}$ = 0.4) containing a sandwich type composite. In addition, a blank sample was prepared in the same manner as the measurement sample except that no test substance was added.

(Surface Plasmon-Field Enhanced Fluorescence Spectroscopic Immunoassay)

The measurement sample was placed in a measurement container, and the measurement container was placed in a fluorescence spectrophotometer ("infinite M200 PRO" manufactured by TECAN Japan Co., Ltd.). A measurement container was irradiated with light having a wavelength of 430 nm, and a fluorescence spectrum was measured. The fluorescence spectrum was measured under measurement conditions of a detection wavelength of 470 nm to 700 nm and an optical path length of 3.5 mm. The fluorescent substance contained in the nanoparticle exhibits an absorption spectrum having a peak at 430 nm and a fluorescence spectrum having a peak at 520 nm. The silver nanoparticle (diameter: 50 nm) contained in the nanoparticle exhibits an absorption spectrum having a peak at 430 nm.

The fluorescence spectrum of the blank sample was also measured in the same manner.

The results of the fluorescence spectra obtained are shown in Fig. 14. In this figure, the horizontal axis represents the fluorescence wavelength (unit: nm), and the vertical axis represents the fluorescence intensity. In Fig. 14, the fluorescence spectrum of the "sample in which the CRP antigen was added" indicated by the solid line had a spectrum profile having a peak with a maximum fluorescence intensity of about 50,000 near 520 nm, whereas the fluorescence spectrum of the "blank sample" indicated by the broken line had a spectrum profile having a peak with a maximum fluorescence intensity of about 43,000. The fluorescence intensity of the sample in which the CRP antigen was added was larger than the fluorescence intensity of the blank sample as a whole. As described above, a significant difference was observed between the fluorescence intensity of the sample in which the CRP antigen was added and the fluorescence intensity of the blank sample.

The fluorescence intensity of the blank sample is caused by fluorescence emitted from a fluorescent substance directly excited through absorption of excitation light (light having a wavelength of 430 nm). On the other hand, the fluorescence intensity of the sample in which the CRP antigen was added is caused by fluorescence emitted by a fluorescent substance directly excited through absorption of excitation light and fluorescence emitted by a fluorescent substance indirectly excited by a near field formed by surface plasmon resonance on the surface of the metallic nanoparticle.

Therefore, it is considered that the significant difference in the observed fluorescence intensity was caused by the fluorescence emitted by the fluorescent substance indirectly excited by the near field formed by the surface plasmon resonance on the surface of the metallic nanoparticle.

In view of the above, it is considered that the fluorescence enhancing effect was obtained in the sample of Example 1 in which the CRP antigen was added.

[Example 2: Shrinkability of Polymer layer]

(Preparation of Nanoparticle)

In the preparation of a nanoparticle of Example 2, a nanoparticle was prepared in the same manner as in Example 1 described above except that the following three conditions were changed. The nanoparticle of Example 2 was a nanoparticle to which a fluorescently labeled VHH antibody was bonded with a linker moiety interposing.

- Fluorescent Substance -

The fluorescent substance was changed from Alexa Fluor 430 carboxylic acid, succinimidyl ester ("A10169" manufactured by Invitrogen) to an NHS-labeled Ru complex derivative represented by the chemical formula of [Chemical Formula 3]:

[Chemical Formula 4]

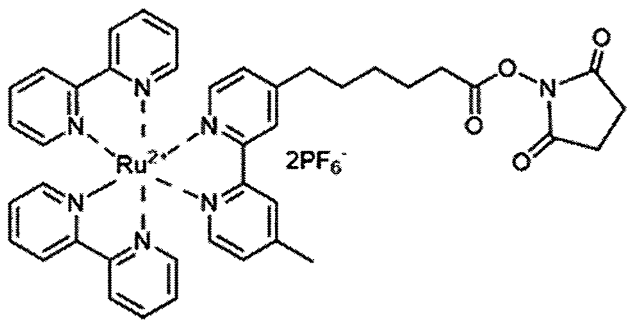

("Ruthenium(II) tris(bipyridyl)-C5-NHS ester" manufactured by Tokyo Chemical Industry Co., Ltd.).

- Crosslinker -

The crosslinker to the fluorescently labeled VHH antibody (NHS-bipyridyl disulfide crosslinker) was changed from 3-(2-pyridyldithio)propionamide-PEG4-NHS ("NHS-PEG4-SPDP" manufactured by Tokyo Chemical Industry Co., Ltd.) to 3-(2-pyridyldithio)propionamide-PEG4-NHS (manufactured by Thermo Fisher SCIENTIFIC Inc., product number "26128", "NHS-PEG4-SPDP").

- Metallic Nanoparticle -

The metallic nanoparticle was changed from ""AGCB50-1M" manufactured by nanoComposix, diameter: 50 nm, $OD_{455}$ = 0.1" to ""AGCB80-1M" manufactured by nanoComposix, diameter: 80 nm, $OD_{455}$ = 0.1".

(Preparation of Composite)

C Reactive Protein ("00-AGN-AP-CRP-00" manufactured by ADVY CHEMICAL) (hereinafter, also referred to as a CRP antigen) as a test substance was added to a phosphate buffer solution of the nanoparticle of Example 2, and the mixture was stirredat room temperature for 5 minutes using a small rotary incubator ("RT-30 mini" manufactured by TAITEC Corporation) to prepare a measurement sample containing a sandwich type composite. As described above, the nanoparticle of Example 2 had a polymer layer covering the surface of the metallic nanoparticle. The polymer constituting the polymer layer had a primary ammonium group as a positively charged group and a pyridyl group as a hydrophobic group at the terminal of a side chain, and a binding site with a sulfur atom interposing therein between the polymer and the surface of the metallic nanoparticle.

(Taking of SEM image)

An SEM image (100 K magnifications) of the composite in the obtained measurement sample of Example 2 was taken using a scanning electron microscope ("Regulus 8220" manufactured by Hitachi High-Tech Corporation). Fig. 15 is shown an SEM image of the composite prepared using the nanoparticle of Example 2. In the SEM image obtained as shown in Fig. 15, the separation distance L1 between the metallic nanoparticles of the nanoparticle in the composite and the thickness T1 of the polymer layer other than between the metallic nanoparticles of the nanoparticle were measured and compared. Note that the thickness T1 of the polymer layer was the thickness of the polymer layer at a non-shrinking portion that was not a target of the separation distance.

As a result, the separation distance L1 was smaller than the thickness ($T1 \times 2$) equivalent to a double of the thickness of each polymer layer. Therefore, it has been found that in the composite prepared from the nanoparticle composition of Example 2, the polymer layer shrank, and two metallic nanoparticles in the composite were closer to each other than a separation distance equivalent to a double of the thickness of each polymer layer ($T1 \times 2$). This has strongly suggested that the plasmonic enhancement effect was further obtained and the fluorescence intensity was further enhanced.

[Reference Example 1: Shrinkability of Inorganic Layer]

In order to clarify the technical significance of the shrinkability of the polymer layer shown in Example 2, an agglomerate of metallic nanoparticles coated with an inorganic layer (Reference Example 1) was examined as a comparative object.

(Preparation of Measurement Sample)

A silica-coated silver nanoparticle (manufactured by nanoComposix, particle diameter of silver nanoparticle (core particle diameter): 50 nm, thickness of silica layer: 20 nm) was diluted with water to prepare an aqueous dispersion of a silica-coated silver nanoparticle. The resulting aqueous dispersion was used as a measurement sample. Specifically, in the measurement sample, an agglomerate in which two particles were agglomerated was also present in addition to the primary particle state of the silica-coated silver nanoparticle. The agglomerate was found and evaluated.

In the same manner as in Example 2, an SEM image (500 K magnifications) was taken, and the thickness T2 of the inorganic layer in the agglomerate and the separation distance L2 between two metallic nanoparticles were measured and compared from the SEM image. As a result, the separation distance L2 was approximately twice the thickness T2 of the inorganic layer. Note that the thickness T2 of the inorganic layer was the thickness of the inorganic layer at a portion that was not a target of the separation distance.

[Example 3: Preparation of Various Nanoparticle Compositions]

(Polymer layer-Coated Silver Nanoparticle)

A nanoparticle composition illustrated in Fig. 16 was prepared. Poly-L-lysine ("3075" manufactured by Peptide Institute, Inc.) as a polymer was added to 1 mL of a dispersion of a silver nanoparticle ("AGCB50-1M " manufactured by nanoComposix, diameter: 50 nm, $OD_{430}$ = 0.1) as a metallic nanoparticle, and the mixture was stirred and mixed at

room temperature (25°C) for 4 hours using a small rotary incubator ("RT-30 mini" manufactured by TAITEC Corporation). As a result, a dispersion of silver nanoparticles coated with a polymer layer (polymer layer-coated silver nanoparticles) was obtained. The polymer layer comprises a binding site with a sulfur atom interposing therein on the surface of the silver nanoparticle, a hydrophobic group that forms a hydrophobic bond with the surface of the silver nanoparticle, a positively charged group (primary ammonium group) that forms an electrostatic bond with the surface of the silver nanoparticle, and an electrically neutral group (primary amino group). The polymer constituting the polymer layer has a binding site with a sulfur atom interposing therein on the surface of the silver nanoparticle, a hydrophobic group that forms a hydrophobic bond with the surface of the silver nanoparticle, a positively charged group (primary ammonium group) that forms an electrostatic bond with the surface of the silver nanoparticle, and an electrically neutral group (primary amino group).

(Fluorescently Labeled Polymer layer-Coated Silver Nanoparticle)

A succinimidyl ester ("Alexa Fluor (registered trademark) 430 NHS ester" manufactured by Thermo Fisher SCIENTIFIC Inc.) as a fluorescent substance was added to 1 mL of the dispersion of the polymer layer-coated silver nanoparticle, and the mixture was stirred and mixed at room temperature for 1 hour using a small rotary incubator ("RT-30 mini" manufactured by TAITEC Corporation). As a result, a dispersion of a polymer layer-coated silver nanoparticle with the fluorescent substance bonded to the polymer layer (hereinafter, also referred to as a fluorescently labeled polymer layer-coated silver nanoparticle) was obtained.

To 1 mL of the dispersion of the fluorescently labeled polymer layer-coated silver nanoparticle was further added 0.1% of sodium polyglutamate ("P4886" manufactured by Sigma-Aldrich Co. LLC, molecular weight: 50,000 to 100,000) as a polyanion-based polymer, and the mixture was stirred and mixed at room temperature overnight using a small rotary incubator ("RT-30 mini" manufactured by TAITEC Corporation). As a result, a nanoparticle composition (solvent: pure water) comprising the fluorescently labeled polymer layer-coated silver nanoparticle (that is, a nanoparticle) and sodium polyglutamate was obtained.

(Taking of SEM image)

An SEM image (200,000 magnifications) of the nanoparticle in the nanoparticle composition obtained was taken using a scanning electron microscope ("Regulus 8220" manufactured by Hitachi High-Tech Corporation). As a result of SEM image observation, it was confirmed that the nanoparticles were dispersed independently in the nanoparticle composition. Further, it has been confirmed from the SEM image that the surface of the silver nanoparticle in the nanoparticle was continuously coated with the polymer layer. In addition, the thickness of the polymer layer covering the silver nanoparticle was measured from the SEM image. The thickness of the polymer layer was about 11 nm.

(Measurement of Absorption Spectrum)

The nanoparticle composition of Example 3 was put into a measurement container. The measurement container was placed in an ultraviolet-visible spectrophotometer ("infinite M200 PRO" manufactured by TECAN Japan Co., Ltd.), and an absorption spectrum was measured. The measurement condition was a measurement wavelength range of 400 to 600 nm. Fig. 17 shows the absorption spectrum of the nanoparticle composition of Example 3. The absorption spectrum obtained had a peak at 455 nm.

(Measurement of Fluorescence Spectrum)

The nanoparticle composition of Example 3 was placed in a measurement container having an optical path length of 1 cm. The measurement container was placed in a fluorometer ("infinite M200 PRO" manufactured by TECAN Japan Co., Ltd.), and a fluorescence spectrum was measured. The measurement conditions were a wavelength of excitation light of 430 nm and a detection wavelength of 470 to 700 nm. Fig. 18 shows the fluorescence spectrum of the nanoparticle composition of Example 3. The fluorescence spectrum obtained had a peak at 535 nm.

[Example 4: Nanoparticle Composition Including Nanoparticle in which Linker is Bonded to Polymer layer]

A method for producing a nanoparticle composition comprising a nanoparticle will be described with reference to Fig. 19. Fig. 19 is a schematic diagram for explaining a method for producing a nanoparticle composition comprising a nanoparticle. A dispersion of a polymer layer-coated silver nanoparticle was prepared in the same manner as in Example 1. To 1 mL of this dispersion were simultaneously added a succinimidyl ester ("Alexa Fluor (registered trademark) 430 NHS ester" manufactured by Thermo Fisher SCIENTIFIC Inc.) as a fluorescent substance and SM(PEG)6 (PEGylated, long-chain SMCC crosslinker) ("22105" manufactured by Thermo Fisher SCIENTIFIC Inc.) as a crosslinker, and the mixture was stirred and mixedat room temperature for 4 hours using a small rotary incubator ("RT-30 mini" manufactured by TAITEC Corporation). As a result, a dispersion of a polymer layer-coated silver nanoparticle with a fluorescent substance and a linker bonded thereto was obtained. Further, sodium heparin ("081-00136" manufactured by FUJIFILM Wako Pure Chemical Corporation) was added as a polyanion-based polymer, and the mixture was stirred and mixedat room temperature for 4 hours using a small rotary incubator ("RT-30 mini" manufactured by TAITEC Corporation). As a result, a nanoparticle composition comprising the polymer layer-coated silver nanoparticle (nanoparticle) with a fluorescent substance and a linker bonded thereto and the polyanion-based polymer was prepared.

(Measurement of Absorption Spectrum and Fluorescence Spectrum)

For the nanoparticle obtained, an absorption spectrum was measured in the same manner as in Example 1. Fig. 20(a) shows the absorption spectrum of the nanoparticle composition of Example 4. The absorption spectrum obtained had a peak at 450 nm.

In addition, for the nanoparticle composition obtained, a fluorescence spectrum was measured in the same manner as in Example 1. Fig. 20(b) shows the fluorescence spectrum of the nanoparticle composition of Example 4. The fluorescence spectrum obtained had a peak at 540 nm. From the fact that the maximum wavelength of the absorption spectrum was not shifted toward a longer wavelength, it was confirmed that the nanoparticle was not agglomerated, and from the fact that fluorescence could be detected, it was confirmed that the fluorescent substance was bonded to the nanoparticle.

[Example 5: Evaluation of Dispersibility of Nanoparticle by Polyanion-Based Polymer]

(Preparation of Nanoparticle Composition)

Poly-L-lysine ("3075" manufactured by Peptide Institute, Inc.) was added to 1 mL of a dispersion of a silver nanoparticle ("AGCB80-1M" manufactured by nanoComposix, diameter: 80 nm, $OD_{455}$ = 0.1) as a metallic nanoparticle, and the mixture was stirred and mixed at room temperature overnight using a small rotary incubator ("RT-30 mini" manufactured by TAITEC Corporation). As a result, a dispersion of silver nanoparticles coated with a polymer layer (polymer layer-coated silver nanoparticles) was obtained.

The polymer layer has a hydrophobic group (alkylene group) that forms a hydrophobic bond with the surface of the silver nanoparticle, and an electrically neutral group (primary amino group) that can form an electrostatic bond with an anionic group of the polyanion-based polymer. That is, the polymer constituting the polymer layer has a hydrophobic group (alkylene group) that forms a hydrophobic bond with the surface of the silver nanoparticle, and an electrically neutral group (primary amino group) that can form an electrostatic bond with an anionic group of the polyanion-based polymer.

To 1 mL of the dispersion of the polymer layer-coated silver nanoparticle was added sodium polyglutamate ("P4886" manufactured by Sigma-Aldrich Co. LLC, molecular weight: 50,000 to 100,000) as a polyanion-based polymer, and the mixture was stirred and mixed at room temperature for 4 hours using a small rotary incubator ("RT-30 mini" manufactured by TAITEC Corporation). As a result, a nanoparticle composition comprising the polymer layer-coated silver nanoparticle and the polyanion-based polymer was obtained.

(Measurement of Size of Associated Body)

As measurement samples, the nanoparticle composition of Example 4 (composition not subjected to washing treatment (hereinafter, also referred to as a zero-time washed composition)) and a composition subjected to washing treatment twice (hereinafter, also referred to as a twice washed composition) were prepared. The washing treatment refers to a series of treatment in which pure water is added as a solvent such that the concentration of the nanoparticle in the composition is 1/2, the mixture is vigorously stirred, the nanoparticle is precipitated at 12,000 G for 30 minutes using at least a centrifuge (centrifugal separator) ("MX307" manufactured by TOMY Seiko Co., Ltd.) after the stirring and before the measurement, the supernatant is removed, and pure water is newly added. Where this series of operation is carried out once, it is defined that the number of washing treatment is 1. When the washing treatment was performed three times, aggregation occurred and the recovery rate was significantly reduced.

The size (particle diameter) of an associated body in each of the two measurement samples prepared above was measured using a dynamic light scattering measuring apparatus ("ZETA SIZER Nanoseries nano-ZS" manufactured by MALVERN PANALYTICAL). Fig. 21 includes diagrams illustrating the frequency distribution of size. Fig. 21(a) shows the frequency distribution of size when the number of times of washing treatment is 0, and Fig. 21(b) shows the frequency distribution of size when the number of times of washing is 2. In Fig. 21, the horizontal axis represents the size (nit: nm,

logarithmic scale), and the vertical axis represents the scattering intensity ratio (SID) (unit: none).

As shown in Figs. 21(a) and 21(b), the frequency distribution of size had a shape having one peak in the vicinity of 100 nm when the number of times of washing treatment was 0, and had a broadened shape having a peak in a region larger than 100 nm when the number of times of washing treatment was 2 (the twice washed composition). From the result that the peak of the frequency distribution was shifted toward a larger size side and the frequency distribution was broadened by the washing treatment as described above, it is considered that as the number of times of washing treatment increased from 0 to 2, measurement objects having an increased size were generated. That is, as illustrated in Figs. 23(a) and 23(b), it is strongly suggested that the number of polyanion-based polymers surrounding the nanoparticle is gradually reduced through the washing treatment with a solvent (more specifically, pure water), so that electrostatic repulsive force between the associated bodies become difficult to act, nanoparticles agglomerate to form an agglomerate, and the size of the agglomerate gradually increases (see the change from Fig. 23(a) to Fig. 23(b)). In Fig. 23, the specifically bondable substance and the fluorescent substance in the nanoparticle are omitted for convenience.

In view of the above, such behavior of the frequency distribution of the size with respect to the number of times of washing treatment strongly suggests the presence of associated bodies in which the polyanion-based polymer associates to surround the nanoparticle in the nanoparticle composition.

(Measurement of Zeta Potential of Associated Body)

Two measurement samples were prepared in the same manner as in the size measurement of an associated body. The zeta potential of the associated body in each of the two measurement samples prepared above was measured using a zeta potential analyzer ("ZETA SIZER Nanoseries nano-ZS" manufactured by MALVERN PANALYTICAL). Fig. 22 includes diagrams illustrating the frequency distribution of zeta potential. Fig. 22(a) shows the frequency distribution of zeta potential when the number of times of washing treatment is 0, and Fig. 22(b) shows the frequency distribution of zeta potential when the number of times of washing is 2. In Fig. 22, the horizontal axis represents zeta potential (unit: mV), and the vertical axis represents frequency (unit: arbitrary unit).

As shown in Figs. 22(a) and 22(b), as to the frequency distribution of zeta potential, the peak of the frequency distribution was present in a region with negative potential when the number of times of washing treatment was 0, and the peak of the frequency distribution was present in a region with positive potential when the number of times of washing treatment was 2. This result of the change in the potential of the peak of the frequency distribution before and after the washing treatment (negative potential $\rightarrow$ positive potential) strongly suggests that the number of negatively charged polyanion-based polymers surrounding a nanoparticle gradually decreases as the number of washing treatment increases from 0 to 2, and a positively charged polymer layer is exposed (see: change from Fig. 23(a) to Fig. 23(b)).

In view of the above, such behavior of the frequency distribution of the zeta potential with respect to the number of times of washing treatment strongly suggests the presence of associated bodies in which the polyanion-based polymer associates to surround the nanoparticle in the nanoparticle composition.

INDUSTRIAL APPLICABILITY

The nanoparticle and the nanoparticle composition according to the present embodiment can be used for detection of a specific test substance in a specimen using a plasmon-excited fluorescence analysis method.

EXPLANATION OF REFERENCES

| | |
|---|---|
| 1 | Nanoparticle |
| 2 | Metallic nanoparticle |
| 3 | Polymer layer |
| 3A | Polymer constituting polymer layer |
| 3a | Binding site with sulfur atom interposing therein |
| 3b | Positively charged group |
| 3c | Hydrophobic group |
| 4 | Specifically bondable substance |
| 7 | Polyanion-based polymer |
| 9 | Associated body |
| 10 | First nanoparticle |
| 12 | First metallic nanoparticle |
| 13 | First polymer layer |

14 First specifically bondable substance
16 First fluorescent substance
20 Second nanoparticle
22 Second metallic nanoparticle
23 Second polymer layer
24 Second specifically bondable substance
26 Second fluorescent substance
30 Test substance
40 Composite
L Separation distance (separative distance)

Second Aspect

(TITLE OF THE INVENTION) Nanoparticle and Method for Producing Same

(TECHNICAL FIELD)

The present invention relates to a nanoparticle, particularly a nanoparticle to be used for plasmon-excited fluorescence analysis, and a method for producing the same.

(BACKGROUND ART)

A biosensor makes a specific test substance to be detected specifically react with a specific specifically bondable substance to form a composite, and detects the test substance by a signal derived from a specific bond in the composite.

In plasmon-excited fluorescence analysis, the composite comprises, for example, a metallic particle, a specifically bondable substance, a fluorescent substance, and a test substance. When the composite is irradiated with excitation light, surface plasmon resonance is generated in the metallic particles in the composite, and a near field is formed in the vicinity of the surface of the metallic particle. The near field increases the fluorescence intensity of the fluorescent substance.

The composite particle for immunochromatogram described in Patent Document 1 is composed of a fine particle that has a structure in which the exterior of a fine particle made of metal is covered with at least one layer of silica containing at least one fluorescent substance, and has a surface modified with a labeling substance that specifically recognizes a target substance. That is, in the composite particle of Patent Document 1, the surface of the metallic particle is covered with the silica layer, and the fluorescent substance and the labeling substance are immobilized on the silica layer.

(PRIOR ART DOCUMENT)

(PATENT DOCUMENT)

(Patent Document 1) JP-A-2011-220705

(SUMMARY OF THE INVENTION)

(PROBLEMS TO BE SOLVED BY THE INVENTION)

Incidentally, the present inventors have found through intensive studies that the sensor as described above has room for further improvement in the detection sensitivity of a nanoparticle as described below. Specifically, in the composite particle described in Patent Document 1, a surface of a metallic particle is covered with a silica layer, and the silica layer is further modified with a fluorescent substance and a labeling substance. Therefore, in the production of a nanoparticle, the site (labeling site) of the fluorescent substance to be labeled on the silica layer competes with the site (bonding site) of the labeling substance to be bonded. As a result, it is difficult to increase the fluorescent substance in the nanoparticle as desired.

The present invention has been devised in light of the above problems. That is, a main object of the present invention is to provide a nanoparticle having a novel form of labeling a fluorescent substance capable of labeling more fluorescent substances, and a method for producing the nanoparticle.

(MEANS FOR SOLVING THE PROBLEMS)

A nanoparticle according to one embodiment of the present invention comprises a metallic nanoparticle, a polymer layer covering a surface of the metallic nanoparticle, and a fluorescent substance,

wherein the fluorescent substance is labeled on a surface of the metallic nanoparticle, and
the polymer layer contains a binding site with a sulfur atom interposing therein between the polymer layer and the surface of the metallic nanoparticle.

A method for producing a nanoparticle according to one embodiment of the present invention, the method comprises:
a step of mixing a polymer to which a fluorescent substance is bonded with a disulfide linkage interposing with a metallic nanoparticle to label the fluorescent substance on a surface of the metallic nanoparticle and simultaneously form a polymer layer on the surface of the metallic nanoparticle.

(EFFECTS OF THE INVENTION)

The nanoparticle according to one embodiment of the present invention has a novel form of labeling a fluorescent substance, and can label more fluorescent substances.

(BRIEF DESCRIPTION OF THE DRAWINGS)

Fig. 24 includes cross-sectional views schematically illustrating a nanoparticle according to the fourth embodiment.
Fig. 25 is an enlarged schematic view of the portion A in Fig. 24.
Fig. 26 is a reaction scheme showing one example of a method for producing the nanoparticle according to Embodiment 5.
Fig. 27 is a cross-sectional view schematically illustrating the composite according to the sixth embodiment.
Fig. 28 is a diagram schematically illustrating a measuring device according to the seventh embodiment.
Fig. 29 is a schematic diagram for explaining the method for producing the nanoparticle of Example 6.
Fig. 30 is a diagram showing the fluorescence spectrum of the nanoparticle of Example 6.
Fig. 31 is a view showing an SEM image of the composite prepared using the nanoparticle of Example 7.

(DETAILED DESCRIPTION)

Hereinafter, a nanoparticle and a method for producing the same, a composite, and a measuring device which are embodiments of the present invention will be described in detail with reference to the embodiments illustrated in drawing. Note that the drawings include some schematic ones and may not reflect actual dimensions or proportions.
The numerical ranges referred to herein are intended to include the lower and upper limits themselves, unless otherwise noted, such as "less than", "greater than" and "smaller than". For example, taking a numerical range such as 1 nm to 50 nm as an example, unless otherwise specified, the numerical range is interpreted as including the lower limit value "1 nm" and the upper limit value "50 nm".

<Fifth Embodiment: Nanoparticle>

The nanoparticle according to the fifth embodiment comprises a metallic nanoparticle, a polymer layer covering a surface of the metallic nanoparticle, and a fluorescent substance,

wherein the fluorescent substance is labeled on a surface of the metallic nanoparticle, and
the polymer layer contains a binding site with a sulfur atom interposing therein between the polymer layer and the surface of the metallic nanoparticle.

[Method for Detecting Test Substance]

First, for convenience of description of the nanoparticle according to the present embodiment, for the purpose of aiding understanding thereof, a method for detecting a test substance using the nanoparticle according to the present embodiment will be described.

The nanoparticle according to the present embodiment is useful for the detection of a test substance. The nanoparticle according to the present embodiment comprises a metallic nanoparticle, a polymer layer covering the surface of the metallic nanoparticle, and a fluorescent substance labeled on the surface of the metallic nanoparticle. The nanoparticle according to the present embodiment may further comprise a specifically bondable substance bonded to the polymer layer. The specifically bondable substance can specifically be bonded to a test substance in a specimen. Hereinafter, the nanoparticle to which the specifically bondable substance is bonded as described above is also referred to as a "specifically bonded nanoparticle (or specifically bonded nanoparticle structure or specifically bonded nanoparticle construction)".

The specifically bonded nanoparticle is dissolved or dispersed in a specimen, and a test substance contained in the specimen is captured to form a composite (this will be described in detail in the sixth embodiment). More specifically, the composite is formed by specifically bonding of the specifically bondable substance of the specifically bonded nanoparticle with the test substance. The composite has a structure (sandwich-type structure) in which two specifically bonded nanoparticles are bonded with one test substance interposing. As described above, the specifically bondable substances possessed, respectively, by the two nanoparticles in the composite bond to the same test substance, whereby the two metallic nanoparticles are disposed apart from each other at a constant distance.

In surface plasmon fluorescence spectroscopy (SPFS), irradiation of the composite with excitation light causes localized surface plasmon resonance (LSPR), so that a near field is efficiently formed near the surfaces of the metallic nanoparticles (in particular, near the surfaces located between two metallic nanoparticles). The near field efficiently excites the fluorescent substance of the composite and increases the fluorescence intensity. By measuring the fluorescence intensity, the test substance in the specimen can be detected.

[Mechanism of Action]

The nanoparticle according to the present embodiment has a novel form of labeling a fluorescent substance capable of labeling more fluorescent substances. That is, in the nanoparticle according to the present embodiment, the novel form of labeling a fluorescent substance can label more fluorescent substances.

Without being bound by a particular theory, the reason for this is presumed as follows. The nanoparticle according to the present embodiment comprises a metallic nanoparticle, a polymer layer covering the surface of the metallic nanoparticle, and a fluorescent substance labeled on the surface of the metallic nanoparticle. Since the fluorescent substance is labeled on the surface of the metallic nanoparticle, the specifically bondable substance can be bonded to a site different from the surface of the metallic nanoparticle of the nanoparticle (for example, a specific functional group of the polymer constituting the polymer layer). As described above, since the labeling site at which the fluorescent substance is labeled in the nanoparticle is different from the bonding site to which the specifically bondable substance is bonded, the number of fluorescent substances in the nanoparticle can be further increased. In view of the above, it is considered that the nanoparticle according to the present embodiment can label more fluorescent substances. Furthermore, since the nanoparticle according to the present embodiment can labeled more fluorescent substances, the nanoparticle is superior in detection sensitivity.

[Configuration of Nanoparticle]

Hereinafter, the configuration of a nanoparticle will be described. With reference to Fig. 24, the nanoparticle will be described. Fig. 24 is a cross-sectional view schematically illustrating a nanoparticle. The nanoparticle 1 according to the present embodiment comprises a metallic nanoparticle 2, a polymer layer 3 covering a surface of the metallic nanoparticle 2, and a fluorescent substance 6 labeled on the surface of the metallic nanoparticle 2 as illustrated in Fig. 24(a). As illustrated in Fig. 24(b), the nanoparticle 1 may further comprise a specifically bondable substance 4 that is bonded to a surface of the polymer layer 3 and is to be specifically bonded to a test substance in a specimen.

The nanoparticle 1 can be used for plasmon-excited fluorescence analysis. In other words, the nanoparticle 1 can be used for surface plasmon-field enhanced fluorescence spectroscopic immunoassay. The nanoparticle 1 can capture a test substance in a specimen and form a composite comprising two nanoparticles 1 and one test substance. When the composite is irradiated with excitation light, localized surface plasmon resonance occurs to form a near field. The near field increases the fluorescence intensity.

The nanoparticle 1 may be blocked with a blocking agent at a nonspecific binding site. The blocked nanoparticle 1 is inhibited from forming a nonspecific bond of the specifically bondable substance 4 to a substance other than the detection target (that is, a substance other than the test substance) to reduce the background and the false positive signal, and can improve the signal-noise ratio (SN ratio). In such a case, the detection sensitivity can be further improved. Examples of the blocking agent include proteins such as bovine serum albumin (BSA), skim milk, and casein, and chemically synthesized polymers.

When the nanoparticle 1 is present in a solvent, the dispersion of the nanoparticle 1 may further contain a

dispersant for the purpose of improving the dispersibility of the nanoparticle 1. Examples of such a dispersant include sodium heparin.

(Metallic Nanoparticle)

The metallic nanoparticle 2 is coated on the surface thereof with a polymer layer 3. The metallic nanoparticle 2 interacts with light having a specific wavelength, which varies depending on the type of metal, and cause localized surface plasmon resonance. There is a plasmon resonance peak in a range of from 400 nm to 530 nm for silver nanoparticles, and from 510 nm to 580 nm for gold nanoparticles. This depends on the particle diameter. For example, nanoparticles made of silver and having a particle diameter of 20 nm resonate with light having a wavelength of 405 nm. Nanoparticles made of gold and having a particle diameter of 20 nm resonate with light having a wavelength of 524 nm. The particle diameter (average primary particle diameter) of the metallic nanoparticles 2 is, for example, 5 nm to 100 nm. The particle diameter of the metallic nanoparticles 2 can be determined by capturing an image of the metallic nanoparticles 2 using a scanning electron microscope (SEM) or a transmission electron microscope (TEM), measuring the particle diameter of the metallic nanoparticles 2 in the image, and calculating the average value (the number of measurements: for example, at least 10) of a plurality of particle diameters.
The metallic nanoparticle 2 preferably comprises gold or silver, and more preferably comprises silver.

(Polymer layer)

The polymer layer 3 covers the surface of the metallic nanoparticle 2. The polymer layer 3 functions as a metal quenching layer. In the composite, the polymer layer 3 can make a fluorescent substance 6 place apart from the surface of the metallic nanoparticle 2 by at least the thickness of the polymer layer 3. Therefore, it is possible to inhibit the excited fluorescent substance 6 from coming into contact with the surface of the metallic nanoparticle 2 and quenching, and to inhibit a decrease in detection sensitivity. The presence of the polymer layer 3 can be confirmed by capturing an image of the nanoparticle 1 using SEM or TEM, and observing the nanoparticle 1 in the image.
The polymer layer 3 will be described with reference to Fig. 25. Fig. 25 is an enlarged view of the portion A in Fig. 24, and is an enlarged schematic diagram of the vicinity of the interface between the polymer layer 3 and the surface of the metallic nanoparticle 2 in the nanoparticle 1. The polymer layer 3 contains a binding site 3a with a sulfur atom interposing therein between the polymer layer 3 and the surface of the metallic nanoparticle 2. The polymer layer 3 may further contain at least one selected from the group consisting of a positively charged group 3b and a hydrophobic group 3c. More specifically, the polymer layer 3 may further contain, in addition to the binding site 3a with a sulfur atom interposing therein, a primary ammonium group ($-NH_3^+$) as the positively charged group 3b and a hydrophobic group 3c between the polymer layer 3 and the surface of the metallic nanoparticle 2. The binding site 3a binds between the surface of the metallic nanoparticle 2 and the polymer layer 3 with a sulfur atom interposing therebetween. The positively charged group 3b forms an electrostatic bond (ionic bond) b with the surface of the negatively charged metallic nanoparticle 2. The hydrophobic group 3c forms a hydrophobic bond c with the surface of the metallic nanoparticle 2.
All of the three bonds are relatively strong bonds with the surface of the metallic nanoparticle 2. The polymer layer 3 is stably fixed to the surface of the metallic nanoparticle 2 by the binding site 3a. Furthermore, the polymer layer 3 can be more stably fixed to the surface of the metallic nanoparticles 2 by the hydrophobic bond c and the electrostatic bond b due to the positively charged group 3b. As described above, since the polymer layer 3 is stably fixed to the surfaces of the metallic nanoparticle 2, the fluorescent substance 6 and the surface of the metallic nanoparticle 2 can be stably separated at a prescribed distance. Therefore, in the present embodiment, quenching of the excited fluorescent substance is inhibited, and a decrease in detection sensitivity can be inhibited.
Furthermore, since the polymer layer 3 can comprise the polymer 3A, the polymer layer 3 is easily chemically modified as compared with a silica layer, and the necessity for surface modification or the like is low. As a result, the thickness of the polymer layer can be made smaller than that of a silica layer, and the distance between metallic nanoparticles 2 in the composite can be moderately reduced. Therefore, a near field is formed more efficiently, and the detection sensitivity can be further improved.
As illustrated in Fig. 25, the polymer 3A that can constitute the polymer layer 3 can contain, in addition to the binding site 3a with a sulfur atom interposing therein, at least one selected from the group consisting of a positively charged group 3b and a hydrophobic group 3c between the polymer 3A and the surface of the metallic nanoparticles 2. The presence of the binding site 3a with a sulfur atom interposing therein, the positively charged group 3b, and the hydrophobic group 3c can be confirmed by measuring signals derived therefrom using infrared spectroscopy and nuclear magnetic resonance spectroscopy.

- Binding Site with Sulfur Atom Interposing Therein-

The binding site 3a with a sulfur atom interposing therein is formed, for example, by mixing a polymer having a moiety containing a disulfide linkage as a side chain with a metallic nanoparticle 2.
The polymer layer 3 may be directly bonded to a surface of the metallic nanoparticle 2, or may be indirectly bonded to the surface of the metallic nanoparticle 2 with interposition of a linker portion (more specifically, $SM(PEG)_n$ wherein n is 4, 6, 8 or the like) derived from a crosslinker. Examples of such a crosslinker include an amino group-sulfhydryl group crosslinker (more specifically, an NHS (N-hydroxysuccinimide)-maleimide group crosslinker or the like).

- Positively Charged Group -

The positively charged group 3b can form a relatively strong electrostatic bond b with the surface of the metallic nanoparticle 2. In the present description, the positively charged group 3b is a group having a valence of one or more and completely positively ionized. In consideration of a plurality of positively charged groups 3b contained in the polymer constituting the polymer layer 3, the positively charged groups 3b refer to groups having a pKa of 7 or more, the pKa being represented by the following expression (1):
(Expression 1)

$$pKa = pH - log\frac{[B]}{[BH^+]} \cdot \cdot \cdot \quad (1)$$

in the expression (1), pKa represents a pKa of a group that is an electrically neutral group contained in the polymer 3A constituting the polymer layer 3 and can become a positively charged group (specifically, a primary ammonium group ($-NH_3^+$) or the like) 3b when positively charged (this type of group is also referred to as an electrically neutral group) (more specifically, a primary amino group ($-NH_2$) or the like); pH represents a pH of an environment where a test substance is detected (more specifically, a specimen or the like); B represents an electrically neutral group contained in the polymer 3A; and $BH^+$ represents a positively charged group 3b contained in the polymer 3A. The positively charged group 3b refers to a group having a concentration ($[BH^+]$) of the positively charged group 3b that is 10 times or more larger than the concentration ([B)) of the electrically neutral group in the case where the electrically neutral group of the polymer 3A constituting the polymer layer 3 and the positively charged group 3b are forming an equilibrium state represented by the following chemical equilibrium formula (2)
(Chemical Formula 1)

$$B + H^+ \rightleftarrows BH^+ \ldots \qquad (2)$$

in an environment where the electrically neutral group and the positively charged group detect a test substance (for example, a specimen having a pH of about 6 to 8).
The positively charged group 3b is preferably at least one selected from the group consisting of a primary ammonium group, a secondary ammonium group, a tertiary ammonium group, a quaternary ammonium group, and a guanidyl group ($-NHC(=NH_2^+)NH_2$).

- Hydrophobic Group -

The hydrophobic group 3c can form a hydrophobic bond c with the surface of the metallic nanoparticle 2.
The hydrophobic group is, for example, at least one selected from the group consisting of an aromatic cyclic group, an aliphatic cyclic group, and an aliphatic chain group.
Examples of the aromatic cyclic group include an aromatic carbocyclic group and an aromatic heterocyclic group. The aromatic carbocyclic group is a group which does not contain any aromatic heterocyclic ring and contains an aromatic ring in which all ring atoms are carbon atoms. Examples of the aromatic carbocyclic group include aryl groups (more specifically, a phenyl group and the like) and arylalkyl groups (more specifically, a benzyl group and the like). The aromatic heterocyclic group is a group containing an aromatic ring in which at least one of the ring atoms is a hetero atom (more specifically, an oxygen atom, a sulfur atom, a nitrogen atom, or the like). Examples of the aromatic heterocyclic group include nitrogen-containing aromatic heterocyclic groups (more specifically, a pyridyl group (pyridinyl group), and the like.), sulfur-containing aromatic heterocyclic groups, and oxygen-containing aromatic heterocyclic groups.
The aliphatic cyclic group is a group which does not contain any aromatic ring and contains a cyclic group composed of a non-aromatic ring. Examples of the aliphatic cyclic group include an aliphatic carbocyclic group and an

aliphatic heterocyclic group. The aliphatic carbocyclic group is a group containing a non-aromatic ring in which all ring atoms are carbon atoms, and examples thereof include a cycloalkyl group. An aliphatic heterocyclic group is a group containing a non-aromatic ring in which at least one of the ring atoms is a heteroatom.

The aliphatic chain group is a chain (more specifically, linear or branched) group containing no aromatic ring and no non-aromatic ring. Examples of the aliphatic chain group include aliphatic carbon chain groups (more specifically, an alkyl group, an alkylene group, and the like) and aliphatic heterochain groups. The alkyl group is, for example, a butyl group. The alkylene group is, for example, a n-butylene group.

The polymer 3A constituting the polymer layer 3 may be directly bonded to the surface of the metallic nanoparticle 2, or may be indirectly bonded to the surface of the metallic nanoparticle 2 with interposition of a linker portion (more specifically, $SM(PEG)_n$ wherein n is 4, 6, 8 or the like) derived from a crosslinker. Examples of such a crosslinker include an amino group-sulfhydryl group crosslinker (more specifically, an NHS-maleimide group crosslinker or the like).

The thickness of the polymer layer 3 is preferably 1 nm to 50 nm, and more preferably 1 nm to 10 nm. When the thickness of the polymer layer 3 is 50 nm or less, a separative distance (separation distance) with which a near field is efficiently formed in a space between two metallic nanoparticles 2 is obtained, and thus detection sensitivity is further improved. When the thickness of the polymer layer 3 is 1 nm or more, the metallic nanoparticle 2 and a fluorescent substance are disposed at a prescribed distance, so that quenching of a fluorescent substance excited in measurement is inhibited, and detection sensitivity is further improved.

In the present description, the separative distance (separation distance) refers to the minimum value (shortest distance) of the distance between the surfaces of the metallic nanoparticles contained in two nanoparticles bound with a test substance interposing therebetween in a composite.

(Fluorescent Substance)

The fluorescent substance 6 is labeled on the surface of the metallic nanoparticle 2. The labeling site of the fluorescent substance 6 is the surface of the metallic nanoparticle 2. On the other hand, the bonding site of the specifically bondable substance 4 is the polymer layer 3 as described later. As described above, in the nanoparticle 1 according to the present embodiment, the labeling site of the fluorescent substance 6 is different from the bonding site of the specifically bondable substance 4. Therefore, the number of fluorescent substances 6 can be sufficiently increased as compared with a nanoparticle whose labeling site and bonding site are the same. Therefore, the present embodiment results in further superior detection sensitivity.

The fluorescent substance 6 contains a binding site with a sulfur atom interposing therein between the fluorescent substance 6 and the surface of the metallic nanoparticle 2 as illustrated in Fig. 25, for example. In this case, the fluorescent substance 6 is bonded to the surface of the metallic nanoparticle 2 with a sulfur atom interposing therebetween. Such a bond can be formed, for example, by mixing the metallic nanoparticle 2 as a starting material with the fluorescent substance 6 having a disulfide linkage.

From the viewpoint of inhibiting fluorescence quenching, the fluorescent substance 6 is preferably labeled on the surface of the metallic nanoparticle 2 with a linker portion (for example, an alkylene group containing an amide group) interposing therebetween. When the fluorescent substance 6 is labeled on the surface of the metallic nanoparticle 2 with the linker portion interposing, the linker portion is located between the fluorescent substance 6 and the surface of the metallic nanoparticle 2, and an excited fluorescent substance 6 is easily prevented from coming into contact with the surface of the metallic nanoparticle 2. This is considered to contribute to further inhibition of fluorescence quenching.

The fluorescent substance 6 is excited by a near field formed by localized surface plasmon resonance and emits fluorescence. Examples of the fluorescent substance include complexes (metal complexes) of metals such as europium and ruthenium. Examples of a ruthenium complex include tris(bipyridine) ruthenium(II) which can have a counter anion.

The fluorescent substance 6 preferably has a large Stokes shift. Here, the Stokes shift is a difference between an absorption peak wavelength (maximum excitation wavelength) in an absorption spectrum of the fluorescent substance 6 and a fluorescence peak wavelength (maximum fluorescence wavelength) in a fluorescence spectrum of the fluorescent substance 6. When the Stokes shift of the fluorescent substance 6 is large, the absorption spectrum and the fluorescence spectrum hardly overlap each other and (scattered light of) excitation light hardly enters the fluorescence to be detected, so that more accurate fluorescence intensity can be measured.

Preferably, the fluorescence spectrum of the fluorescent substance 6 is sharp. When the fluorescence spectrum is sharp, the fluorescence spectrum hardly overlaps the absorption spectrum of the fluorescent substance 6, and thus (scattered light of) excitation light hardly enters the fluorescence to be detected, so that fluorescence intensity can be measured more accurately.

(Specifically Bondable Substance)

The nanoparticle 1 may further comprise a specifically bondable substance 4. The specifically bondable substance 4 is a nanosized (having a size of 3 to 15 nm at the longest) substance that is to be specifically bonded to a test substance (described in the sixth embodiment) in a specimen.

In the present embodiment, it has already been described that since labeling with more fluorescent substances 6 can be attained, the detection sensitivity is further superior. Although this is a description focusing on the fluorescent substance 6, the viewpoint will be changed to focus on the specifically bondable substance 4. In the present embodiment, the bonding site of the specifically bondable substance 4 in the nanoparticle 1 is different from the labeling site with the fluorescent substance 6. Therefore, for the same reason as that with the fluorescent substance 6, it is possible to increase the number of specifically bondable substances 4 in the nanoparticle 1. Therefore, it is considered that to the nanoparticle 1 according to the present embodiment can be bonded a larger number of specifically bondable substances 4.

By bonding a larger number of specifically bondable substances 4, the detection sensitivity can be further improved. Without being bound by a particular theory, the reason is presumed as follows. When a specifically bonded nanoparticle is dissolved or dispersed in a specimen, the specifically bonded nanoparticle collides with (meets) a test substance in the specimen. Through the collision, the specifically bonded nanoparticle and the test substance form a bond (specific bond) at their specific sites, and as a result, a composite is formed.

Here, it is considered that a specific bond is formed at a certain ratio with respect to the number of collisions between the specifically bonded nanoparticle and the test substance. This is because when the specifically bonded nanoparticle and the test substance collide with each other, they do not necessarily have an arrangement relationship advantageous for forming the specific bond. That is, in order to form a specific bond, specific sites of the specifically bonded nanoparticle and the test substance need to be in contact with each other or at least close to each other at the time of the collision. For example, when the specifically bondable substance is an antibody and the test substance is an antigen, the specific sites are an antigen binding site of the antibody and an antigenic determinant or an epitope of the antigen.

In consideration of such a matter, if more specifically bondable substances 4 are bonded to the specifically bonded nanoparticle, the probability of contact and close approach between specific sites in the collision increases. Therefore, since the nanoparticle according to the present embodiment can bind more specifically bondable substances 4, it is considered that the nanoparticle easily form a composite, and can further improve the detection sensitivity.

The specifically bondable substance 4 is at least one selected from the group consisting of an antibody (hereinafter, referred to as a nanoantibody), a ligand, an enzyme, and a nucleic acid strand (more specifically, a DNA strand and an RNA strand). In the present embodiment, the nanoparticle 1 in which such a specifically bondable substance 4 is bonded is further superior in detection sensitivity. For example, the nanoantibody as the specifically bondable substance 4 is specifically bonded to an antigen as a test substance at the tip portion (antigen binding site) of the nanoantibody through an antigen-antibody reaction to form a composite. The ligand as the specifically bondable substance 4 forms a composite by specifically protein-ligand bonding to a protein as a test substance through a ligand receptor reaction. A nucleic acid strand as the specifically bondable substance 4 forms a pair (double strand) of a nucleic acid strand and another nucleic acid strand in a complementary relationship on the basis of the complementarity of a base pair. An enzyme as the specifically bondable substance 4 forms an enzyme-substrate composite with a substrate as a test substance on the basis of the substrate specificity (stereospecificity) at the active moiety (active center) of the enzyme. These specific bonds are non-covalent bonds, for example, hydrogen bonds and bonds caused by intermolecular force, hydrophobic interaction, and charge interaction.

The nanoantibody is, for example, at least one selected from the group consisting of VHH (variable domain of heavy chain antibody) antibodies, fragmented antibodies (more specifically, Fab (fragment antigen binding) antibodies, and so on), and variants thereof. A VHH antibody is a single domain antibody. The variant is an antibody in which a part of an amino acid sequence is recombined or an antibody in which a substituent is introduced, as long as the variant has specific binding to an antigen. When the nanoantibody is at least one selected from the group consisting of VHH antibodies, fragmented antibodies, and variants thereof, because of relatively small volumes of these nanoantibodies, it is possible to reduce the distance (separation distance) between two metallic nanoparticles 2 in a composite, more efficiently form a near field, and further increase the fluorescence intensity.

The molecular mass of the nanoantibody is preferably 60,000 Da or less, more preferably 30,000 Da or less, and still more preferably 20,000 Da or less. When the molecular mass is 60,000 Da or less (in particular, 30,000 Da or less, or 20,000 Da or less), since the volume of the nanoantibody is relatively small, it is possible to reduce the separation distance in a composite to efficiently form a near field and further increase the fluorescence intensity. Examples of the method for measuring the molecular mass include electrophoresis (SDS-PAGE), gel filtration chromatography, and static light scattering.

The specifically bondable substance 4 may be directly bonded to the polymer layer 3, or may be indirectly bonded to the polymer layer 3 with interposition of a linker portion (more specifically, $SM(PEG)_n$ wherein n is 4, 6, 8 or

the like) derived from a crosslinker. Examples of such a crosslinker include an amino group-sulfhydryl group crosslinker (more specifically, an NHS-maleimide group crosslinker or the like).

[Method for Producing Nanoparticle]

The method for producing the nanoparticle 1 according to the fifth embodiment comprises a step of mixing a polymer to which a fluorescent substance 6 is bonded with a disulfide linkage interposing with a metallic nanoparticle 2 to label the fluorescent substance 6 onto a surface of the metallic nanoparticle 2 and simultaneously form a polymer layer 3 on the surface of the metallic nanoparticle 2 (this step is hereinafter also referred to as a "fluorescent labeling and film formation step").

The method for producing the nanoparticle 1 according to the fifth embodiment comprises a fluorescent labeling and film formation step in which fluorescent labeling to the surface of the metallic nanoparticle and polymer layer formation can be carried out in parallel. Therefore, the process of fluorescent labeling can be simplified and the cost can be reduced as compared with the conventional technique. In addition, in the present production method, since the fluorescent substance 6 can be labeled at a site different from the specifically bondable substance 4, the fluorescent labeling density in the nanoparticle 1 can be increased.

Hereinafter, one example of a method for producing the nanoparticle 1 according to the present embodiment will be described.

The method for producing the nanoparticle 1 may further comprise a fluorescent substance thiolation step, a polymer fluorescent labeling step, and a specifically bondable substance bonding step in addition to the fluorescent labeling and layer formation step.

With reference to Fig. 26, the method for producing the nanoparticle 1 will be described. Fig. 26 is a reaction scheme showing one example of a method for producing the nanoparticle 1.

(Fluorescent Substance Thiolation Step)

In the fluorescent substance thiolation step, the fluorescent substance 6 is thiolated. More specifically, as expressed by the reaction formula (R-1) in the reaction scheme, (A) a fluorescent substance having an ester linkage (hereinafter, also referred to as a fluorescent substance) is reacted with (B) a thiol having an amino group (hereinafter, also referred to as aminoalkanethiol) to synthesize (C) a fluorescent substance thiolated by forming an amide linkage (hereinafter, also referred to as thiolated fluorescent substance).

The reaction represented by the reaction formula (R-1) (hereinafter, also referred to as reaction (R-1)) can proceed, for example, in a buffer solution. The reaction ratio (molar ratio) of the fluorescent substance (A) to the aminoalkanethiol (B) is, for example, 2:1 to 1:2. In the reaction (R-1), the reaction temperature is, for example, 20 to 30°C. The reaction time is, for example, 0.5 to 2 hours. The reaction (R-1) can also be carried out under stirring conditions.

(A) $R^1$ in the fluorescent substance represents an alkylene group. Examples of the alkylene group include alkylene groups having 2 to 5 carbon atoms (more specifically, an ethylene group, a propylene group, a butylene group, and a pentylene group). $T^1$ in the fluorescent substance (A) represents a terminal group. Examples of the terminal group $T^1$ include an NHS group.

(B) Examples of the aminoalkanethiol include aminoethanethiol (cysteamine). $R^2$ in the aminoalkanethiol (B) represents an alkylene group. The alkylene group represented by $R^2$ is the same as the alkylene group represented by $R^1$ described above. The aminoalkanethiol (B) may be in the form of a salt. Examples of such a salt include hydrochloride of aminoalkanethiol (more specifically, cysteamine hydrochloride or the like).

(C) $R^1$ and $R^2$ in the thiolated fluorescent substance are the same as $R^1$ in the fluorescent substance (A) and $R^2$ in the aminoalkanethiol (B), respectively.

(Polymer Fluorescent Labeling Step)

In the polymer fluorescent labeling step, a polymer is fluorescently labeled. More specifically, in the reaction scheme, as represented by the reaction formula (R-2), the thiolated fluorescent substance (C) is reacted with (D) a polymer having a disulfide linkage (hereinafter, also referred to as a polymer) to synthesize a polymer labeled with (E) a fluorescent substance (hereinafter, also referred to as a fluorescently labeled polymer).

In the reaction represented by the reaction formula (R-2) (hereinafter, also referred to as reaction (R-2)), the molar ratio of the thiolated fluorescent substance (C) to the disulfide linkage of the polymer (D) is, for example, 3:1 to 1:1. The reaction temperature is, for example, 30 to 40°C. The reaction time is, for example, 0.5 to 2 hours. The reaction (R-2) can be carried out under stirring conditions.

(Fluorescent Labeling and Layer Formation Step)

In the fluorescent labeling and layer formation step, (E) the fluorescently labeled polymer (the polymer to which the fluorescent substance 6 is bonded with a disulfide linkage interposing) and (F) the metallic nanoparticle 2 are mixed, whereby the fluorescent substance 6 is labeled onto the surface of the metallic nanoparticle 2 and simultaneously the polymer layer 3 is formed on the surface of the metallic nanoparticle 2. That is, in the fluorescent labeling and layer formation step, the labeling of the fluorescent substance 6 onto the surface of (F) the metallic nanoparticle 2 and the formation of the polymer layer 3 on the surface of (F) the metallic nanoparticle 2 can be carried out in parallel. Since the fluorescent labeling and the layer formation can be carried out not in two steps but in one step as described above, this step is preferable from the viewpoint of cost reduction. The polymer layer 3 formed contains a binding site 3a with a sulfur atom interposing therein between the polymer layer 3 and the surface of (F) the metallic nanoparticle 2. In the reaction represented by the reaction formula (R-3) (hereinafter, also referred to as reaction (R-3)) in Fig. 26, there is shown an enlarged schematic view of the vicinity of the interface between the polymer layer 3 and the surface of the metallic nanoparticle 2 of the nanoparticle 1 as a product of the reaction.

In the reaction (R-3), the reaction time is, for example, 12 to 36 hours. The reaction temperature is, for example, 20 to 30°C. The reaction (R-3) can be carried out under stirring conditions. In the reaction (R-3), for the purpose of labeling the fluorescent substance 6 as much as possible on the surface of (F) the metallic nanoparticle 2, the fluorescently labeled polymer (E) is excessively added to the surface of (F) the metallic nanoparticle 2 until the reaction (R-3) substantially no longer proceeds.

Through the fluorescent substance thiolation step, the polymer fluorescent labeling step, and the fluorescent labeling and layer formation step, it is possible to produce the nanoparticle 1 comprising the metallic nanoparticle 2, the polymer layer 3 covering the surface of the metallic nanoparticle 2, and the fluorescent substance 6 labeled on the surface of the metallic nanoparticle 2.

(Specifically Bondable Substance Bonding Step)

In the specifically bondable substance bonding step, the specifically bondable substance 4 is bonded to the polymer layer 3. The bonding site of the polymer layer 3 that is bonded to the specifically bondable substance 4 is, for example, a functional group of the fluorescently labeled polymer (E). Such a functional group is, for example, at least one functional group selected from the group consisting of an amino group, a carboxyl group, a thiol group, an N-hydroxysuccinimide group (NHS group), and a maleimide group.

Through the specifically bondable substance bonding step in addition to the fluorescent substance thiolation step, the polymer fluorescent labeling step, and the fluorescent labeling and layer formation step, it is possible to produce the nanoparticle 1 comprising the metallic nanoparticle 2, the polymer layer 3 covering the surface of the metallic nanoparticle 2, the fluorescent substance 6 labeled on the surface of the metallic nanoparticle 2, and the specifically bondable substance 4 bonded to the polymer layer 3.

The production method of the present embodiment may, as necessary, further comprise an optional step (more specifically, a purification step of performing purification by removing impurities) in addition to the step of carrying out the reactions (R-1) to (R-3).

<Sixth Embodiment: Composite>

The composite will be described with reference to Fig. 27. Fig. 27 is a cross-sectional view schematically illustrating the composite. The composite 40 comprises a test substance 30 to be detected and two nanoparticles 10 and 20. In the composite 40, the two nanoparticles 10 and 20 are bound with the test substance 30 interposing therebetween. That is, the nanoparticles 10 and 20 according to the fifth embodiment form the composite according to the sixth embodiment bound with the test substance 30 interposing. Of the two nanoparticles 10 and 20, one is referred to as a first nanoparticle 10, and the other is referred to as a second nanoparticle 20. As described above, the composite 40 includes the first nanoparticle 10 and the second nanoparticle 20 as the nanoparticle 1.

In the composite 40, the first nanoparticle 10 comprises a first metallic nanoparticle 12 as the metallic nanoparticle, a first polymer layer 13 as the polymer layer, and a first fluorescent substance 16 as the fluorescent substance, and the second nanoparticle 20 comprises a second metallic nanoparticle 22 as the metallic nanoparticle, a second polymer layer 23 as the polymer layer, and a second fluorescent substance 26 as the fluorescent substance. The fluorescent substances 16 and 26 are labeled on at least one of a surface of the first metallic nanoparticle 12 and a surface of the second metallic nanoparticle 22.

Specifically, in the composite 40, the first nanoparticle 10 comprises the first metallic nanoparticle 12, the first polymer layer 13 covering the surface of the first metallic nanoparticle 12, and the first fluorescent substance 16 labeled on the surface of the first metallic nanoparticle 12. The second nanoparticle 20 comprises the second metallic nanoparticle

22, the second polymer layer 23 covering the surface of the second metallic nanoparticle 22, and the second fluorescent substance 26 labeled on the surface of the second metallic nanoparticle 22.

The first nanoparticle 10 further comprises a first specifically bondable substance 14 as the specifically bondable substance, and the second nanoparticle 20 further comprises a second specifically bondable substance 24 as the specifically bondable substance.

Specifically, the first nanoparticle 10 further comprises the first specifically bondable substance 14 bonded to the first polymer layer 13, and the second nanoparticle 20 further comprises the second specifically bondable substance 24 bonded to the second polymer layer 23.

From the viewpoint of further enhancing the fluorescence intensity, the separation distance L is preferable to be as small as possible as long as excited fluorescent substances 16 and 26 are hardly quenched. More specifically, in a preferred embodiment, the two nanoparticles 10 and 20 in the composite 40 are close to each other. In a more preferred embodiment, the two nanoparticles 10, 20 are close to each other such that the first polymer layer 13 of the first nanoparticle 10 and the second polymer layer 23 of the second nanoparticle 20 in the composite 40 are in contact with each other. In a still more preferred embodiment, the two nanoparticles 10 and 20 are close to each other in such a manner that the first polymer layer 13 of the first nanoparticle 10 and the second polymer layer 23 of the second nanoparticle 20 in the composite 40 come in contact with each other with at least one of the polymer layers shrinking.

In a further preferred embodiment, in the case where the polymer layers 13 and 23 come into contact with each other with at least one of the polymer layers shrinking, for example, in the composite 40 illustrated in Fig. 27, it is considered to be possible to make at least one of the test substance 30, the specifically bondable substances 14 and 24 bonded to the test substance 30, and the fluorescent substances 16 and 26 to be embedded in the polymer layers 13 and 23. In a still further preferred embodiment, in the case where the polymer layers 13 and 23 are in contact with each other, for example, in the composite 40 illustrated in Fig. 27, it is considered to be possible, similarly to the further preferred embodiment described above, to make at least one of the test substance 30, the specifically bondable substances 14 and 24 that are bonded to the test substance 30, and the fluorescent substances 16 and 26 to be embedded in the polymer layers 13 and 23.

In the present embodiment, since the layers covering the surfaces of the metallic nanoparticles 12 and 22 are the polymer layers 13 and 23, the fluorescence intensity can be enhanced. The reason for this is presumed as follows. The layers covering the surfaces of the metallic nanoparticles 12 and 22 are the polymer layers 13 and 23, and the polymer layers 13 and 23 have relatively high flexibility as compared with an inorganic layer comprising an inorganic oxide. For this reason, in the composite 40, the polymer layers 13 and 23 can shrink, so that the two metallic nanoparticles 12 and 22 can be closer to each other than a distance equivalent to a double of the thickness of each polymer layer (the thickness of the polymer layer 13 + the thickness of the polymer layer 23). That is, since the layers covering the surfaces of the metallic nanoparticles 12 and 22 are the polymer layers 13 and 23, the separation distance L can be less than a double of the thickness of each polymer layer. As a result, the plasmonic enhancement effect is easily obtained, and the fluorescence intensity is further enhanced. In the present description, the thickness of the polymer layer in "a double of the thickness of each polymer layer" is not the thickness of the polymer layer 13 or 23 at the shrinking portion, which serves as the target of the separation distance, but is the thickness of the polymer layer 13 or 23 at a non-shrinking portion, which does not serve as the target of the separation (for example, $T_1$ in Fig. 31 to be described later).

In the present embodiment, the polymer layers 13 and 23 contain a binding site 3a with a sulfur atom interposing therein between the polymer layers and the surfaces of the metallic nanoparticles. For example, the polymer 3A constituting the polymer layers 13 and 23 contains a binding site 3a with a sulfur atom interposing therein. As a result, the fluorescence intensity can be further enhanced. The reason for this is presumed as follows. In such a case, it is considered that since the binding site 3a forms a bond with the surfaces of the metallic nanoparticles 12 and 22, the polymer 3A has a network structure and covers the surfaces of the metallic nanoparticles 12 and 22 in a network manner. As described above, since the polymer 3A has a network structure, the polymer 3A further has relatively high flexibility. For this reason, in the composite 40, the polymer layers 13 and 23 can further shrink, so that the two metallic nanoparticles 12 and 22 can be closer to each other than a distance equivalent to a double of the thickness of each polymer layer. Therefore, in the present embodiment, the separation distance L can be less than a double of the thickness of each polymer layer, the plasmonic enhancement effect is further obtained, and the fluorescence intensity is further enhanced.

In a preferred embodiment, the polymer layers 13 and 23 each further contain, between the polymer layer and the surface of the metallic nanoparticle, at least one selected from the group consisting of a positively charged group 3b and a hydrophobic group 3c in addition to the binding site 3a with a sulfur atom interposing therein. As a result, the fluorescence intensity is further enhanced. The reason for this is presumed as follows. In such a case, since at least one selected from the group consisting of the positively charged group 3b and the hydrophobic group 3c forms a bond with the surfaces of the metallic nanoparticles 12 and 22, it is considered that the polymer 3A has a network structure and covers the surfaces of the metallic nanoparticles 12 and 22 in a network shape. As described above, since the polymer 3A has a network structure, the polymer 3A further has relatively high flexibility. For this reason, in the composite 40, the polymer layers 13 and 23 can further shrink, so that the two metallic nanoparticles 12 and 22 can be closer to

each other than a distance equivalent to a double of the thickness of each polymer layer. Therefore, in the present embodiment, the separation distance L can be less than a double of the thickness of each polymer layer, the plasmonic enhancement effect is further obtained, and the fluorescence intensity is further enhanced.

In a preferred embodiment, the polymer 3A constituting the polymer layers 13 and 23 contains a binding site 3a with a sulfur atom interposing therein on a side chain (more specifically, a side chain terminal) thereof. In a preferred embodiment, the fluorescence intensity can be further enhanced. The reason for this is presumed as follows. In such a case, the binding site 3a forms a bond with the surfaces of the metallic nanoparticles 12 and 22. For this reason, it is considered that the polymer 3A has a network structure and covers the surfaces of the metallic nanoparticles 12 and 22 in a network manner with side chains thereof used as binding sites. As described above, since the polymer 3A has a network structure, the polymer 3A further has relatively high flexibility. For this reason, in the composite 40, the polymer layers 13 and 23 can further shrink, so that the two metallic nanoparticles 12 and 22 can be closer to each other than a distance equivalent to a double of the thickness of each polymer layer. Therefore, in the present embodiment, the separation distance L can be less than a double of the thickness of each polymer layer, the plasmonic enhancement effect is further obtained, and the fluorescence intensity is further enhanced.

In a preferred embodiment, the polymer 3A constituting the polymer layers 13 and 23 contains at least one selected from the group consisting of the positively charged group 3b and the hydrophobic group 3c on a side chain (more specifically, a side chain terminal) of the polymer. In a preferred embodiment, the fluorescence intensity can be further enhanced. The reason for this is presumed as follows. In such a case, the positively charged group 3b and/or the hydrophobic group 3c forms a bond with the surfaces of the metallic nanoparticles 12 and 22. For this reason, it is considered that the polymer 3A has a network structure and covers the surfaces of the metallic nanoparticles 12 and 22 in a network manner with side chains thereof used as binding sites. As described above, since the polymer 3A has a network structure, the polymer 3A further has relatively high flexibility. For this reason, in the composite 40, the polymer layers 13 and 23 can further shrink, so that the two metallic nanoparticles 12 and 22 can be closer to each other than a distance equivalent to a double of the thickness of each polymer layer. Therefore, in the present embodiment, the separation distance L can be less than a double of the thickness of each polymer layer, the plasmonic enhancement effect is further obtained, and the fluorescence intensity is further enhanced.

The separation distance L between the first nanoparticle 10 and the second nanoparticle 20 is, for example, 12 nm to 52 nm, and preferably 12 nm to 27 nm. The separation distance L is a distance between the first metallic nanoparticle 12 and the second metallic nanoparticle 22, and is a distance with which a line segment connecting between a first point P1 on the surface of the first nanoparticle 10 and a second point P2 on the surface of the second nanoparticle 20 is minimized. In a case where the separation distance L is 52 nm or less, when the composite 40 is irradiated with excitation light, a near field occurs more efficiently in a space near the surfaces between the first and second metallic nanoparticles 12 and 22, so that the fluorescence intensity can be further enhanced.

The layers covering the surfaces of the metallic nanoparticles 12 and 22 are the polymer layers 13 and 23, and the polymer layers 13 and 23 each contain a binding site 3a with a sulfur atom interposing therein. Therefore, as described above, the separation distance L can be smaller than the distance equivalent to a double of the thickness of each polymer layer covering the surfaces of the two metallic nanoparticles 12 and 22 in the composite 40. For example, when the thicknesses of the polymer layers 13 and 23 are 5 nm, the separation distance L can be less than 10 nm (more specifically, 2 to 9 nm, 3 to 8 nm, 4 to 7 nm, or the like).

(Fluorescent Substance)

Preferably, as shown in Fig. 27, the fluorescent substances 16 and 26 are at least positioned between the first metallic nanoparticle 12 and the second metallic nanoparticle 22 in the composite 40. This is because, since the near field is efficiently generated a space between the metallic nanoparticles 12 and 22, the fluorescence intensity is easily enhanced by positioning the fluorescent substances 16 and 26 in the space between the metallic nanoparticles 12 and 22.

(Test Substance)

The test substance 30 is a substance to be detected contained in a specimen. Examples of the test substance 30 include an antigen, a protein, a substrate, and a nucleic acid strand. The test substance 30 is specifically bonded to the specifically bondable substances 14 and 24. For example, an antigen has at least two antigenic determinants and forms specific bonding with the first and second specifically bondable substances 14 and 24 at the antigenic determinants. Examples of the antigen include proteins such as c-reactive protein, myoglobin, troponin T, troponin I, and BNP, and antigen proteins of viruses such as influenza virus and RS virus. The test substance 30 is a test substance derived from a specimen such as blood, plasma, urine, or saliva. That is, examples of the specimen containing the test substance 30 include blood, plasma, serum, urine, and saliva. The specimen may further comprise a solvent and a buffer (more specifically, phosphate-buffered saline (PBS), Tris buffer, HEPES buffer, MOPS buffer, MES buffer, or the like).

<Seventh Embodiment: Measuring Device>

The measuring device will be described with reference to Fig. 28. Fig. 28 is a diagram schematically illustrating the measuring device according to the seventh embodiment. As illustrated in Fig. 28, the measuring device 100 includes an excitation light source 110, an excitation light radiation optical system 120, a reagent container 130, a light receiving optical system 140, and a light receiving element 150.

The excitation light source 110 emits excitation light 112. The excitation light source 110 is, for example, a laser. The excitation light radiation optical system 120 performs adjustment of the cross-sectional diameter like condensing of the excitation light 112, and outputs the incident excitation light 122. The excitation light radiation optical system 120 includes a lens 124 and a polarizing element (λ/2 plate) 126. The incident excitation light 122 output from the excitation light radiation optical system 120 is incident on the reagent container 130, and the measurement sample in the reagent container 130 is irradiated with the incident excitation light. The reagent container 130 is, for example, a detachable container (more specifically, a cell, a preparation, or the like) and a microchannel chip. The microchannel chip is a chip having a minute channel. When the reagent container 130 is a microchannel chip, for example, a nanoparticle (reagent) according to the fifth embodiment and a specimen can be mixed and continuously supplied. Therefore, it is not necessary to prepare a measurement sample by mixing in advance, and it is possible to continuously perform measurement.

The measurement sample irradiated with the incident excitation light 122 emits fluorescence (detection light 132). The light receiving optical system 140 is arranged in a direction perpendicular to the traveling direction of the incident excitation light 122 toward the reagent container 130. The light receiving optical system 140 adjusts the cross-sectional diameter or the like of the detection light 132 emitted from the measurement sample, and can remove scattered light of the incident excitation light 122 or adjust the amount of light. The light receiving optical system 140 includes a lens 144 and an optical filter 146. The optical filter 146 includes, for example, a band pass filter and a dichroic mirror.

Fluorescence 142 having passed through light receiving optical system 140 is detected by the light receiving element 150. The light receiving element 150 includes, for example, a PD, an APD, a PMT, a CCD camera, and a spectroscope. The light receiving element 150 can measure the amount of fluorescence of a single wavelength, measure a fluorescence spectrum, and create two-dimensional planar fluorescence imaging.

The present invention is not limited to the above-described embodiments, and can be modified in design without departing from the gist of the present invention.

In the fifth and sixth embodiments, four fluorescent substances 6, 16, and 26 are labeled on the nanoparticles 1, 10, and 20, respectively, but the present invention is not limited this configuration. For example, the number of the fluorescent substances labeled on the nanoparticle 1, 10, and 20 may be 5 or more.

In the fifth and sixth embodiments, four specifically bondable substances 4, 14, and 24 are labeled on the nanoparticles 1, 10, and 20, respectively (Fig. 24(b) and Fig. 26), but the present invention is not limited to this configuration. For example, the number of the specifically bondable substances 4, 14, and 24 labeled on the nanoparticles 1, 10, and 20 may be 5 or more.

In the seventh embodiment, the light receiving optical system 140 in the measuring device 100 is disposed in a direction perpendicular to the traveling direction of the incident excitation light 122 toward the reagent container 130, but the present invention is not limited this configuration. For example, the light receiving optical system 140 may be arranged in a direction parallel to the traveling direction of the incident excitation light 122, or may be arranged in a direction having an acute angle or an obtuse angle with respect to the traveling direction of the incident excitation light 122.

(EXAMPLES)

Hereinafter, the present invention will be described in more detail with reference to Examples. The present invention is not limited by the following Examples at all. In addition, unless otherwise specified, parts and % in the Examples are on mass basis.

In Examples, the concentration of a metallic nanoparticle in a dispersion may be expressed by absorbance. The absorbance was measured using an ultraviolet-visible spectrophotometer ("infinite M200 PRO" manufactured by TECAN Japan Co., Ltd.). Note that a subscript number attached to the notation OD of the absorbance indicates an absorption wavelength. For example, "$OD_{455}$ = 0.1" indicates that the absorbance at a wavelength of 455 nm is 0.1.

[Example 6: Preparation of Nanoparticle]

The nanoparticle of Example 6 was produced as follows.

(Introduction of Thiol Group into Ruthenium Complex Derivative)

(b) Cysteamine hydrochloride ("A0296" manufactured by Tokyo Chemical Industry Co., Ltd.) and (a) an NHS-labeled Ru complex derivative ("Ruthenium(II) tris(bipyridyl)-C5-NHS ester" manufactured by Tokyo Chemical Industry Co., Ltd.) were stirred and mixed at room temperature (for example, 25°C) for 1 hour using a small rotary incubator ("RT-30 mini " manufactured by TAITEC Corporation). As a result, a thiol group was introduced at a terminal of the ligand of the Ru complex. This synthesis reaction is a nucleophilic substitution reaction in which the primary amino group of the cysteamine hydrochloride (b) attacks the NHS ester group of the NHS-labeled Ru complex derivative (a) as expressed by the reaction formula (r-1).

(Chemical Formula 2)

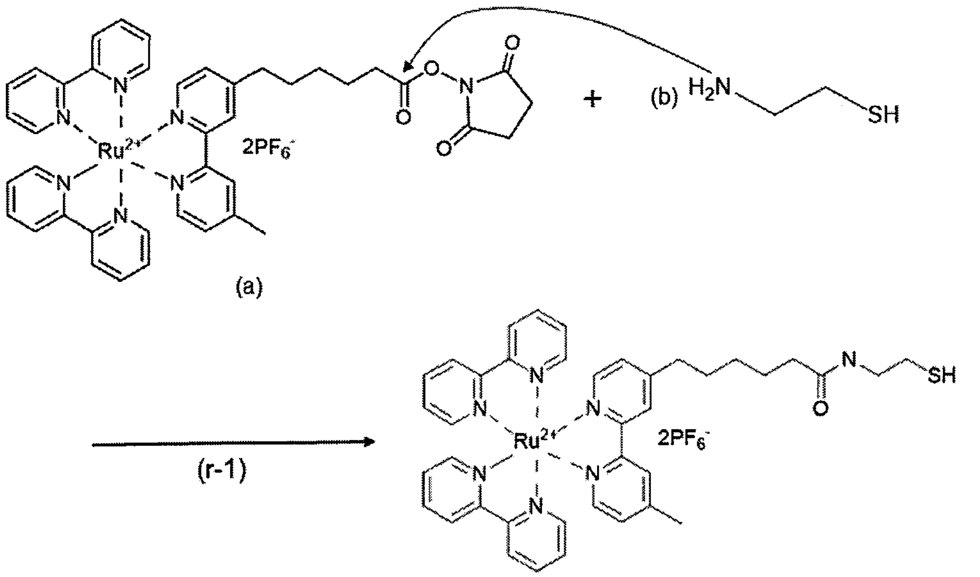


(a) (b) (c)

In this way, (c) a Ru complex derivative having a thiol group (hereinafter, also referred to as "Ru complex-SH") was prepared. The Ru complex moiety in the Ru complex-SH (c) obtained is a fluorescent substance.

(Introduction of Disulfide Linkage to Polymer)

On the other hand, poly-L-lysine ("3075 " manufactured by Peptide Institute, Inc.) and 3-(2-pyridyldithio)propionamide-PEG4-NHS (manufactured by Thermo Fisher SCIENTIFIC Inc., serial number "26128", "NHS-PEG4-SPDP") were stirred and mixed at room temperature for 4 hours using a small rotary incubator ("RT-30 mini" manufactured by TAITEC Corporation). As a result, (d) a polymer having a pyridyl group (pyridinyl group) on a side chain with an SPDP linker and a disulfide linkage interposing therebetween (hereinafter, also referred to as a "polymer having a disulfide group") was obtained. This synthesis reaction is a nucleophilic substitution reaction in which a primary amino group of poly-L-lysine attacks the NHS ester group of 3-(2-pyridyldithio)propionamide-PEG4-NHS as expressed by the reaction formula (r-4).

(Chemical Formula 3)

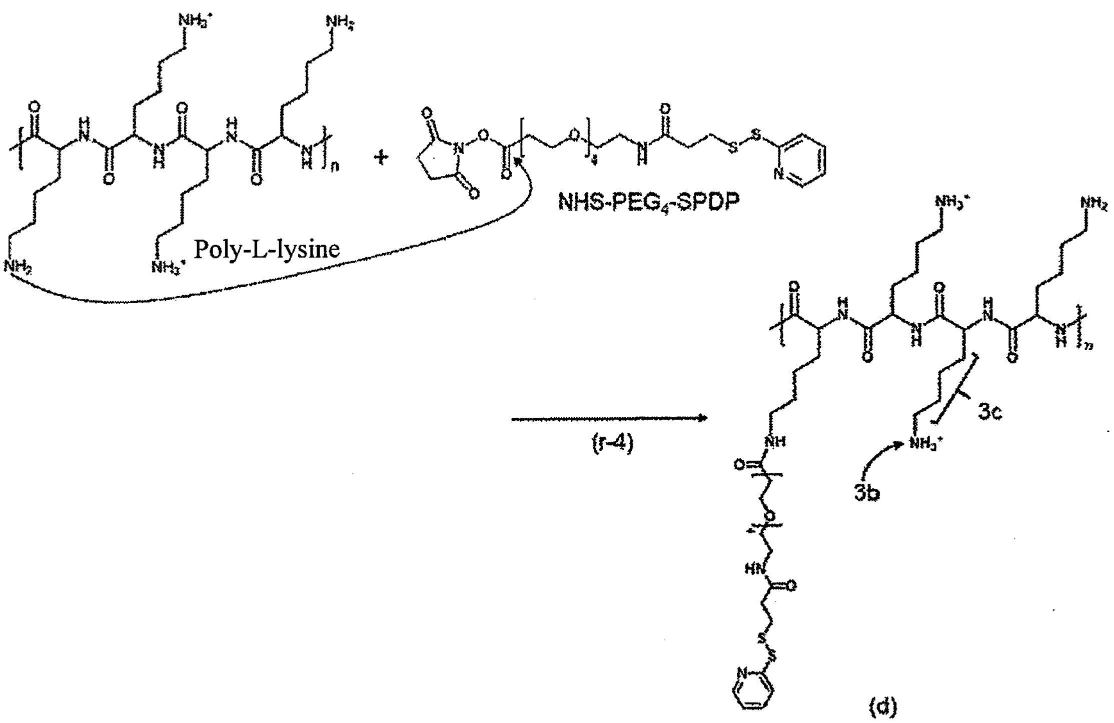


(Introduction of Ruthenium Complex Derivative to Polymer)

The Ru complex-SH (c) and the polymer having a disulfide group (d) were stirred and mixed at 37°C for 1 hour. As a result, (e) a polymer in which a Ru complex was attached to a side chain with an SPDP linker and a disulfide linkage interposing therebetween (hereinafter, also referred to as "Ru complex-labeled polymer") was obtained. The Ru complex-labeled polymer (e) synthesized had a hydrophobic group (n-butylene group) and a positively charged group (primary ammonium group). This synthesis reaction is a thiol nucleophilic reaction in which the thiol group of the Ru complex-SH (c) attacks the disulfide group of the polymer having a disulfide group (d) as shown in the reaction formula (r-2).

(Chemical Formula 4)

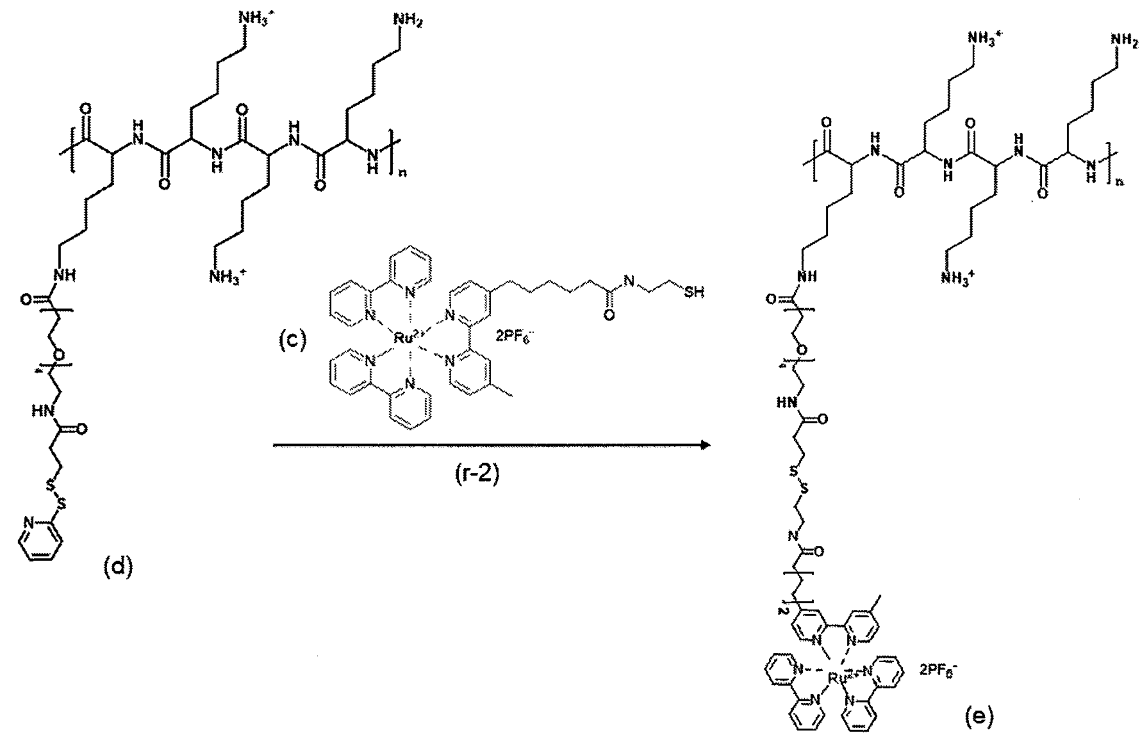


(Labeling with Fluorescent Substance and Formation of Polymer layer: Preparation of Nanoparticle)

The Ru complex-labeled polymer (e) obtained was added to 1 mL of a dispersion of silver nanoparticles ("AGCB80-1M" manufactured by nanoComposix, Inc., diameter: 80 nm, $OD_{455}$ = 0.1) as the metallic nanoparticle (f), and the mixture was stirred and mixed at room temperature overnight using a small rotary incubator ("RT-30 mini" manufactured by TAITEC Corporation). As a result, a dispersion of silver nanoparticles coated with a polymer layer and labeled with a fluorescent substance was obtained. In this reaction, the disulfide linkage of the Ru complex-labeled polymer (e) is cleaved by the reducing action of silver. The poly-L-lysine moiety generated by the cleavage and the ruthenium complex moiety are each bonded to the surface of the silver nanoparticle with a sulfur atom interposing therebetween. As a result, as shown in Fig. 29, the Ru complex as a fluorescent substance is labeled on the surface of the silver nanoparticle with a sulfur atom interposing therebetween, and the polymer layer is bonded to the surface of the silver nanoparticle with the sulfur atom interposing therebetween. That is, the labeling with the fluorescent substance and the formation of the polymer layer proceed in parallel. Fig. 29 is a schematic diagram for explaining the method for producing the nanoparticle of Example 6.

[Measurement Method and Evaluation Method]

It was confirmed, by the following method, that the nanoparticle of Example 6 was labeled with a fluorescent substance. The nanoparticle obtained was placed in a measurement container. The measurement container was placed in a fluorometer ("infinite M200 PRO" manufactured by TECAN Japan Co., Ltd.), and a fluorescence spectrum was measured. The measurement conditions were a wavelength of excitation light of 430 nm and a detection wavelength of 490 to 700 nm. The fluorescence spectrum measured is shown in Figure 30.

Fig. 30 shows a fluorescence spectrum (horizontal axis: fluorescence wavelength (unit: nm) and vertical axis: fluorescence intensity (unit: arbitrary unit)) of the nanoparticle of Example 6. The fluorescence spectrum obtained had a peak at about 620 nm. The shape of the fluorescence spectrum almost matched the shape of the fluorescence spectrum of the Ru complex. From this result, it was confirmed that the Ru complex was labeled on the nanoparticle of Example 6.

[Example 7: Preparation of Nanoparticle]

In the preparation of a nanoparticle of Example 7, first, a nanoparticle was prepared by labeling a fluorescent substance onto the polymer layer of a polymer layer-coated silver nanoparticle. Next, a polyanion-based polymer was

added to the nanoparticle to prepare a nanoparticle composition containing the nanoparticle of Example 7.

(Polymer layer-Coated Silver Nanoparticle)

Poly-L-lysine ("3075" manufactured by Peptide Institute, Inc.) and 3-(2-pyridyldithio)propionamide-PEG4-NHS (manufactured by Thermo Fisher SCIENTIFIC Inc., product number "26128", "NHS-PEG4-SPDP") were stirred and mixed at room temperature for 4 hours using a small rotary incubator ("RT-30 mini" manufactured by TAITEC Corporation). As a result, a polymer was obtained. This synthesis reaction is a nucleophilic substitution reaction in which a primary amino group of poly-L-lysine attacks the NHS ester group of 3-(2-pyridyldithio)propionamide-PEG4-NHS. The polymer obtained was added to 1 mL of a dispersion of a silver nanoparticle ("AGCB80-1M" manufactured by nanoComposix, diameter: 80 nm, $OD_{430}$ = 0.1) as a metallic nanoparticle, and the mixture was stirred and mixed at room temperature overnight using a small rotary incubator ("RT-30 mini" manufactured by TAITEC Corporation). As a result, a dispersion of silver nanoparticles coated with a polymer layer (hereinafter, also referred to as polymer layer-coated silver nanoparticles) was obtained.

(Bonding of Crosslinker to Polymer layer-Coated Silver Nanoparticle)

Next, to 1 mL of the prepared dispersion of a polymer layer-coated silver nanoparticle were further added a crosslinker SM(PEG)6 (PEGylated, long-chain SMCC crosslinker) ("22105" manufactured by Thermo Fisher SCIENTIFIC Inc.) and sodium heparin (" 081-00136 " manufactured by FUJIFILM Wako Pure Chemical Corporation), and the mixture was stirred and mixed at room temperature for 1 hour using a small rotary incubator ("RT-30 mini" manufactured by TAITEC Corporation). As a result, a dispersion of a silver nanoparticle coated with a polymer layer to which a crosslinker SM(PEG)6 is bonded (hereinafter, also referred to as a polymer layer-coated silver nanoparticles to which an SM(PEG)6 linker is bonded) was obtained. The SM(PEG)6 linker bonded to the polymer layer-coated silver nanoparticle had a maleimide group.

(Fluorescently Labeling to VHH Antibody)

An NHS-labeled Ru complex derivative ("Ruthenium(II) tris(bipyridyl)-C5-NHS ester" manufactured by Tokyo Chemical Industry Co., Ltd.) was added to 100 μg of a VHH antibody (manufactured by RePHAGEN Co., Ltd., molecular mass: 18,000 Da), and the mixture was stirred and mixed at room temperature for 1 hour using a small rotary incubator ("RT-30 mini" manufactured by TAITEC Corporation). As a result, a VHH antibody with a fluorescent substance bonded thereto (hereinafter, also referred to as a fluorescently labeled VHH antibody) was obtained.

(Bonding of Crosslinker to Fluorescently Labeled VHH Antibody)

Subsequently, 3-(2-pyridyldithio)propionamide-PEG4-NHS (manufactured by Thermo Fisher SCIENTIFIC Inc., product number "26128", "NHS-PEG4-SPDP") as an NHS-bipyridyl disulfide crosslinker was added to the fluorescently labeled VHH antibody in a molar ratio of 8 times equivalent, and the mixture was stirred and mixed at room temperature for 1 hour using a small rotary incubator ("RT-30 mini" manufactured by TAITEC Corporation). As a result, a VHH antibody to which a fluorescent substance and an SPDP linker were bonded (hereinafter, also referred to as a fluorescently labeled VHH antibody with an SPDP linker bonded thereto) was obtained.

(Thiolation of Fluorescently Labeled VHH Antibody with Crosslinker Bonded Thereto)

Next, to the fluorescently labeled VHH antibody with an SPDP linker bonded thereto, a reducing agent TCEP ("77720" manufactured by Thermo Fisher SCIENTIFIC Inc.) was added in a molar ratio of 2 times equivalent, and the mixture was stirred and mixed at 37°C for 1 hour using a stirrer ("TS-100" manufactured by BioSan). As a result, a VHH antibody to which a fluorescent substance and an SPDP linker having been reduced (hereinafter, also referred to as a reduced SPDP linker) were bonded (hereinafter, also referred to as a fluorescently labeled VHH antibody with a reduced SPDP linker bonded thereto) was obtained. The reduced SPDP linker had a thiol group (-SH group) generated through reduction of a disulfide linkage.

(Bonding of Fluorescently Labeled VHH Antibody to Silver Nanoparticle)

Subsequently, the fluorescently labeled VHH antibody with a reduced SPDP linker bonded thereto was added to a dispersion ($OD_{430}$ = 0.1) of a polymer layer-coated silver nanoparticle with a maleimide group-containing SM(PEG)6 linker bonded thereto, and the mixture was stirred and mixed at room temperature overnight using a small rotary incubator

("RT-30 mini" manufactured by TAITEC Corporation). As a result, the maleimide group of the SM(PEG)6 linker reacted with the thiol group of the reduced SPDP linker, affording a dispersion of a silver nanoparticle to which a fluorescently labeled VHH antibody was bond with a linker moiety interposing (a nanoparticle composition). The resulting nanoparticle composition contained the nanoparticle of Example 7 and a solvent containing sodium heparin as a polyanion-based polymer.

[Shrinkability of Polymer layer]

(Preparation of Composite)

65 μL of a phosphate buffer solution ("PBS-T" manufactured by FUJIFILM Wako Pure Chemical Corporation) and C Reactive Protein ("00-AGN-AP-CRP-00" manufactured by ADVY CHEMICAL) (hereinafter, also referred to as a CRP antigen) as a test substance were added to 35 μL of a dispersion of the obtained nanoparticle composition, and the mixture was stirred at room temperature for 5 minutes using a small rotary incubator ("RT-30 mini" manufactured by TAITEC Corporation) to prepare a measurement sample containing a sandwich type composite.

(Taking of SEM image)

An SEM image (100 K magnifications) of the composite in the obtained measurement sample of Example 7 was taken using a scanning electron microscope ("Regulus 8220" manufactured by Hitachi High-Tech Corporation). Fig. 31 is shown an SEM image of the composite prepared using the nanoparticle of Example 7. In the SEM image obtained as shown in Fig. 31, the separation distance Li between the metallic nanoparticles of the nanoparticle in the composite and the thickness $T_1$ of the polymer layer other than between the metallic nanoparticles of the nanoparticle were measured and compared. Note that the thickness $T_1$ of the polymer layer was the thickness of the polymer layer at a non-shrinking portion that was not a target of the separation distance.

As a result, the separation distance Li was smaller than the thickness ($T_1 \times 2$) equivalent to a double of the thickness of each polymer layer. Therefore, it has been found that in the composite prepared from the nanoparticle of Example 7, the polymer layer shrank, and two metallic nanoparticles in the composite were closer to each other than a separation distance equivalent to a double of the thickness of each polymer layer ($T_1 \times 2$). This has strongly suggested that the plasmonic enhancement effect was further obtained and the fluorescence intensity was further enhanced.

[Reference Example 1: Shrinkability of Inorganic Layer]

In order to clarify the technical significance of the shrinkability of the polymer layer shown in Example 7, an agglomerate of metallic nanoparticles coated with an inorganic layer (Reference Example 1) was examined as a comparative object.

(Preparation of Measurement Sample)

A silica-coated silver nanoparticle (manufactured by nanoComposix, particle diameter of silver nanoparticle (core particle diameter): 50 nm, thickness of silica layer: 20 nm) was diluted with water to prepare an aqueous dispersion of a silica-coated silver nanoparticle. The resulting aqueous dispersion was used as a measurement sample. Specifically, in the measurement sample, an agglomerate in which two particles were agglomerated was also present in addition to the primary particle state of the silica-coated silver nanoparticle. The agglomerate was found and evaluated.

In the same manner as in Example 7, an SEM image (500 K magnifications) was taken, and the thickness $T_2$ of the inorganic layer in the agglomerate and the separation distance $L_2$ between two metallic nanoparticles were measured and compared from the SEM image. As a result, the separation distance $L_2$ was approximately twice the thickness $T_2$ of the inorganic layer. Note that the thickness $T_2$ of the inorganic layer was the thickness of the inorganic layer at a portion that was not a target of the separation distance.

(EXPLANATION OF REFERENCES)

| | |
|---|---|
| 1 | Nanoparticle |
| 2 | Metallic nanoparticle |
| 3 | Polymer layer |
| 3A | Polymer constituting polymer layer |

3a Binding site with sulfur atom interposing therein
3b Positively charged group
3c Hydrophobic group
4 Specifically bondable substance
6 Fluorescent substance
10 First nanoparticle
12 First metallic nanoparticle
13 First polymer layer
14 First specifically bondable substance
16 First fluorescent substance
20 Second nanoparticle
22 Second metallic nanoparticle
23 Second polymer layer
24 Second specifically bondable substance
26 Second fluorescent substance
30 Test substance
40 Composite
L Separation distance (separative distance)

Embodiments according to the present invention also include the following embodiments.

[1] A nanoparticle comprising a metallic nanoparticle, a polymer layer covering a surface of the metallic nanoparticle, and a nanosized specifically bondable substance that is bonded to a surface of the polymer layer and is to be specifically bonded to a test substance in a specimen,
wherein the polymer layer contains at least one selected from the group consisting of a binding site with a sulfur atom interposing therein, a positively charged group, and a hydrophobic group between the polymer layer and a surface of the metallic nanoparticle.
[2] The nanoparticle according to [1], wherein a polymer constituting the polymer layer contains at least one selected from the group consisting of the binding site, the positively charged group, and the hydrophobic group in a side chain thereof.
[3] The nanoparticle according to [1] or [2], wherein the polymer layer contains at least the binding site with the sulfur atom interposing therein.
[4] The nanoparticle according to any one of [1] to [3], wherein the polymer layer contains at least the positively charged group.
[5] The nanoparticle according to [4], wherein the positively charged group is at least one selected from the group consisting of a primary ammonium group, a secondary ammonium group, a tertiary ammonium group, a quaternary ammonium group, and a guanidyl group (-NHC(=NH2+)NH2).
[6] The nanoparticle according to any one of [1] to [5], wherein the polymer layer contains at least the hydrophobic group.
[7] The nanoparticle according to [6], wherein the hydrophobic group is at least one selected from the group consisting of an aromatic cyclic group, an aliphatic cyclic group, and an aliphatic chain group.
[8] The nanoparticle according to one of [1] to [7], wherein the polymer constituting the polymer layer has a moiety containing a disulfide linkage as a side chain.
[9] The nanoparticle according to [8] dependent on [3] or [4], wherein a moiety containing the disulfide linkage has the positively charged group.
[10] The nanoparticle according to claim 8 dependent on claim 5 or 6, wherein the moiety containing the disulfide linkage has the hydrophobic group.
[11] The nanoparticle according to any one of [1] to [10], wherein the polymer layer has a thickness of 1 nm to 10 nm.
[12] The nanoparticle according to any one of [1] to [11], which is a nanoparticle to be used for plasmon-excited fluorescence analysis.
[13] The nanoparticle according to any one of [1] to [12], wherein the specifically bondable substance is a nanoantibody.
[14] The nanoparticle according to any one of [1] to [13], wherein the specifically bondable substance is a VHH antibody.
[15] The nanoparticle according to any one of [1] to [14], wherein the metallic nanoparticle comprises gold or silver.
[16] The nanoparticle according to any one of [1] to [15], wherein a fluorescent substance is labeled on at least one of a surface of the polymer layer and the specifically bondable substance.
[17] The nanoparticle according to any one of [1] to [16],

wherein a first nanoparticle and a second nanoparticle are contained as the nanoparticle;
the first nanoparticle comprises a first metallic nanoparticle as the metallic nanoparticle, a first polymer layer as the polymer layer, and a first specifically bondable substance as the specifically bondable substance;
the second nanoparticle comprises a second metallic nanoparticle as the metallic nanoparticle, a second polymer layer as the polymer layer, and a second specifically bondable substance as the specifically bondable substance; and
the fluorescent substance is labeled on at least one combination of the first polymer layer and the second polymer layer, and the first specifically bondable substance and the second specifically bondable substance.

[18] The nanoparticle according to [17], wherein a composite in which the first nanoparticle and the second nanoparticle are bound with the test substance interposing therebetween is formed.
[19] The nanoparticle according to [18], wherein the test substance is a test substance derived from the specimen that is blood, plasma, urine, or saliva.
[20] The nanoparticle according to [18] or [19], in the composite, the fluorescent substance is positioned between the first nanoparticle and the second nanoparticle.

Embodiments according to the present invention also include the following embodiments.

[1] A nanoparticle comprising a metallic nanoparticle, a polymer layer covering a surface of the metallic nanoparticle, and a fluorescent substance,

wherein the fluorescent substance is labeled on a surface of the metallic nanoparticle, and
the polymer layer comprises a binding site with a sulfur atom interposing therein between the polymer layer and the surface of the metallic nanoparticle.

[2] The nanoparticle according to [1], wherein a polymer constituting the polymer layer contains the binding site in a side chain thereof.
[3] The nanoparticle according to [1] or [2], wherein the fluorescent substance contains a binding site with a sulfur atom interposing therein between the fluorescent substance and the surface of the metallic nanoparticle.
[4] The nanoparticle according to any one of [1] to [3], further comprising a specifically bondable substance that is bonded to the polymer layer and is to be specifically bonded to a test substance in a specimen.
[5] The nanoparticle according to any one of [1] to [4], wherein the polymer layer further contains at least one selected from the group consisting of a positively charged group and a hydrophobic group.
[6] The nanoparticle according to [5], wherein the polymer constituting the polymer layer further contains at least one selected from the group consisting of the positively charged group and the hydrophobic group in a side chain thereof.
[7] The nanoparticle according to [5], wherein the polymer layer comprises at least the positively charged group, and the positively charged group is at least one selected from the group consisting of a primary ammonium group, a secondary ammonium group, a tertiary ammonium group, a quaternary ammonium group, and a guanidyl group (-NHC(=NH2+)NH2).
[8] The nanoparticle according to [5], wherein the polymer layer contains at least the hydrophobic group, and
the hydrophobic group is at least one selected from the group consisting of an aromatic cyclic group, an aliphatic cyclic group, and an aliphatic chain group.
[9] The nanoparticle according to any one of [1] to [8], wherein the polymer layer has a thickness of 1 nm to 10 nm.
[10] The nanoparticle according to any one of [1] to [9], which is a nanoparticle to be used for plasmon-excited fluorescence analysis.
[11] The nanoparticle according to any one of [5] to [10] dependent on [4], wherein the specifically bondable substance is at least one selected from the group consisting of a nanoantibody, a ligand, an enzyme, and a nucleic acid strand.
[12] The nanoparticle according to any one of [5] to [11] dependent on [4], wherein the specifically bondable substance is at least one nanoantibody selected from the group consisting of a VHH antibody, a fragmented antibody, and variants thereof.
[13] The nanoparticle according to any one of claims 1 to 12, wherein the metallic nanoparticle comprises gold or silver.
[14] The nanoparticle according to any one of [1] to [13],

wherein a first nanoparticle and a second nanoparticle are comprised as the nanoparticle;
the first nanoparticle comprises a first metallic nanoparticle as the metallic nanoparticle and a first polymer layer as the polymer layer;
the second nanoparticle comprises a second metallic nanoparticle as the metallic nanoparticle and a second

polymer layer as the polymer layer; and
the fluorescent substance is labeled on at least one of a surface of the first metallic nanoparticle and a surface of the second metallic nanoparticle.

[15] The nanoparticle according to [14], wherein wherein the first nanoparticle and the second nanoparticle form a composite by being bonded with each other with the test substance interposing therebetween.
[16] The nanoparticle according to [15], wherein the test substance is a test substance derived from a specimen that is blood, plasma, urine, or saliva.
[17] The nanoparticle according to [15] or [16], in the composite, at least the fluorescent substance is positioned between the first nanoparticle and the second nanoparticle.
[18] A method for producing a nanoparticle, the method comprising a step of mixing a polymer to which a fluorescent substance is bonded with a disulfide linkage interposing therebetween with a metallic nanoparticle to label the fluorescent substance onto a surface of the metallic nanoparticle and simultaneously form a polymer layer on the surface of the metallic nanoparticle.

Embodiments according to the present invention also include the following embodiments.

[1] A nanoparticle composition comprising a nanoparticle and a solvent comprising the nanoparticle,

wherein the nanoparticle comprises a metallic nanoparticle, a coating layer that covers a surface of the metallic nanoparticle, and a specifically bondable substance that is bonded to a surface of the coating layer or a surface of the metallic nanoparticle and is to be specifically bonded to a test substance in a specimen, and
the solvent contains a polyanion-based polymer in addition to the nanoparticle.

[2] The nanoparticle composition according to [1], wherein the polyanion-based polymer has at least one anionic group selected from the group consisting of a carboxylate salt group, a sulfate salt group, a sulfonate salt group, a nitrate salt group, a phosphate salt group, and a borate salt group.
[3] The nanoparticle composition according to [1] or [2], wherein the polyanion-based polymer is at least one selected from the group consisting of polyglutamic acid, heparin, polyaspartic acid, polyacrylic acid, salts thereof, and DNA.
[4] The nanoparticle composition according to any one of [1] to [3],
wherein the coating layer is a polymer layer is:

an inorganic layer comprising an inorganic oxide; or
a polymer layer comprising at least one selected from the group consisting of a binding site with a sulfur atom interposing therein, a positively charged group, and a hydrophobic group between the polymer layer and the surface of the metallic nanoparticle.

[5] The nanoparticle composition according to [4], wherein the coating layer is the polymer layer, and
a polymer constituting the polymer layer has at least one selected from the group consisting of the binding site with a sulfur atom interposing therein, the positively charged group, and the hydrophobic group at a terminal of a side chain.
[6] The nanoparticle composition according to any one of [1] to [5], wherein the specifically bondable substance is a nanoantibody.
[7] The nanoparticle composition according to any one of [1] to [6], wherein the specifically bondable substance is crosslinked with at least one selected from the group consisting of a polyalkylene ether chain and an alkyl chain.
[8] The nanoparticle composition according to any one of [1] to [7], wherein the polyanion-based polymer is present to surround individual particles of the nanoparticle.
[9] The nanoparticle composition according to any one of [1] to [8], wherein the solvent comprises an aqueous solvent.
[10] The nanoparticle composition according to any one of claims 1 to 9, comprising an associated body of the polyanion-based polymer and the nanoparticle.
[11] The nanoparticle composition according to any one of [1] to [10], which is a nanoparticle to be used for plasmon-excited fluorescence analysis.
[12] The nanoparticle composition according to any one of [1] to [11], wherein the metallic nanoparticle comprises gold or silver.
[13] The nanoparticle composition according to any one of [1] to [12], wherein a fluorescent substance is labeled on at least one of a surface of the coating layer and the specifically bondable substance.
[14] The nanoparticle composition according to any one of [1] to [13],

wherein a first nanoparticle and a second nanoparticle are comprised as the nanoparticle;

the first nanoparticle comprises a first metallic nanoparticle as the metallic nanoparticle, a first coating layer as the coating layer, and a first specifically bondable substance as the specifically bondable substance;
the second nanoparticle comprises a second metallic nanoparticle as the metallic nanoparticle, a second coating layer as the coating layer, and a second specifically bondable substance as the specifically bondable substance; and
the fluorescent substance is labeled on at least one combination of the first coating layer and the second coating layer, and the first specifically bondable substance and the second specifically bondable substance.

[15] The nanoparticle composition according to [14], wherein the first nanoparticle and the second nanoparticle form a composite by being bonded with each other with the test substance interposing therebetween.
[16] The nanoparticle composition according to [15], in the composite, the fluorescent substance is positioned between the first nanoparticle and the second nanoparticle.
[17] The nanoparticle composition according to any one of [1] to [16], wherein the test substance is a test substance derived from blood, plasma, urine, or saliva.

## Claims

1. A nanoparticle comprising a metallic nanoparticle, a polymer layer covering a surface of the metallic nanoparticle, and a nanosized specifically bondable substance that is bonded to a surface of the polymer layer and is to be specifically bonded to a test substance in a specimen.

2. The nanoparticle according to claim 1, wherein the polymer layer comprises at least one selected from the group consisting of a binding site with a sulfur atom interposing therein, a positively charged group, and a hydrophobic group between the polymer layer and a surface of the metallic nanoparticle.

3. The nanoparticle according to claim 2, further comprising a fluorescent substance,
wherein the fluorescent substance is labeled on at least one selected from the group consisting of a surface of the metallic nanoparticle, a surface of the polymer layer, and the specifically bondable substance.

4. The nanoparticle according to claim 3, wherein the fluorescent substance is labeled on a surface of the metallic nanoparticle, and
the fluorescent substance comprises a binding site with a sulfur atom interposing therein between the fluorescent substance and the surface of the metallic nanoparticle.

5. The nanoparticle according to claim 2 or 3, wherein a polymer constituting the polymer layer comprises at least one selected from the group consisting of the binding site, the positively charged group, and the hydrophobic group in a side chain thereof.

6. The nanoparticle according to claim 2 or 3, wherein the polymer layer comprises at least the binding site with the sulfur atom interposing therein.

7. The nanoparticle according to claim 2 or 3, wherein the polymer layer comprises at least the positively charged group.

8. The nanoparticle according to claim 7, wherein the positively charged group is at least one selected from the group consisting of a primary ammonium group, a secondary ammonium group, a tertiary ammonium group, a quaternary ammonium group, and a guanidyl group ($-NHC(=NH_2^+)NH_2$).

9. The nanoparticle according to claim 2 or 3, wherein the polymer layer comprises at least the hydrophobic group.

10. The nanoparticle according to claim 9, wherein the hydrophobic group is at least one selected from the group consisting of an aromatic cyclic group, an aliphatic cyclic group, and an aliphatic chain group.

11. The nanoparticle according to claim 2 or 3, wherein a polymer constituting the polymer layer has a moiety comprising a disulfide linkage as a side chain.

12. The nanoparticle according to claim 7, wherein a polymer constituting the polymer layer has a moiety comprising a disulfide linkage as a side chain, and

the moiety comprising the disulfide linkage has the positively charged group.

**13.** The nanoparticle according to claim 9, wherein a polymer constituting the polymer layer has a moiety comprising a disulfide linkage as a side chain, and
the moiety comprising the disulfide linkage has the hydrophobic group.

**14.** The nanoparticle according to claim 2 or 3, wherein the polymer layer has a thickness of 1 nm to 10 nm.

**15.** The nanoparticle according to claim 2 or 3, which is a nanoparticle to be used for plasmon-excited fluorescence analysis.

**16.** The nanoparticle according to claim 2 or 3, wherein the specifically bondable substance is at least one selected from the group consisting of a nanoantibody, a ligand, an enzyme, and a nucleic acid strand.

**17.** The nanoparticle according to claim 2 or 3, wherein the specifically bondable substance is at least one nanoantibody selected from the group consisting of a VHH antibody, a fragmented antibody, and variants thereof.

**18.** The nanoparticle according to claim 2 or 3, wherein the metallic nanoparticle comprises gold or silver.

**19.** The nanoparticle according to claim 2 or 3,

wherein a first nanoparticle and a second nanoparticle are comprised as the nanoparticle;
the first nanoparticle comprises a first metallic nanoparticle as the metallic nanoparticle, a first polymer layer as the polymer layer, and a first specifically bondable substance as the specifically bondable substance;
the second nanoparticle comprises a second metallic nanoparticle as the metallic nanoparticle, a second polymer layer as the polymer layer, and a second specifically bondable substance as the specifically bondable substance; and
the fluorescent substance is labeled on at least one combination among a surface of the first metallic nanoparticle and a surface of the second metallic nanoparticle, the first polymer layer and the second polymer layer, and the first specifically bondable substance and the second specifically bondable substance.

**20.** The nanoparticle according to claim 19, wherein the first nanoparticle and the second nanoparticle form a composite by being bonded with each other with the test substance interposing therebetween.

**21.** The nanoparticle according to claim 19, wherein the test substance is a test substance derived from the specimen that is blood, plasma, urine, or saliva.

**22.** The nanoparticle according to claim 19, further comprising a fluorescent substance,
wherein in a composite in which the first nanoparticle and the second nanoparticle are bound with the test substance interposing therebetween, the fluorescent substance is positioned between the first nanoparticle and the second nanoparticle.

**23.** A method for producing a nanoparticle, the method comprising a step of mixing a polymer to which a fluorescent substance is bonded with a disulfide linkage interposing with a metallic nanoparticle to label the fluorescent substance on a surface of the metallic nanoparticle and simultaneously form a polymer layer on the surface of the metallic nanoparticle.

**24.** A nanoparticle composition comprising the nanoparticle according to claim 1 and a solvent comprising the nanoparticle,

wherein the specifically bondable substance is bonded to a surface of the polymer layer or a surface of the metallic nanoparticle, and
wherein the solvent comprises a polyanion-based polymer in addition to the nanoparticle.

**25.** The nanoparticle composition according to claim 24, wherein the polyanion-based polymer has at least one anionic group selected from the group consisting of a carboxylate salt group, a sulfate salt group, a sulfonate salt group, a nitrate salt group, a phosphate salt group, and a borate salt group.

**26.** The nanoparticle composition according to claim 24 or 25, wherein the polyanion-based polymer is at least one selected from the group consisting of polyglutamic acid, heparin, polyaspartic acid, polyacrylic acid, salts thereof, and DNA.

**27.** The nanoparticle composition according to claim 24 or 25,
wherein the polymer layer contains at least one selected from the group consisting of a binding site with a sulfur atom interposing therein, a positively charged group, and a hydrophobic group between the polymer layer and the surface of the metallic nanoparticle.

**28.** The nanoparticle composition according to claim 24 or 25, wherein a polymer constituting the polymer layer has at least one selected from the group consisting of the binding site with a sulfur atom interposing therein, the positively charged group, and the hydrophobic group at a terminal of a side chain.

**29.** The nanoparticle composition according to claim 24 or 25, wherein the specifically bondable substance is a nanoantibody.

**30.** The nanoparticle composition according to claim 24 or 25, wherein the specifically bondable substance is crosslinked with at least one selected from the group consisting of a polyalkylene ether chain and an alkyl chain.

**31.** The nanoparticle composition according to claim 24 or 25, wherein the polyanion-based polymer is present to surround individual particles of the nanoparticle.

**32.** The nanoparticle composition according to claim 24 or 25, wherein the solvent comprises an aqueous solvent.

**33.** The nanoparticle composition according to claim 24 or 25, comprising an associated body of the polyanion-based polymer and the nanoparticle.

**34.** The nanoparticle composition according to claim 24 or 25, which is a nanoparticle to be used for plasmon-excited fluorescence analysis.

**35.** The nanoparticle composition according to claim 24 or 25, wherein the metallic nanoparticle comprises gold or silver.

**36.** The nanoparticle composition according to claim 24 or 25, wherein a fluorescent substance is labeled on at least one of a surface of the polymer layer and the specifically bondable substance.

**37.** The nanoparticle composition according to claim 24 or 25,

wherein a first nanoparticle and a second nanoparticle are comprised as the nanoparticle;
the first nanoparticle comprises a first metallic nanoparticle as the metallic nanoparticle, a first polymer layer as the polymer layer, and a first specifically bondable substance as the specifically bondable substance;
the second nanoparticle comprises a second metallic nanoparticle as the metallic nanoparticle, a second polymer layer as the polymer layer, and a second specifically bondable substance as the specifically bondable substance; and
the fluorescent substance is labeled on at least one combination of the first polymer layer and the second polymer layer, and the first specifically bondable substance and the second specifically bondable substance.

**38.** The nanoparticle composition according to claim 37, wherein the first nanoparticle and the second nanoparticle form a composite by being bonded with each other with the test substance interposing therebetween.

**39.** The nanoparticle composition according to claim 38, in the composite, the fluorescent substance is positioned between the first nanoparticle and the second nanoparticle.

**40.** The nanoparticle composition according to claim 24 or 25, wherein the test substance is a test substance derived from blood, plasma, urine, or saliva.

FIG.1

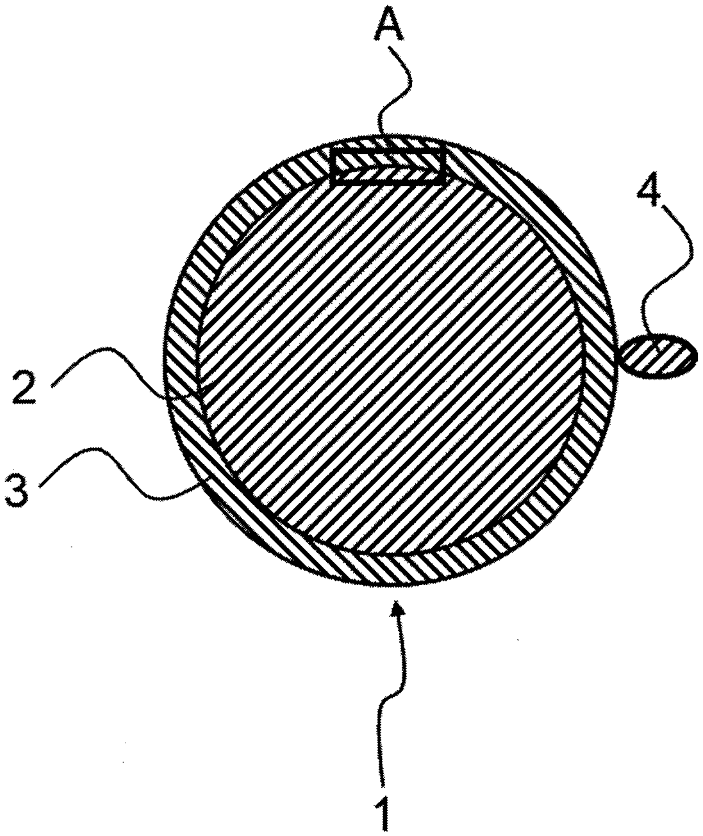

FIG.2

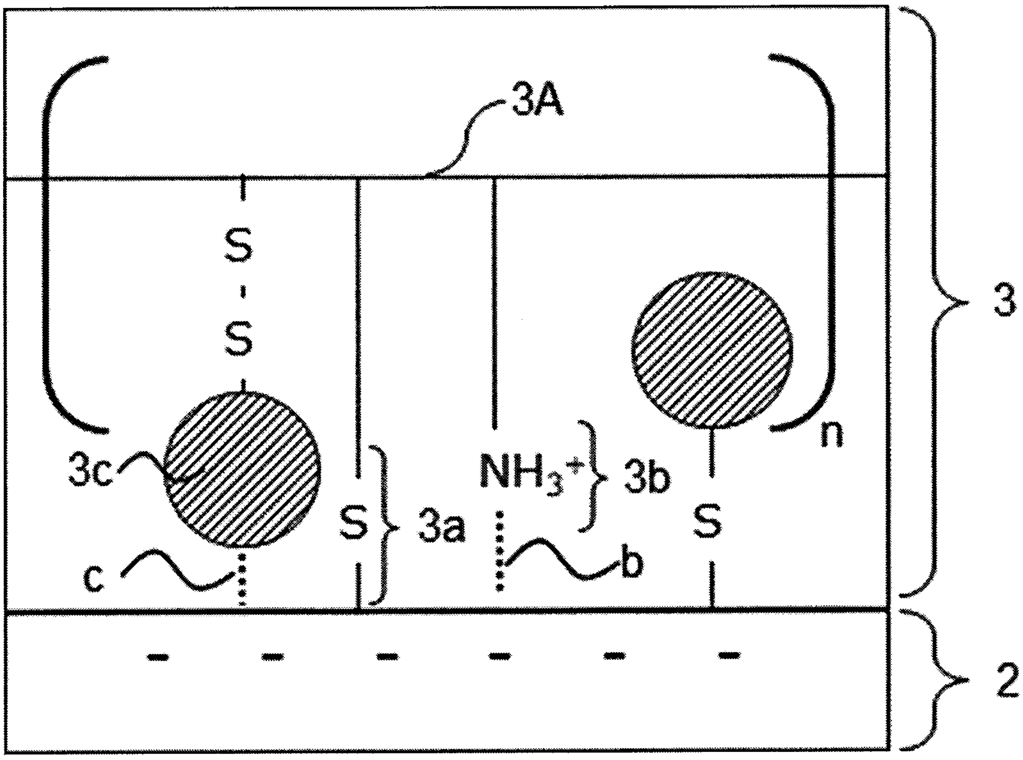


FIG.3

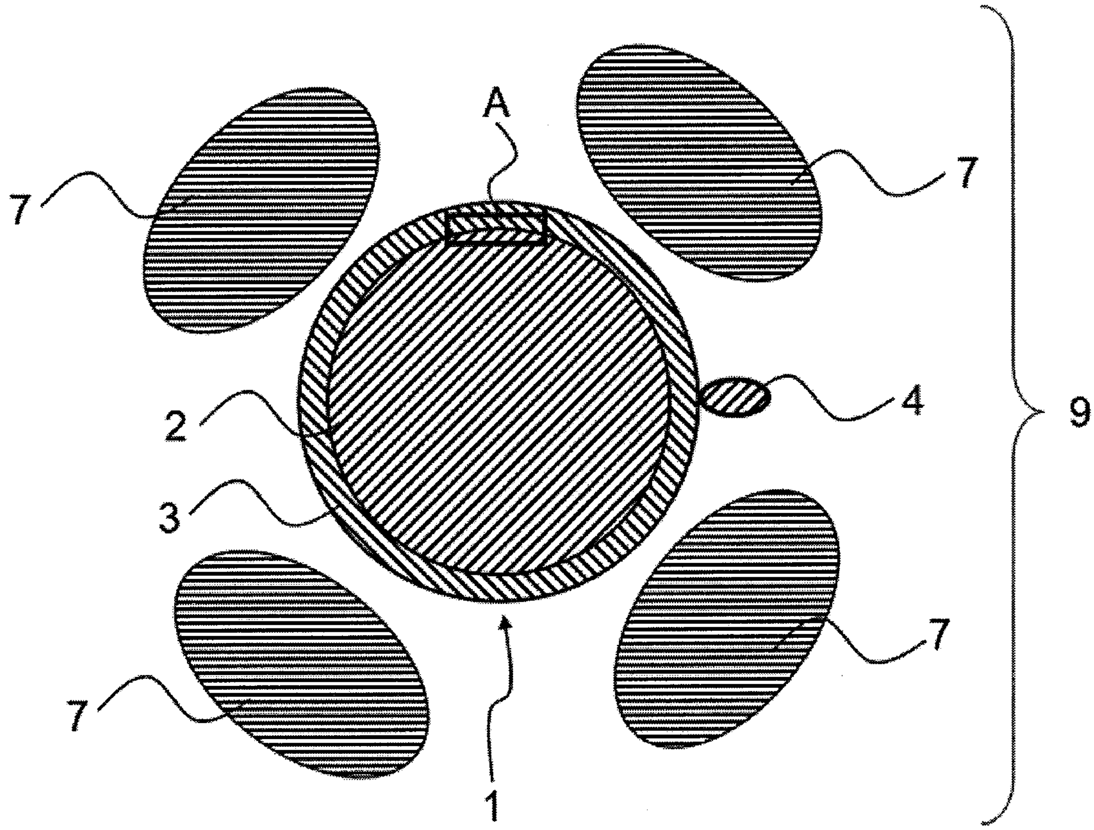

FIG.4

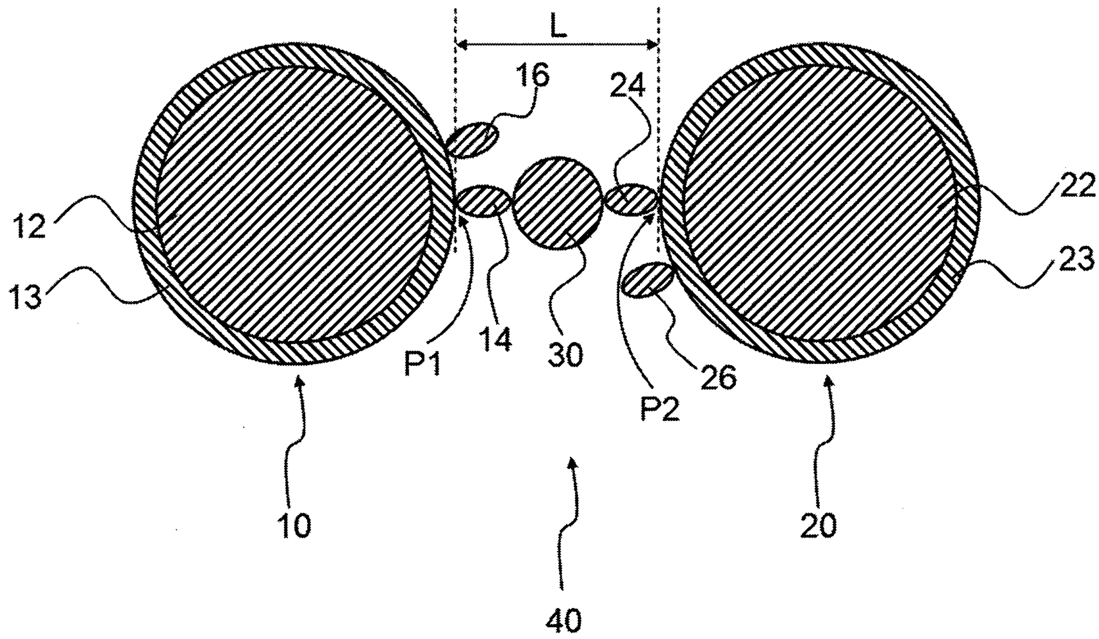
L
16
24
22
12
23
13
14
30
P1
26
P2
10
20
40

FIG.5

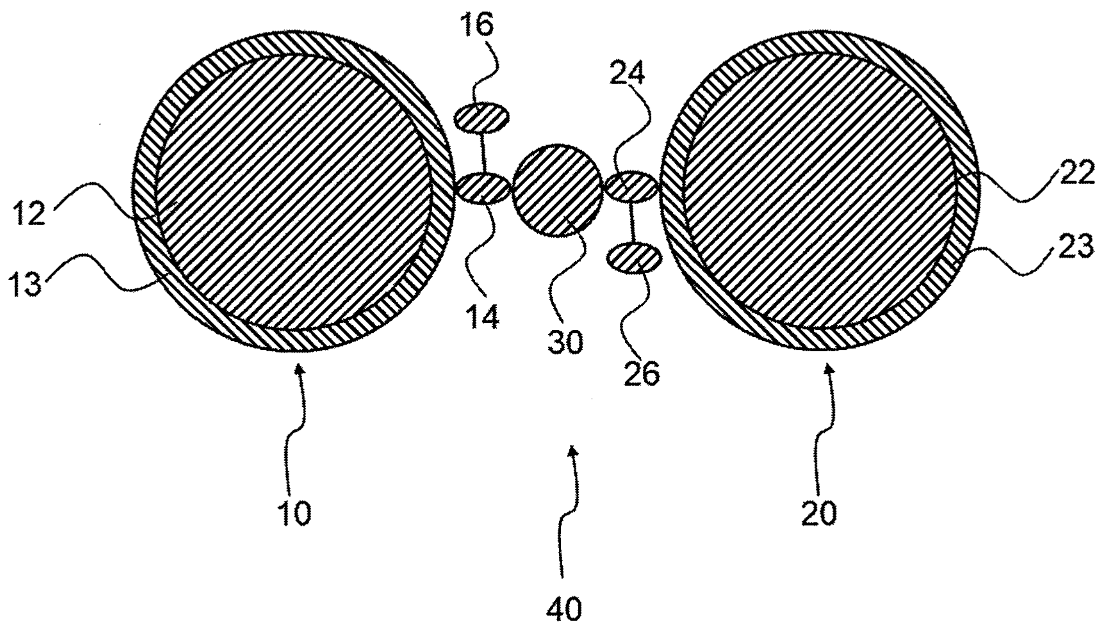
16
24
22
12
23
13
14
30
26
10
20
40

FIG.6

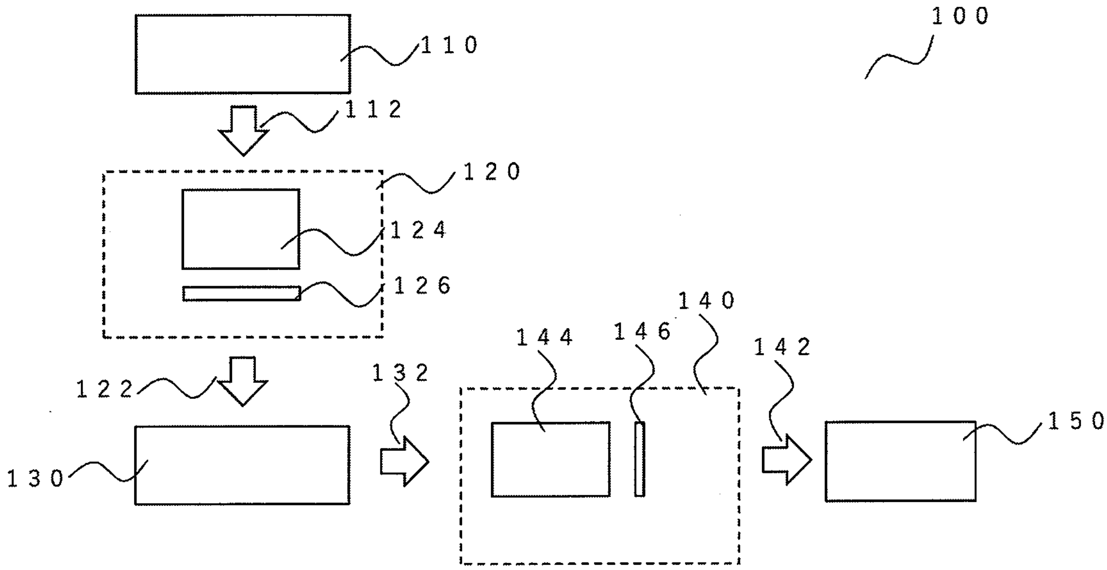

FIG.7

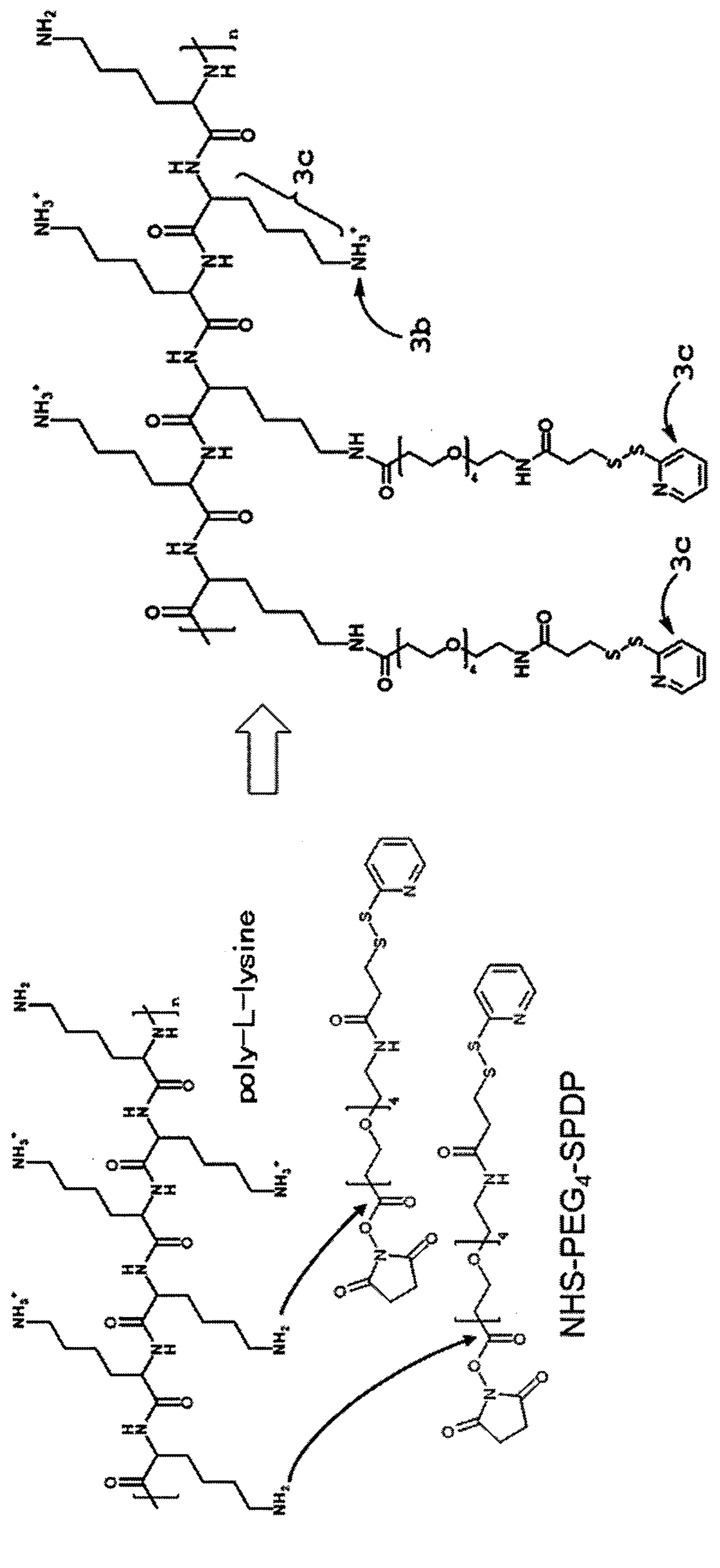

FIG.8

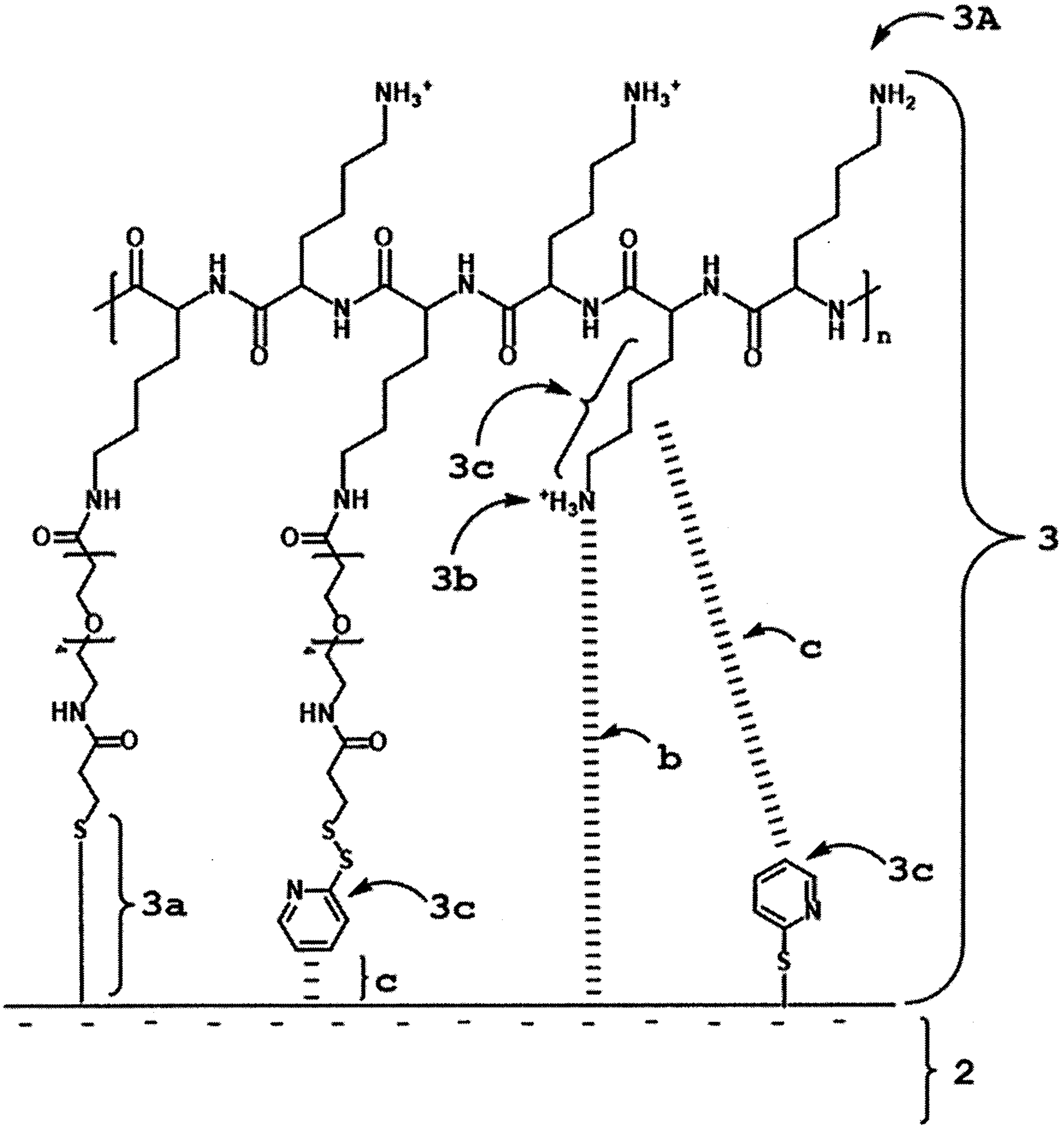

FIG. 9

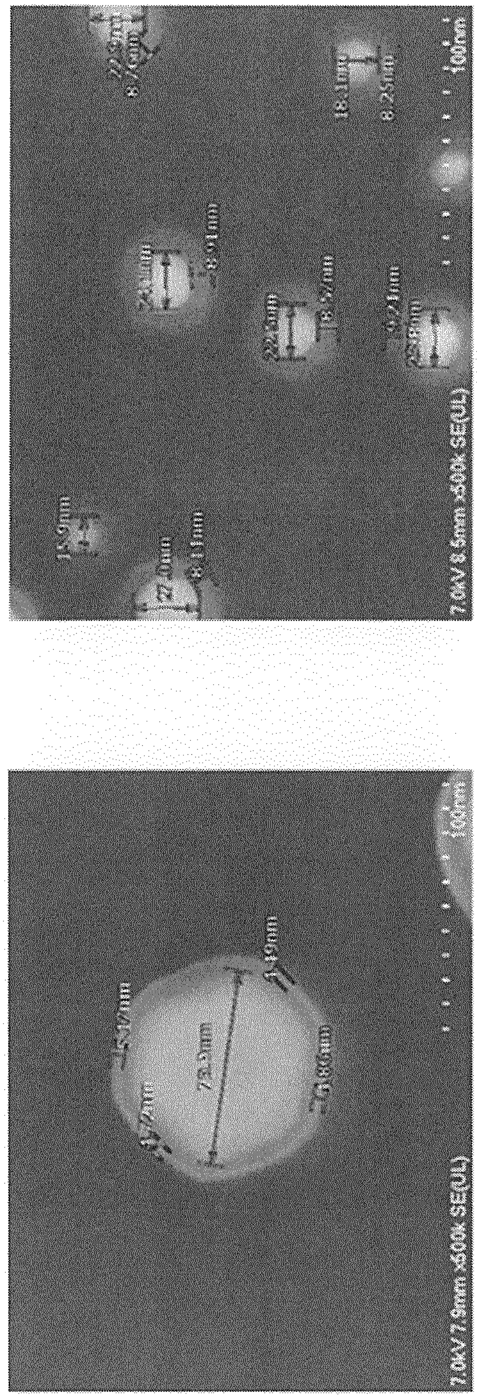

(a) silver nanoparticle (particle diameter: 80 nm)

(b) gold nanoparticle (particle diameter: 20 nm)

Fig. 10

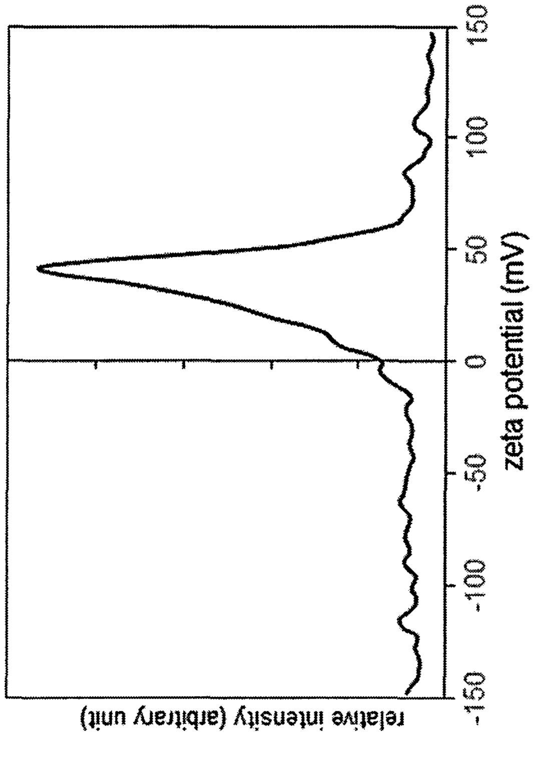
relative intensity (arbitrary unit)
-150
-100
-50
0
50
100
150
zeta potential (mV)
(a)

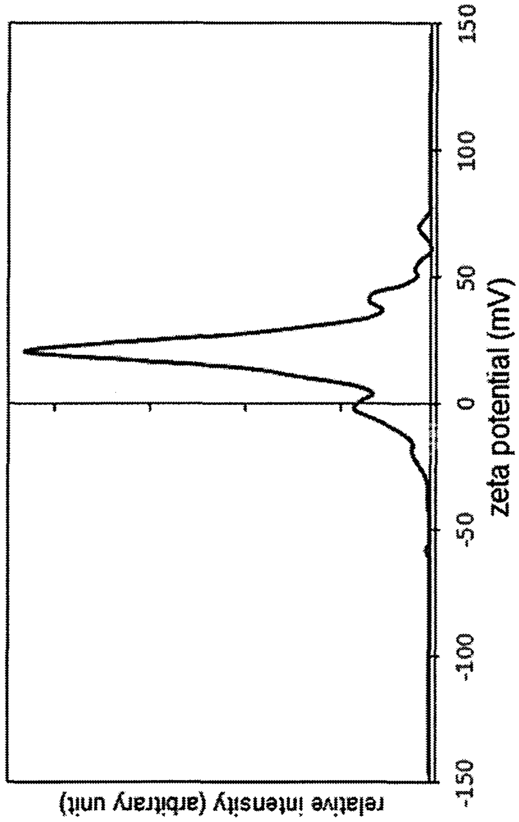
relative intensity (arbitrary unit)
-150
-100
-50
0
50
100
150
zeta potential (mV)
(b)

FIG. 11

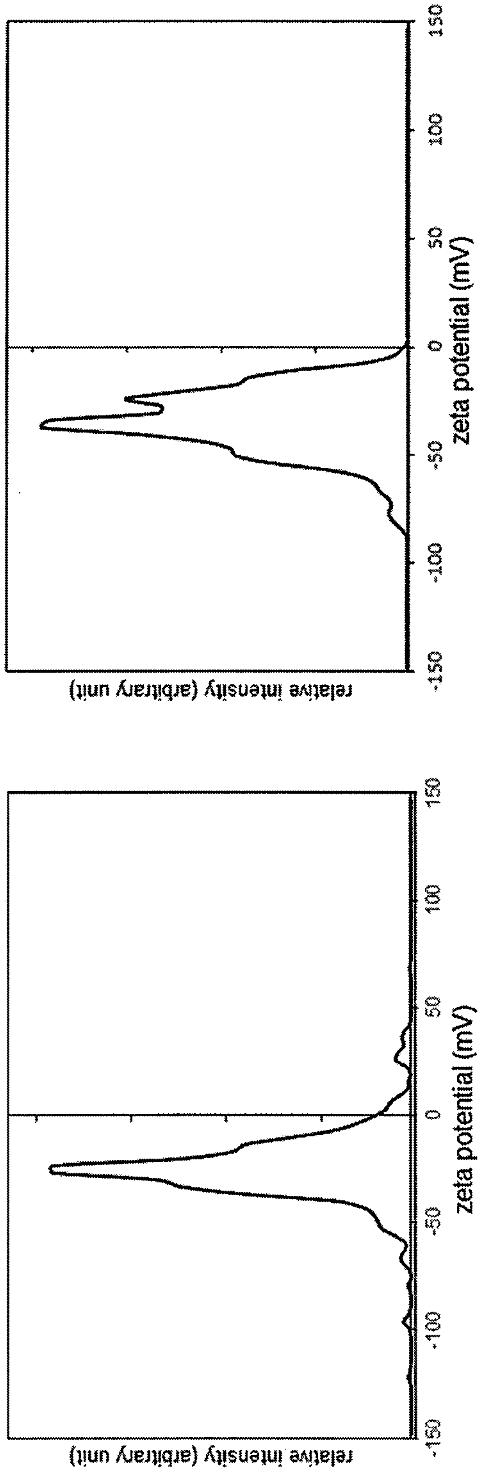
relative intensity (arbitrary unit)
zeta potential (mV)
-150
-100
-50
0
50
100
150
(b)
relative intensity (arbitrary unit)
zeta potential (mV)
-150
-100
-50
0
50
100
150
(a)

FIG. 12

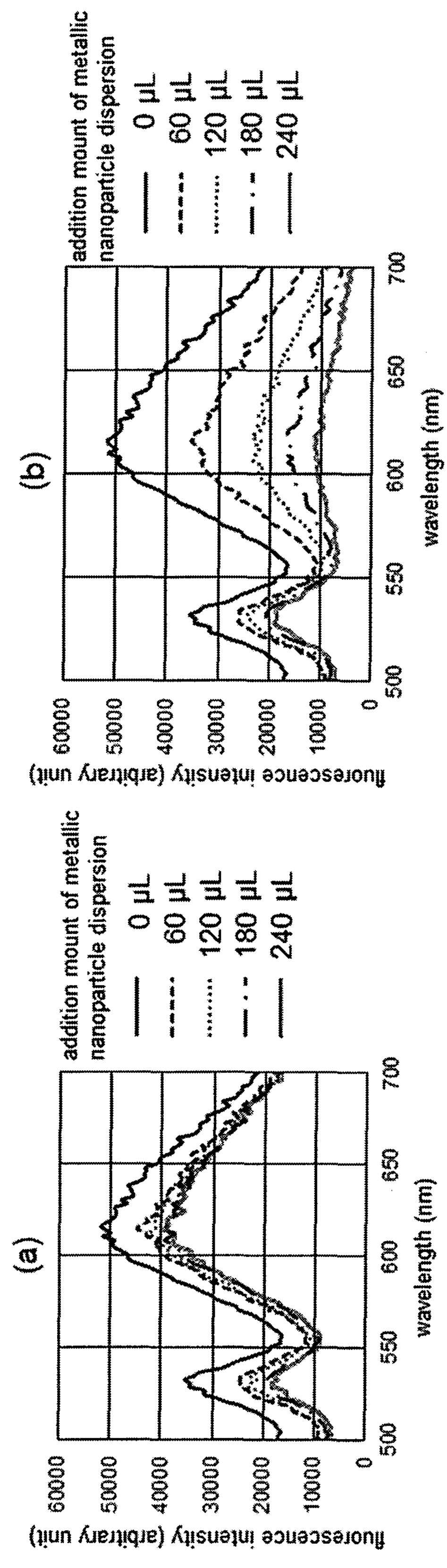
(a)
(b)
fluorescence intensity (arbitrary unit)
60000
50000
40000
30000
20000
10000
0
500
550
600
650
700
wavelength (nm)
addition mount of metallic nanoparticle dispersion
0 μL
60 μL
120 μL
180 μL
240 μL

FIG. 13

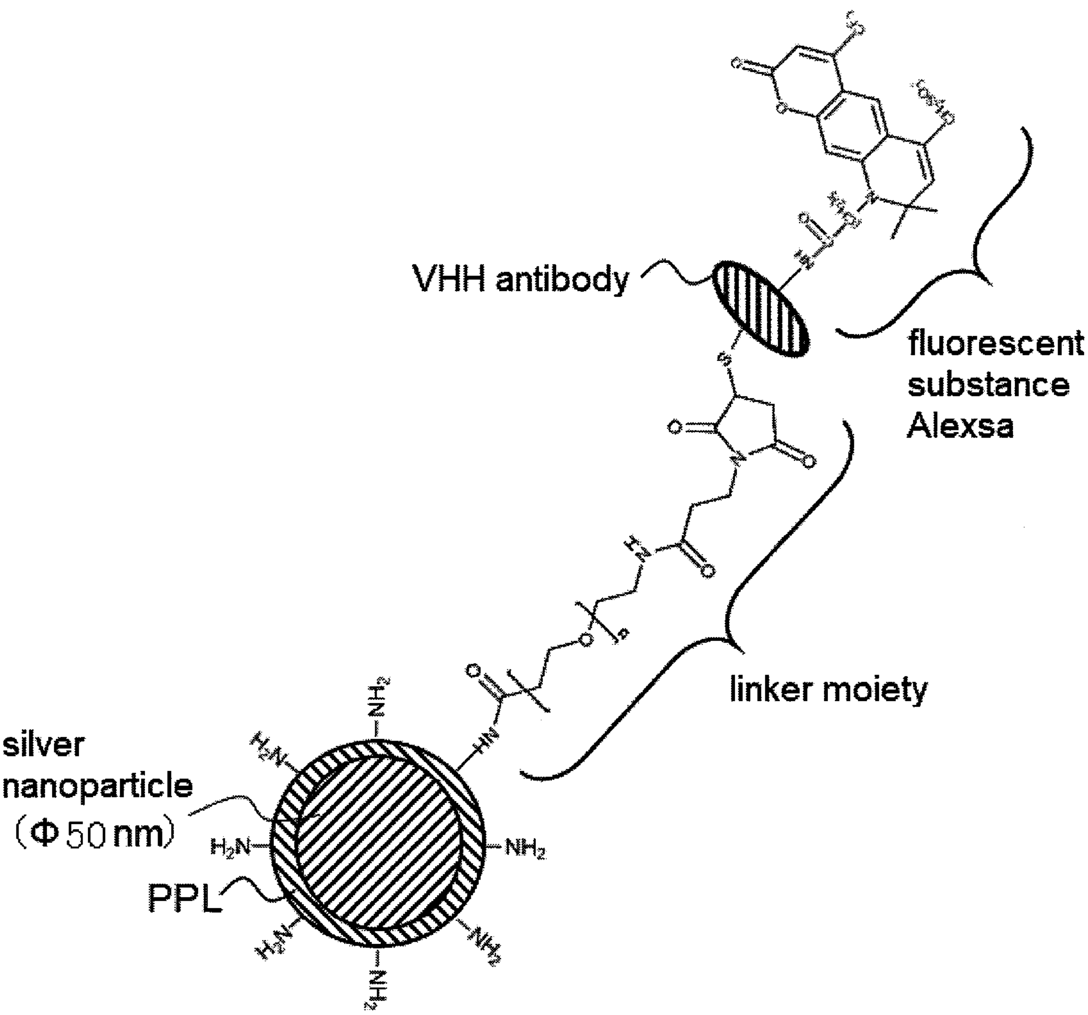
VHH antibody
fluorescent substance Alexsa
linker moiety
silver nanoparticle (Φ50nm)
PPL

FIG. 14

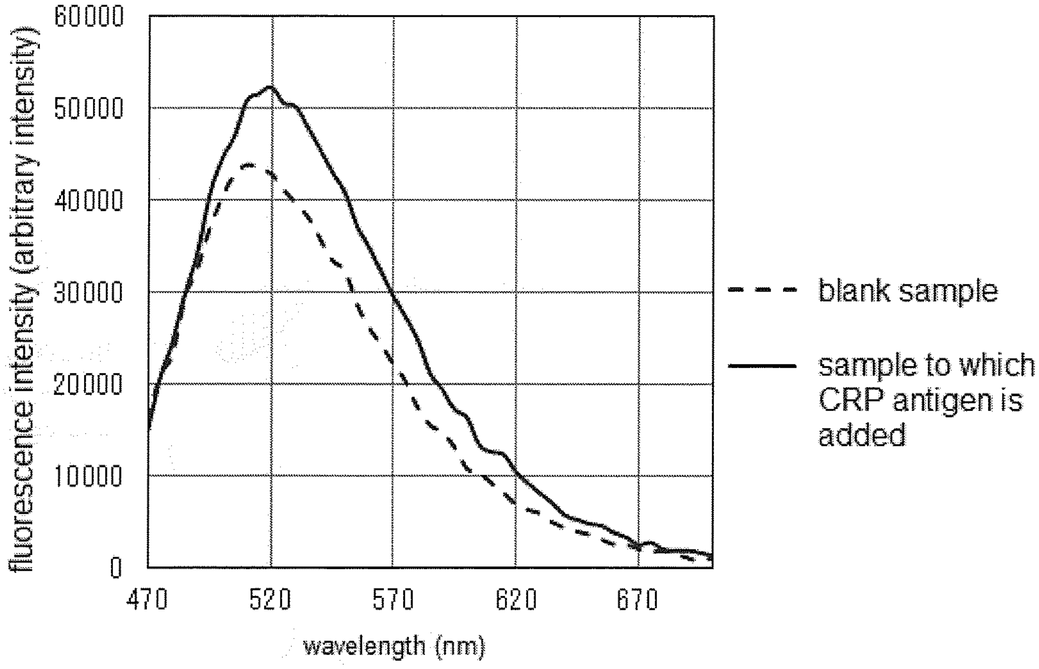
fluorescence intensity (arbitrary intensity)
60000
50000
40000
30000
20000
10000
0
470
520
570
620
670
wavelength (nm)
blank sample
sample to which CRP antigen is added

FIG.15

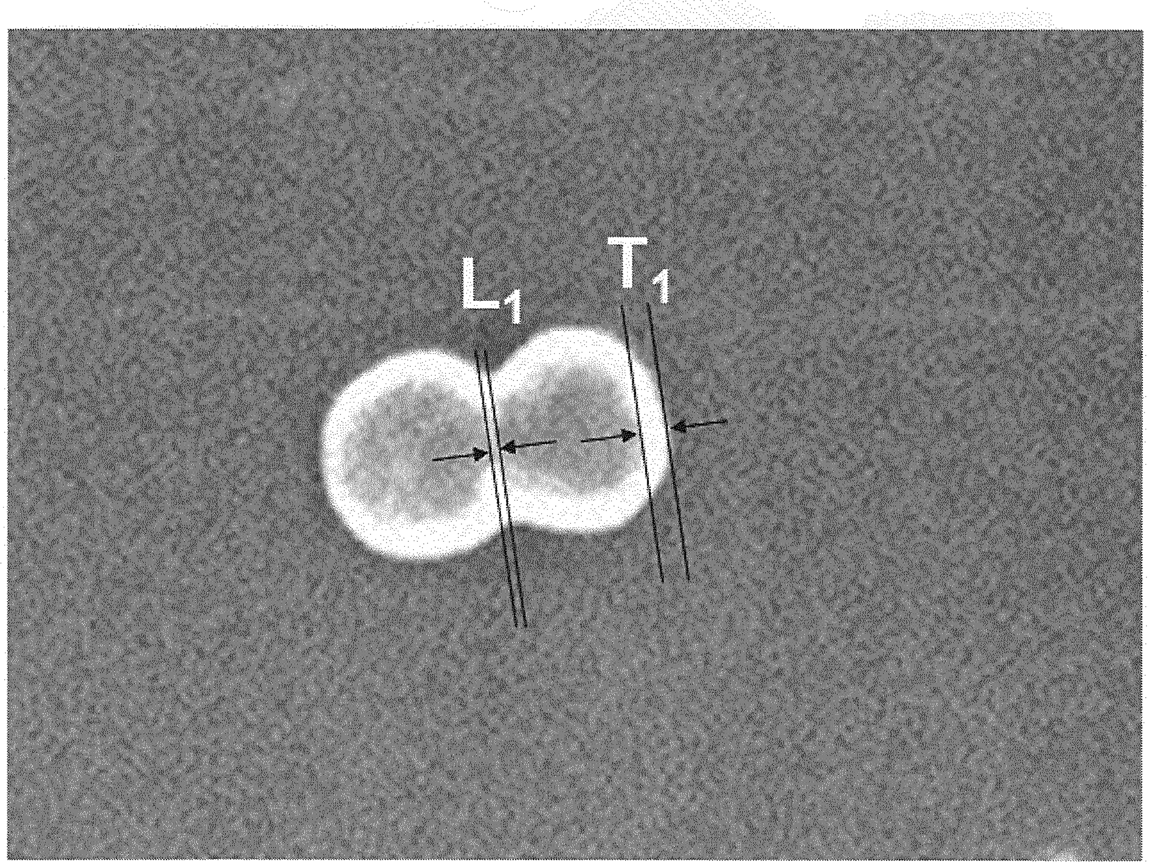
L1
T1

FIG.16

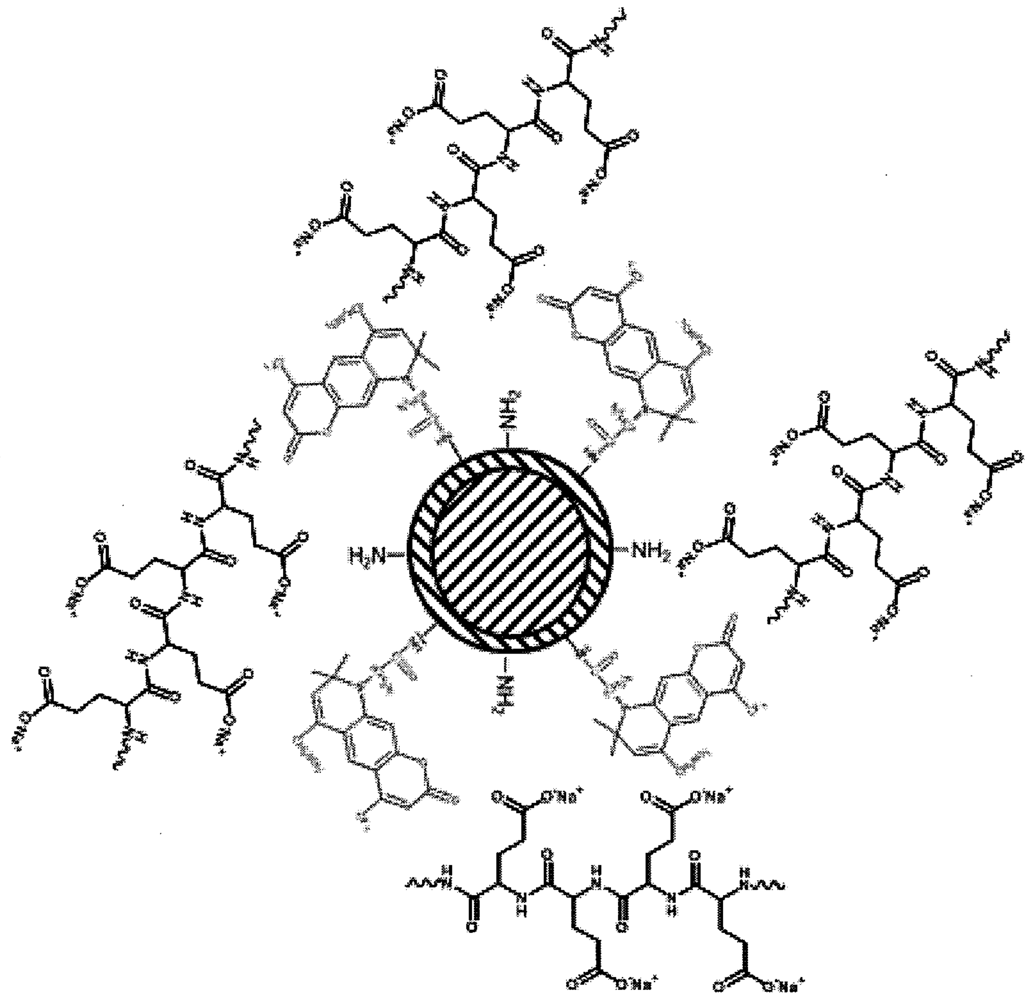

FIG. 17

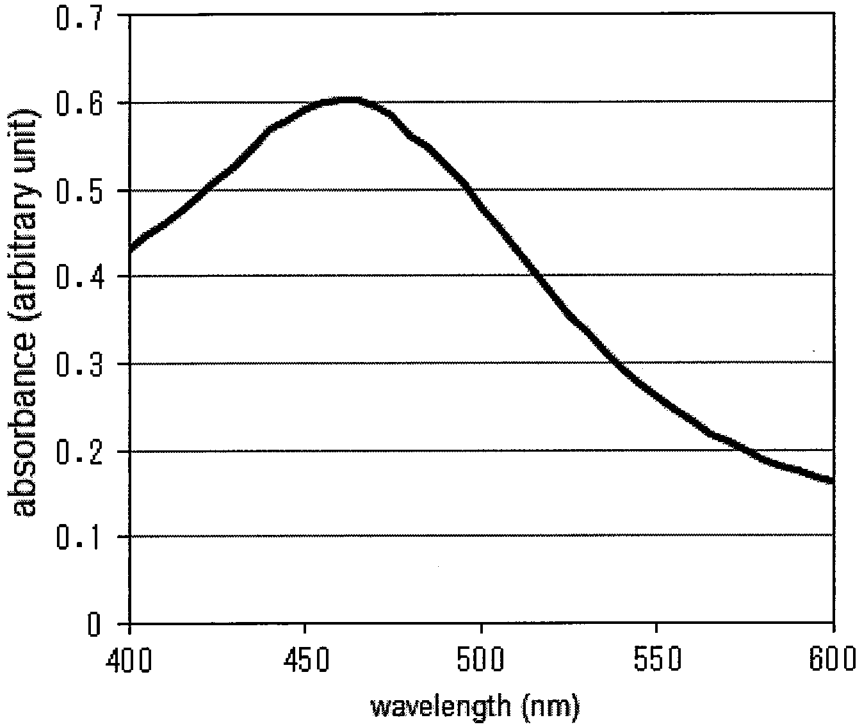
0.7
0.6
0.5
0.4
0.3
0.2
0.1
0
absorbance (arbitrary unit)
400
450
500
550
600
wavelength (nm)

Fig. 18

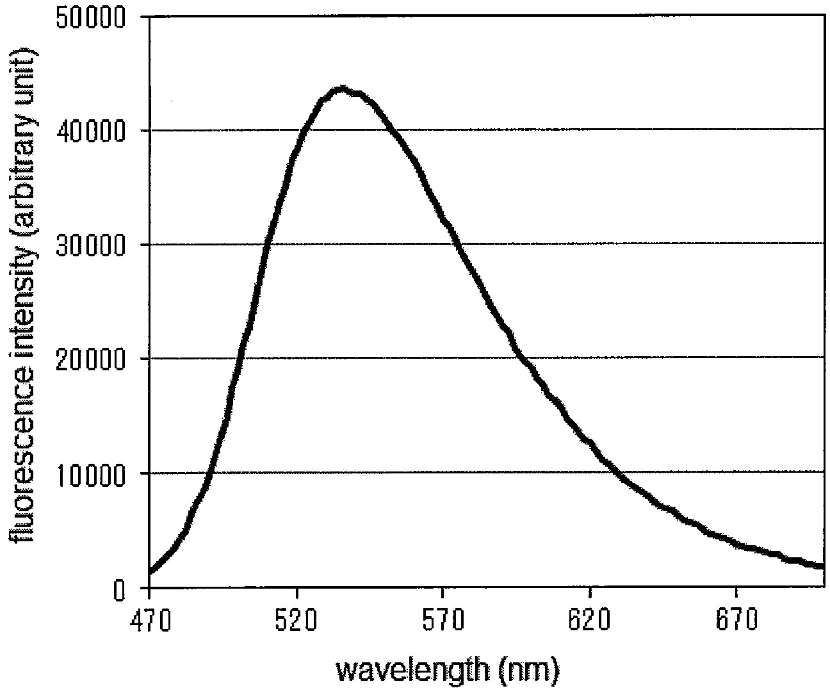
50000
40000
30000
20000
10000
0
fluorescence intensity (arbitrary unit)
470
520
570
620
670
wavelength (nm)

Fig. 19

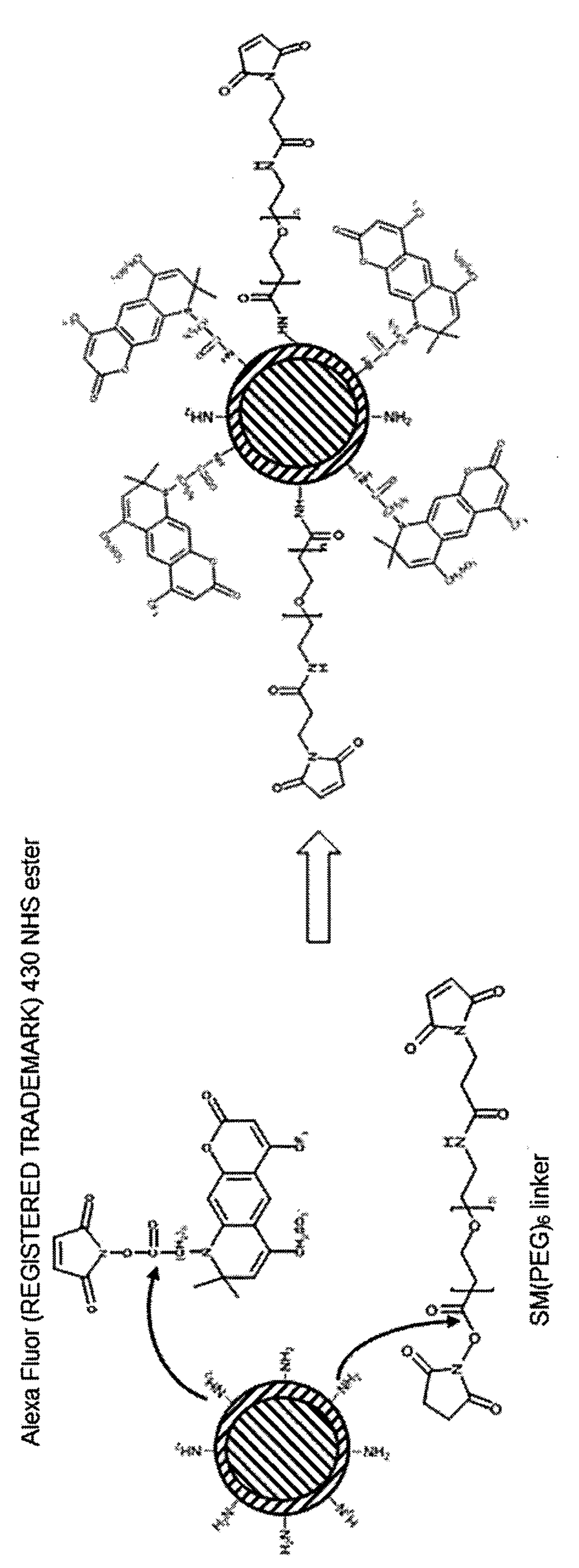
Alexa Fluor (REGISTERED TRADEMARK) 430 NHS ester
SM(PEG)6 linker

Fig. 20

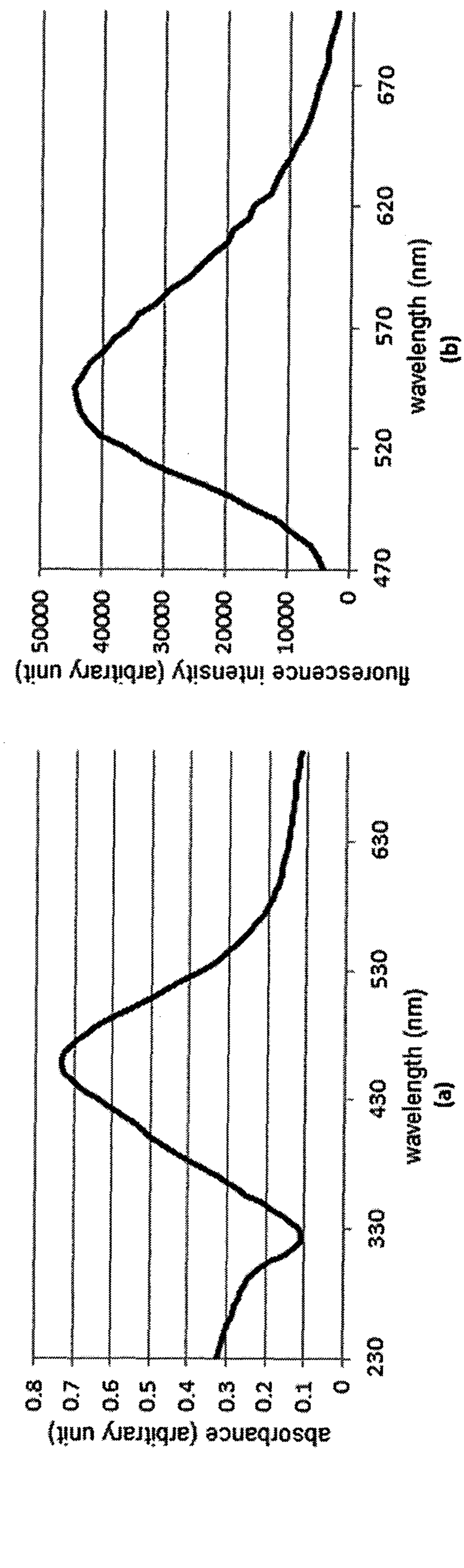
fluorescence intensity (arbitrary unit)
50000
40000
30000
20000
10000
0
470
520
570
620
670
wavelength (nm)
(b)
absorbance (arbitrary unit)
0.8
0.7
0.6
0.5
0.4
0.3
0.2
0.1
0
230
330
430
530
630
wavelength (nm)
(a)

FIG. 21

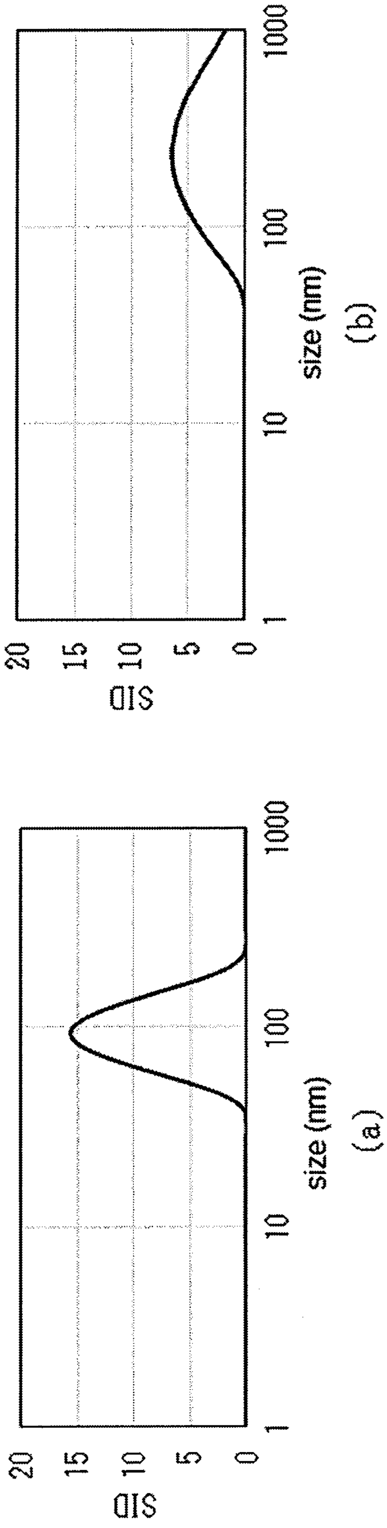
SID
20
15
10
5
0
1
10
100
1000
size (nm)
(b)
SID
20
15
10
5
0
1
10
100
1000
size (nm)
(a)

FIG. 22

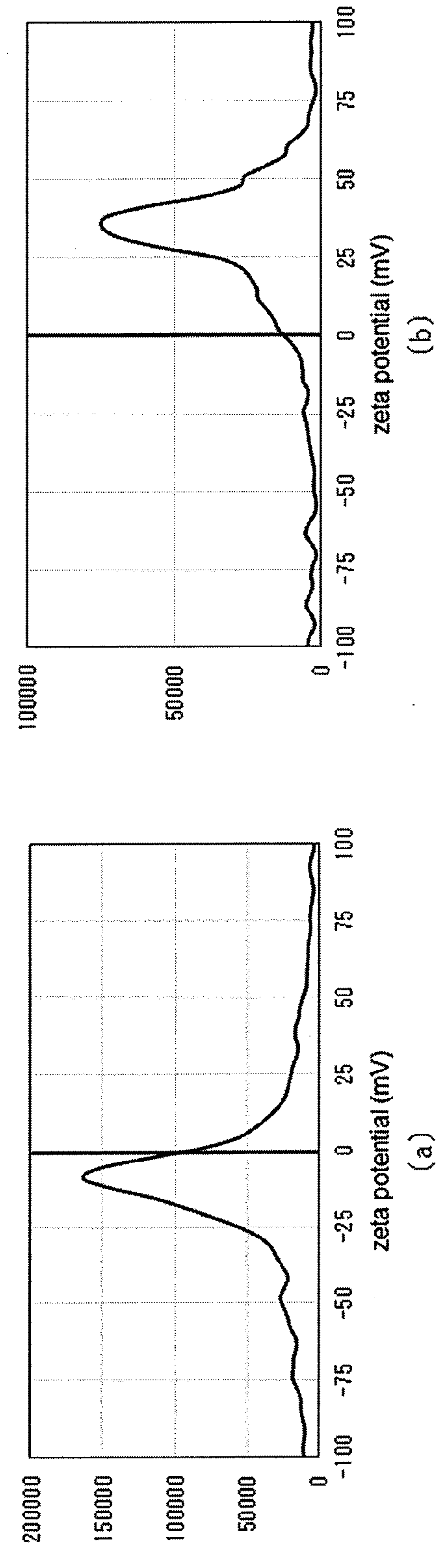
100000
50000
0
-100
-75
-50
-25
0
25
50
75
100
zeta potential (mV)
(b)
200000
150000
100000
50000
0
-100
-75
-50
-25
0
25
50
75
100
zeta potential (mV)
(a)

Fig. 23

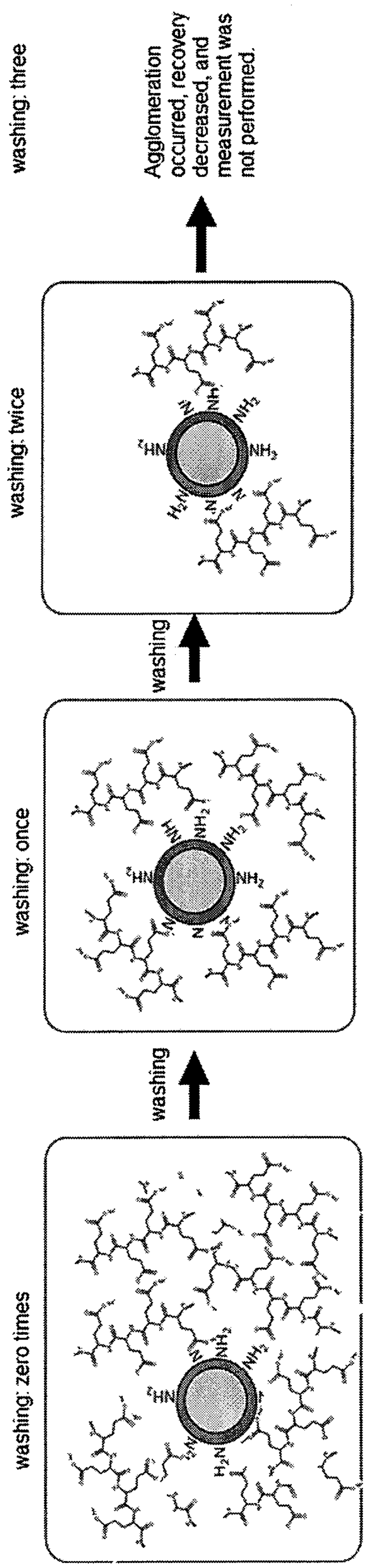
washing: zero times
washing
washing: once
washing
washing: twice
washing: three
Agglomeration occurred, recovery decreased, and measurement was not performed.

FIG.24

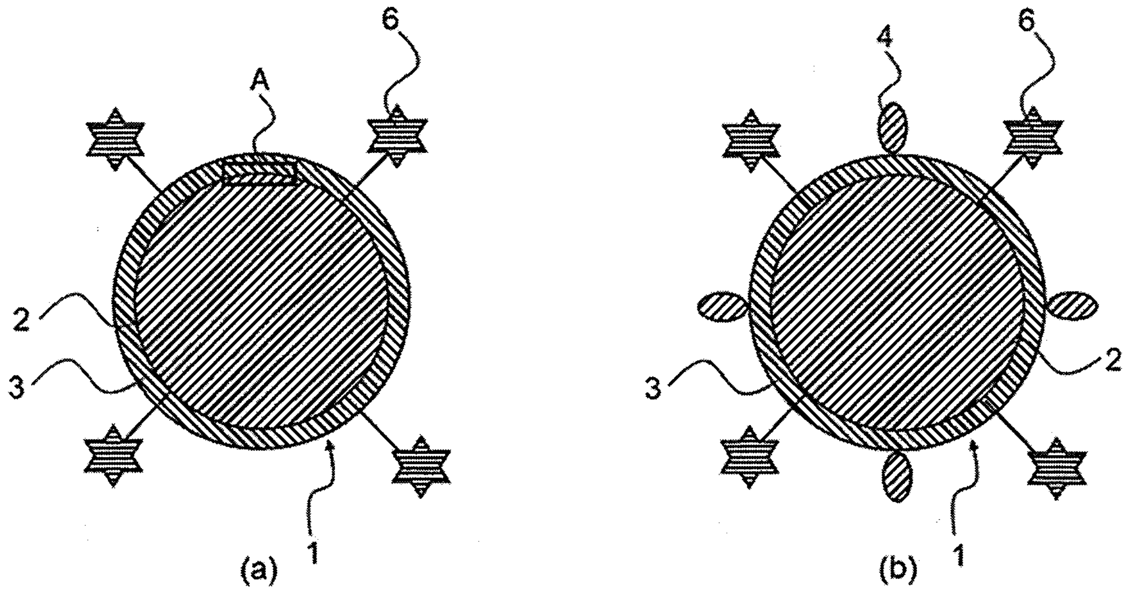


FIG.25

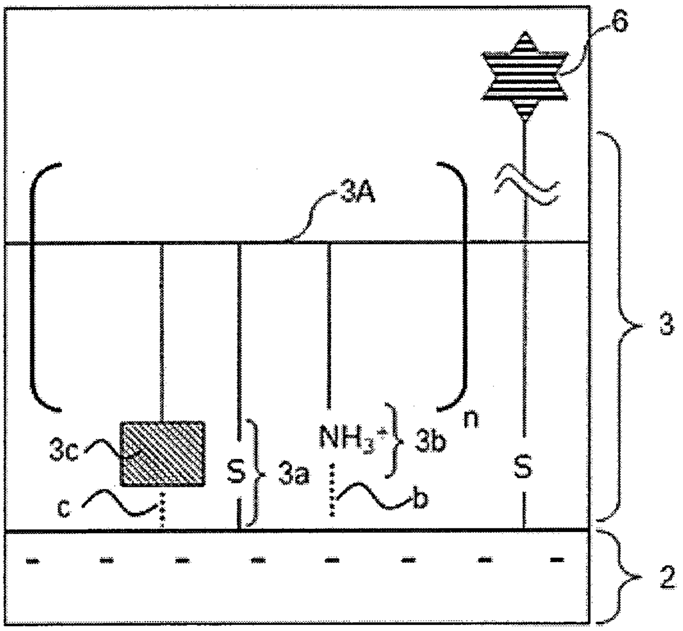

Fig. 26

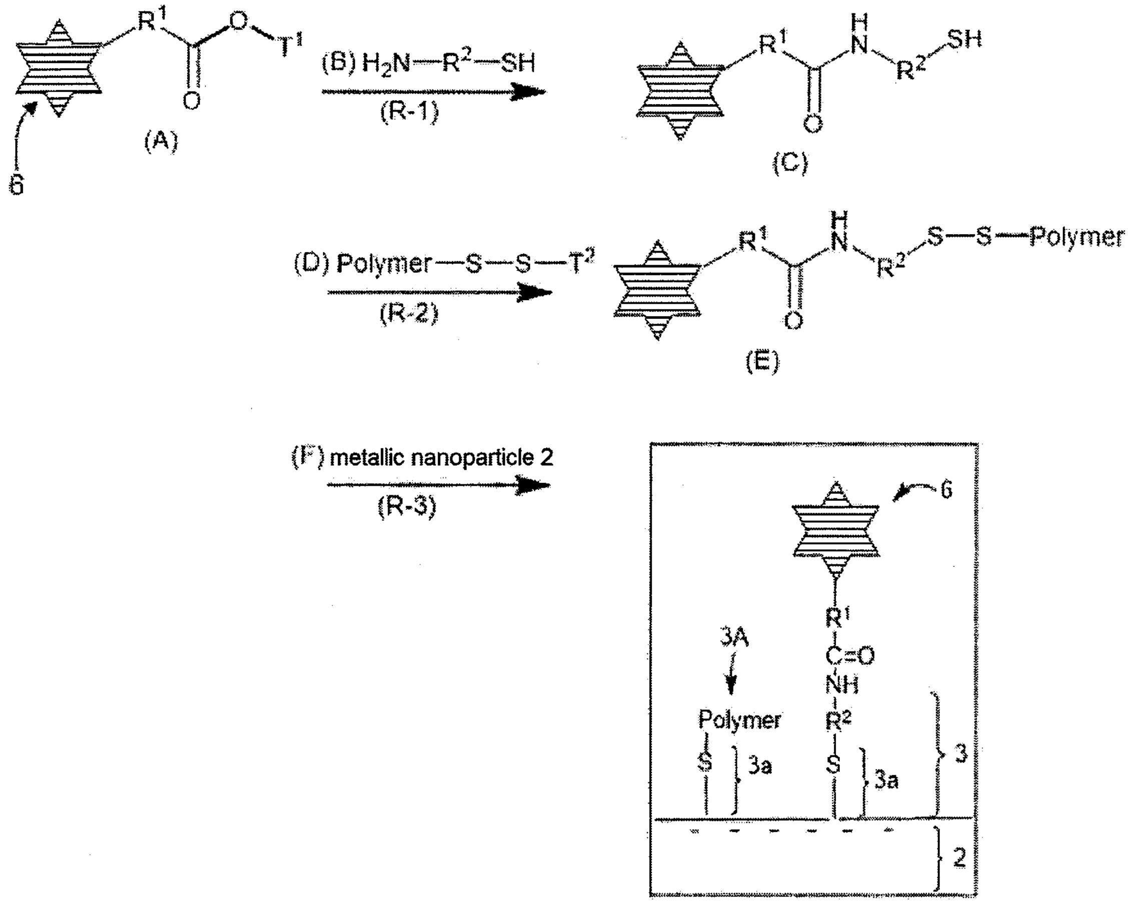
(A)
6
(B) $H_2N—R^2—SH$
(R-1)
(C)
(D) Polymer—S—S—$T^2$
(R-2)
(E)
(F) metallic nanoparticle 2
(R-3)
6
3A
3a
3a
3
2

FIG.27

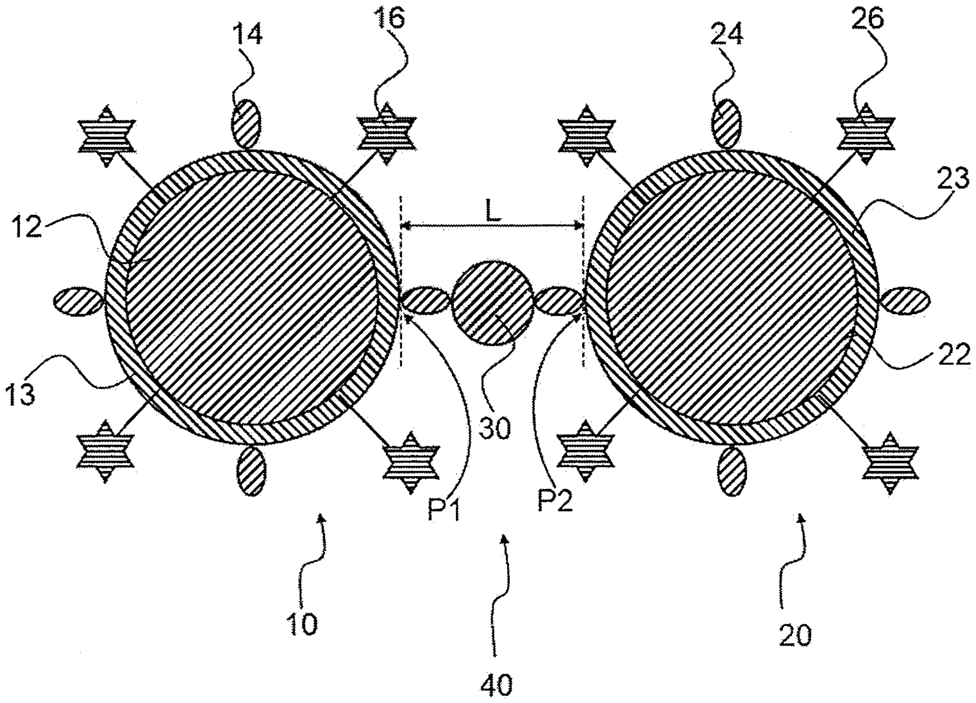
14
16
24
26
23
12
L
22
13
30
P1
P2
10
40
20

FIG.28

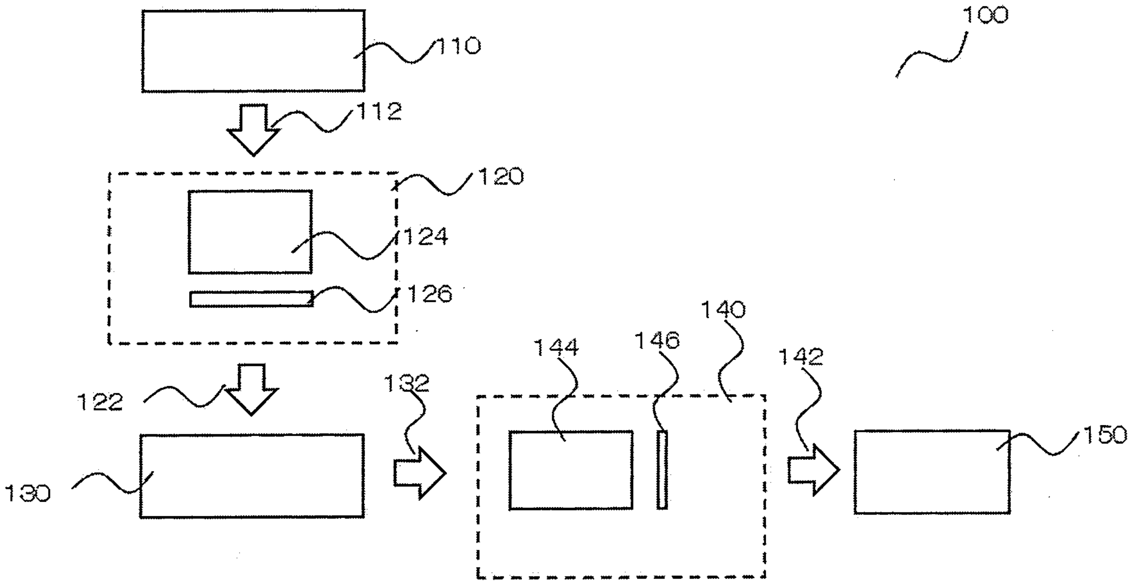


Fig. 29

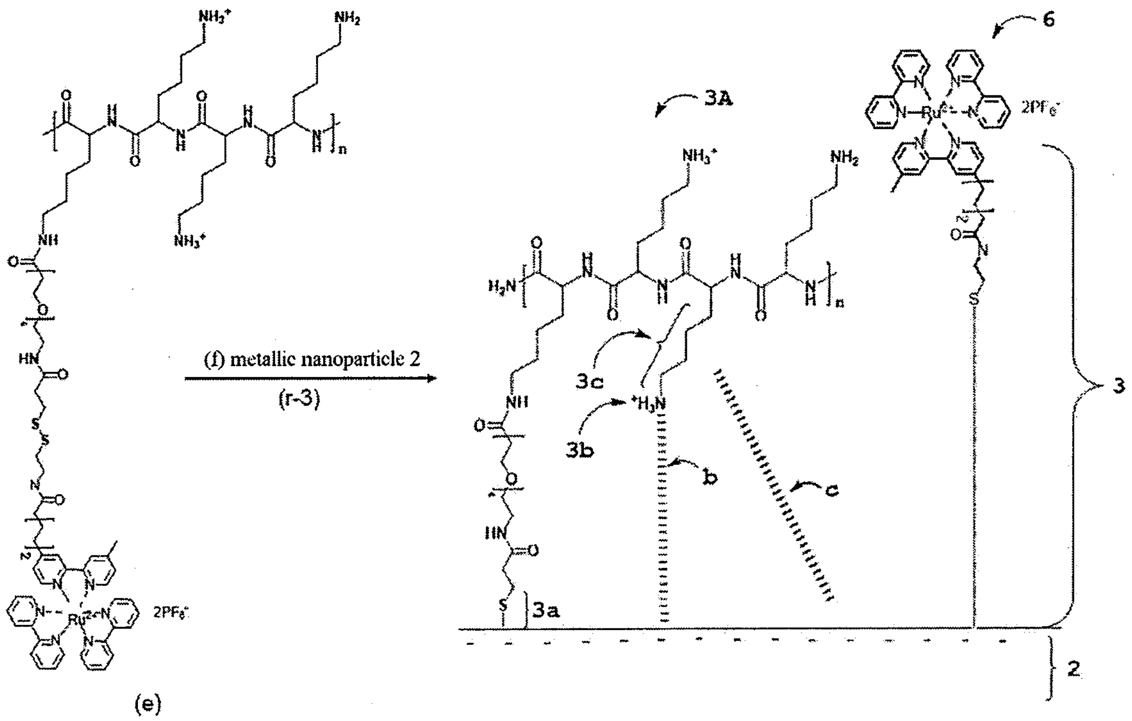


(e)

Fig. 30

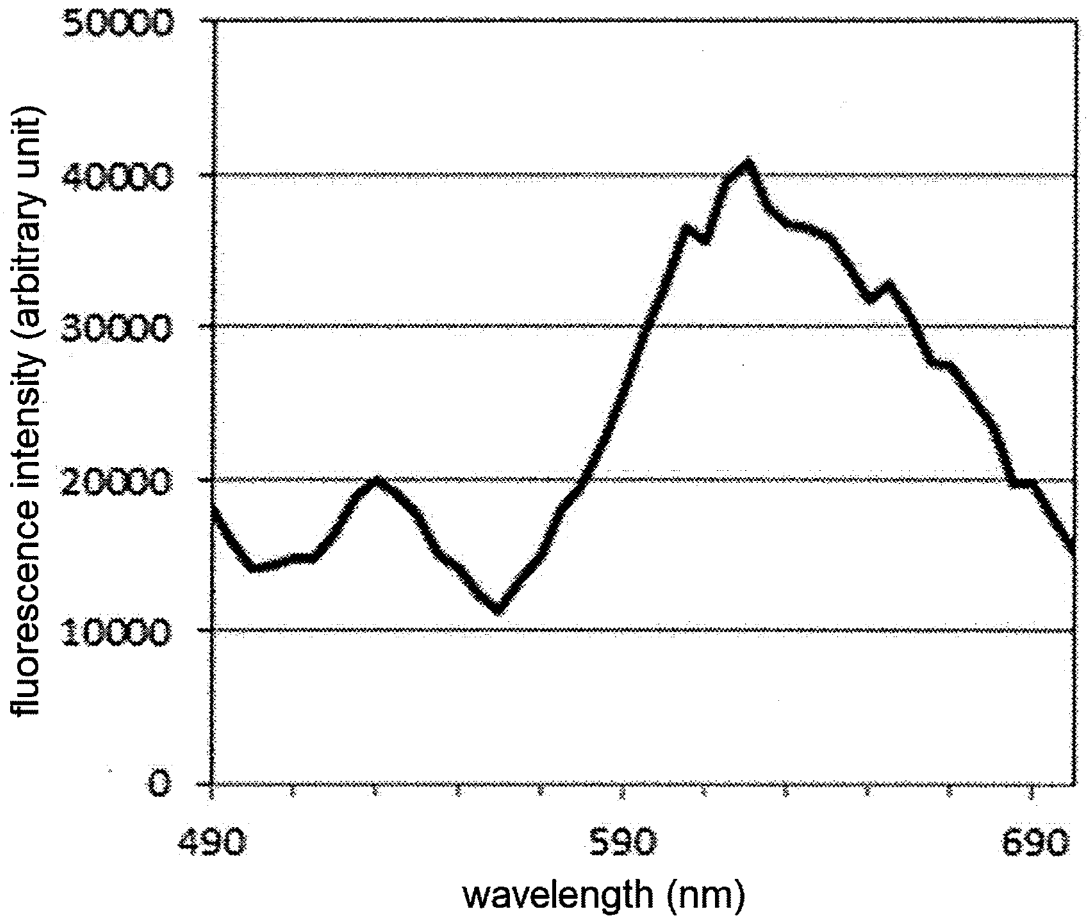
50000
40000
30000
20000
10000
0
fluorescence intensity (arbitrary unit)
490
590
690
wavelength (nm)

FIG.31

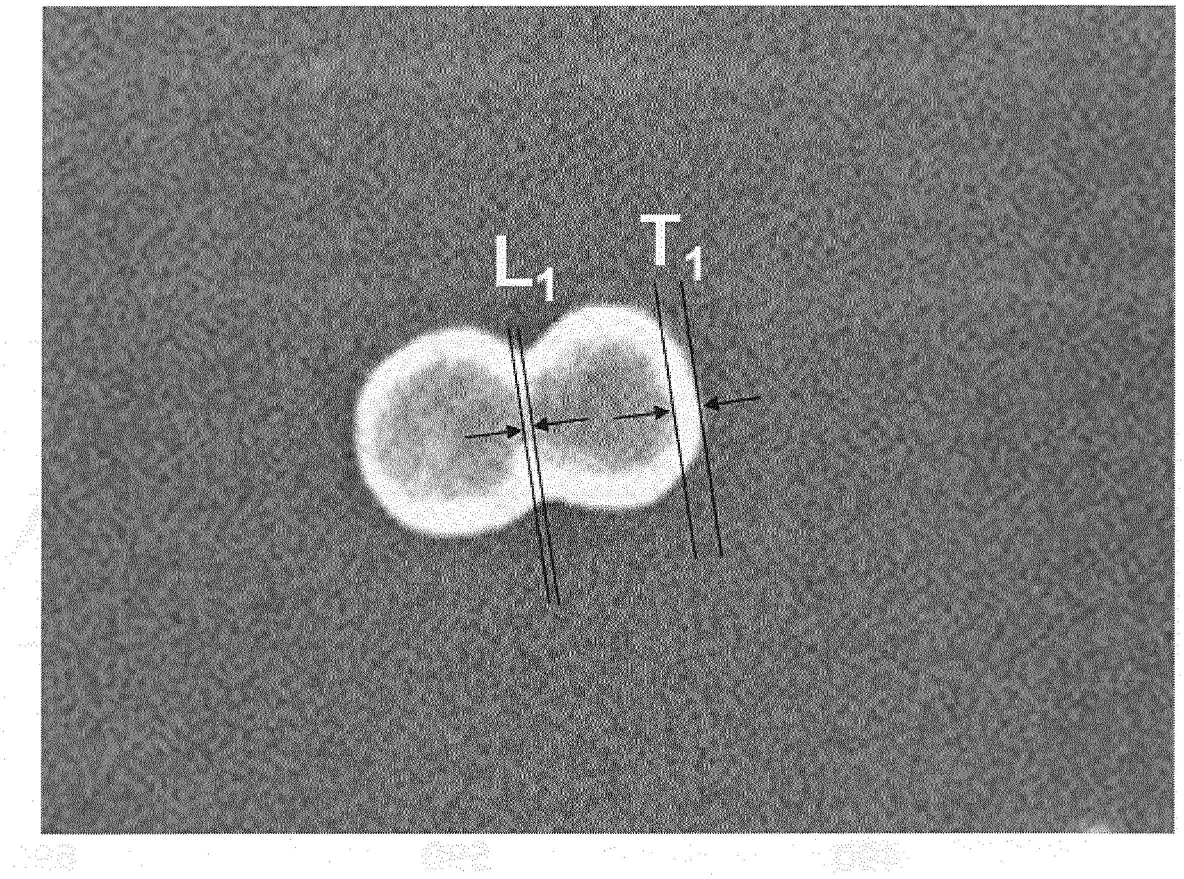

**INTERNATIONAL SEARCH REPORT**

International application No.
**PCT/JP2022/021270**

**A. CLASSIFICATION OF SUBJECT MATTER**

***G01N 21/64***(2006.01)i; ***B82Y 15/00***(2011.01)i; ***B82Y 30/00***(2011.01)i; ***C09K 11/06***(2006.01)i; ***C09K 11/08***(2006.01)i; ***C09K 11/58***(2006.01)i; ***G01N 21/78***(2006.01)i; ***G01N 33/543***(2006.01)i; ***G01N 33/553***(2006.01)i
FI: G01N21/64 G; B82Y15/00; B82Y30/00; C09K11/06 ZNM; C09K11/08 G ZNM; C09K11/58; G01N21/64 F; G01N21/64 Z; G01N21/78 C; G01N33/543 501M; G01N33/543 541Z; G01N33/543 565U; G01N33/543 565W; G01N33/543 575; G01N33/543 595; G01N33/553

According to International Patent Classification (IPC) or to both national classification and IPC

**B. FIELDS SEARCHED**

Minimum documentation searched (classification system followed by classification symbols)

G01N21/00-21/958; G01N33/48-33/98; B82Y15/00; B82Y30/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2015-129773 A (KONICA MINOLTA, INCORPORATED) 16 July 2015 (2015-07-16) paragraphs [0010], [0023], [0027]-[0032], [0066], [0092]-[0094], fig. 2 | 1-6, 11, 14-22 |
| Y | paragraphs [0010], [0023], [0027]-[0032], [0066], [0092]-[0094], fig. 2 | 7-8, 12, 24-40 |
| A | entire text, all drawings | 23 |
| Y | WO 2009/001994 A1 (CHIN, Chur) 31 December 2008 (2008-12-31) page 5 | 7-8, 12 |
| X | US 2011/0151586 A1 (NANYANG TECHNOLOGICAL UNIVERSITY) 23 June 2011 (2011-06-23) paragraphs [0085]-[0086], [0100]-[0114], [0142], fig. 1 | 1-2, 9-10, 13 |
| Y | JP 2015-182334 A (TORAY IND., INCORPORATED) 22 October 2015 (2015-10-22) paragraph [0043] | 24-40 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 July 2022** | **09 August 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/021270**

| Patent document cited in search report | Publication date (day/month/year) | Patent family member(s) | Publication date (day/month/year) |
|---|---|---|---|
| JP 2015-129773 A | 16 July 2015 | WO 2011/096394 A1 | |
| WO 2009/001994 A1 | 31 December 2008 | US 2010/0087522 A1<br>US 2012/0295969 A1<br>KR 10-2007-0075390 A<br>KR 10-2007-0083226 A<br>KR 10-2007-0103729 A<br>KR 10-2007-0116210 A<br>KR 10-2007-0116768 A<br>KR 10-2008-0114476 A | |
| US 2011/0151586 A1 | 23 June 2011 | WO 2009/142604 A1 | |
| JP 2015-182334 A | 22 October 2015 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011220705 A